# EUROPEAN PATENT SPECIFICATION

(11) **EP 3 727 435 B1**
(45) Date of publication and mention of the grant of the patent: **18.02.2026**
(21) Application number: 18891243.0
(22) Date of filing: 21.12.2018
(51) Int. Cl.: A61K 39/00, C07H 21/00, C12Q 1/6827, C12Q 1/6883, C07H 21/02, C07H 21/04

(54) **BIVALENT NUCLEIC ACID LIGANDS AND USES THEREFOR**
BIVALENTE NUKLEINSÄURELIGANDEN UND VERWENDUNGEN DAVON
LIGANDS BIVALENTS D'ACIDES NUCLÉIQUES ET LEURS UTILISATIONS

(30) Priority: 21.12.2017 US 201762708783 P; 14.02.2018 US 201862710262 P
(43) Date of publication of application: 28.10.2020
(73) Proprietor: CARNEGIE MELLON UNIVERSITY, Pittsburgh, Pennsylvania 15213 (US)
(72) Inventor: LY, Danith, H., Pittsburgh, Pennsylvania 15213 (US); THADKE, Shivaji, A., Pittsburgh, Pennsylvania 15217 (US); PERERA, J. Dinithi R., Pittsburgh, Pennsylvania 15217 (US)
(74) Representative: Grünecker Patent- und Rechtsanwälte PartG mbB
(86) International application number: PCT/US2018/067080
(87) International publication number: WO 2019/126638

(56) References cited:
- WO-A1-2015/172058
- WO-A1-2018/058091
- WO-A2-2014/169206
- WO-A2-2014/169206
- US-A1- 2016 083 433
- THADKE SHIVAJI A. ET AL: "Shape selective bifacial recognition of double helical DNA", vol. 1, no. 1, 7 November 2018 (2018-11-07), XP055828508, Retrieved from the Internet <URL:http://www.nature.com/articles/s42004-018-0080-5> DOI: 10.1038/s42004-018-0080-5
- THADKE SHIVAJI A. ET AL: "Design of Bivalent Nucleic Acid Ligands for Recognition of RNA-Repeated Expansion Associated with Huntington's Disease", BIOCHEMISTRY, vol. 57, no. 14, 21 March 2018 (2018-03-21), pages 2094 - 2108, XP055828551, ISSN: 0006-2960, DOI: 10.1021/acs.biochem.8b00062

## Description

### STATEMENT REGARDING FEDERAL FUNDING

This invention was made with government support under Grant No. R21NS098102 awarded by the National Institutes of Health, and Grant No. CHE1039870 awarded by the National Science Foundation. The government has certain rights in this invention.

### CROSS REFERENCE TO RELATED APPLICATIONS

This application claims the benefit of co-pending U.S. Provisional Application, No. 62/708,783, filed December 21, 2017 and U.S. Provisional Application, No. 62/710,262, filed February 14, 2018.

The Sequence Listing associated with this application is filed in electronic format via EFS-Web. The name of the text file containing the Sequence Listing is 6526_1807597_ST25.txt. The size of the text file is 444 bytes, and the text file was created on December 21, 2018.

### BACKGROUND

### 1. Field of the Invention

Described herein are genetic recognition reagents for binding nucleic acids. The provided genetic recognition reagents may be used in a method of treating an expanded repeat disease, for example, a PolyQ disease such as Huntington's disease.

### 2. Description of the Related Art

RNA-repeated expansions are prevalent in neuromuscular disorders (or disease). One such example is Huntington's disease (HD), an autosomal dominant disorder that affects muscle coordination, and leads to behavioral changes, cognitive decline, and dementia. HD typically manifests in midlife, and death ensues 10 to 20 years after onset. It is caused by an expansion of CAG-repeats in the first exon of the *huntingtin (htt)* gene, from a normal range of 6-29 to a pathogenic range of 40-180. The length of the CAG-repeat expansion (CAG^{exp}) is inversely related to the age of onset. *Htt* encodes a 348-kDa protein that is ubiquitously expressed, but most predominantly in the neurons of CNS, with diverse physiological roles, including embryonic development and neuroprotection. The CAG-repeats are translated into a polyglutamine (polyQ) sequence in the N-terminal region of Htt. Despite the vast knowledge of molecular dysfunctions and clinical manifestations, the exact mechanism by which CAG^{exp} causes HD is not yet fully understood; however, emerging evidence suggests that it is a multivariate disorder.

A loss of protein function could contribute to the etiology of HD, but it is unlikely the main culprit since heterozygous and homozygous patients with CAG^{exp} have similar clinical features. Furthermore, an individual has been identified who showed no abnormal phenotype despite a 50% reduction in the normal Htt protein level due to deletion in one of the *htt* alleles. Likewise, mice heterozygous for the *htt* null mutation do not show clinical features of HD, and homozygotes die in early embryo genesis. These findings underscore the importance of Htt in embryonic development, but not in the pathogenesis of HD. Emerging evidence points to deleterious gain-of-function as a more plausible cause of the disease. This suggestion is substantiated by the observations that CAG-repeats exceeding a similar threshold (30-40 units) in unrelated genes are responsible for several neuromuscular disorders in addition to HD, including, DRPLA (dentatorubral-pallidoluysian atrophy), SBMA (Spinal and bulbar muscular atrophy), SCA1 (Spinocerebellar ataxia Type 1), SCA2 (Spinocerebellar ataxia Type 2), SCA3 (Spinocerebellar ataxia Type 3 or Machado-Joseph disease), SCA6 (Spinocerebellar ataxia Type 6), SCA7 (Spinocerebellar ataxia Type 7), and SCA17 (Spinocerebellar ataxia Type 17). These disorders, referred to as polyQ diseases, have many traits, including subcortical and cortical atrophy, and nuclear aggregates that are in common with HD.

Three leading cytotoxic mechanisms have been proposed for HD and other polyQ diseases. The first is polyQ toxicity. PolyQ has a propensity to aggregate and interact with other polyQ-containing proteins to form large amyloid-like structures. The binding and sequestration of these key proteins would lead to the losses of their physiological functions, resulting in dysregulation of a cascade of molecular and cellular events. The second is toxic-gain of RNA function. Upon transcription, rCAG^{exp} adopts an imperfect hairpin structure that sequesters the muscleblind-like protein 1 (MBNLI), an alternative RNA-splicing regulator, and other key proteins. While the normal rCAG-repeats can also adopt a hairpin motif, it does so in a different sequence context from the expanded version. Association of rCAG^{exp} with MBNL 1 results in a complex that is trapped in the nucleus as nuclear foci, precluding its export to the cytoplasm for the production of Htt protein. The gene transcripts that are mis-spliced as the result of the loss of MBNLI are diverse. The third pathogenic mechanism is protein toxicity. It has been shown that rCAGrepeats beyond a certain length (>42 units) can be translated in the absence of an ATG-starting codon *via* repeat-associated non-ATG (RAN) translation, leading to the production of toxic polyQ and polyalanine (poly A) proteins, along with polyserine (polyS). The latter two mechanisms are further supported by the findings that expression of a long tract of untranslated rCAG-repeats is deleterious in animal models, with abnormal behavioral phenotypes similar to that of HD.

Collectively, these findings point to HD as a multivariate disorder caused by toxic-gain of RNA and protein functions. As such, one potential strategy to remedy HD would be to selectively target the expanded transcript, as this would interfere with all three disease pathways. Described herein are bivalent nucleic acid ligands for recognition of rCAG repeats and other RNA-repeated sequences.

Thadke et al. ("Shape selective bifacial recognition of double helical DNA", Communications chemistry, vol. 1(1), 2018) describes bifacial nucleic acid recognition elements for targeting double stranded DNA. Thadke et al. ("Design of Bivalent Nucleic Acid Ligands for Recognition of RNA-Repeated Expansion Associated with Huntington's Disease", Biochemistry, vol. 57(14): 2094-2108, 2018) describes nucleic acid ligands consisting of Janus bases and the MPγPNA backbone for targeting rCAG-repeats. WO 2014/169206 A concerns divalent nucleobases for detection of a target sequence in a nucleic acid sequences or for the isolation and purification of a nucleic acid containing a target sequence. WO 2018/058091 A describes nucleobases to target double-stranded nucleic acid sequences and bring together mismatched sequences.

### SUMMARY

The present invention is defined in the claims.

In one aspect, a genetic recognition reagent is provided. The reagent comprises
(a) a nucleic acid analog backbone comprising (i) three or more nucleic acid analog backbone residues, (ii) a first end and a second end, and (iii) a first concatenating group attached to the first end of the backbone and a second concatenating group attached to the second end, either binding non-covalently or self-ligating with the first concatenating group,
   wherein the first concatenating group and the second concatenating group are each independently a two- to five-ring fused polycyclic aromatic moiety that is xanthene, riboflavin, mangostin, or mangiferin that stack with an aryl moiety of an adjacent recognition reagent when recognition reagents are hybridized to adjacent sequences of a target nucleic acid,(b) a sequence of bivalent nucleobases attached to the three or more nucleic acid analog backbone residues, where the sequence of bivalent nucleobases binds to a unit target sequence, or one or more sequential iterations of a unit target sequence of an expanded repeat of a repeat expansion disease on two nucleic acid strands, wherein the sequence of bivalent nucleobases comprises a unit recognition reagent sequence that is
      3-6-4,
      6-4-3,
      4-3-6,
      4-6-3,
      6-3-4,
      or 3-4-6,
      wherein:
   each 3 is independently 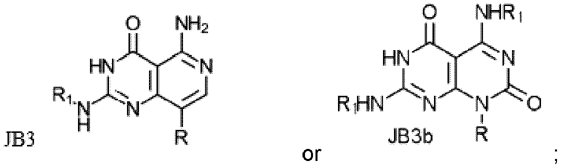
   each 4 is independently 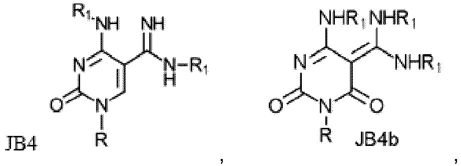 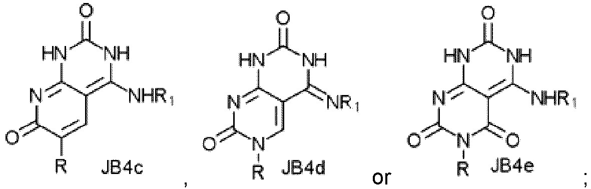
   each 6 is independently 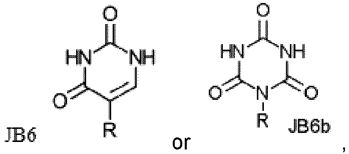
   wherein R is a nucleic acid analog backbone residue,
   wherein and R₁ is a protecting group or H.

In another aspect but not part of the invention defined in the claims, also provided is a method of binding a nucleic acid containing an expanded repeat associated with an expanded repeat disease. The method comprises contacting a nucleic acid comprising the expanded repeat with a genetic recognition reagent comprising a nucleic acid or nucleic acid analog backbone comprising three or more ribose, deoxyribose, or nucleic acid analog backbone residues, a sequence of bivalent nucleobases attached to the backbone residues, where the sequence of bivalent nucleobases bind to a unit target sequence, or one or more sequential iterations of a unit target sequence of an expanded repeat of an repeat expansion disease on two nucleic acid strands.

In yet another aspect but not part of the invention defined in the claims, a method of identifying the presence of a nucleic acid containing an expanded repeat associated with an expanded repeat disease in a sample obtained from a patient is provided. The method comprises contacting a nucleic acid sample obtained from the patient with a genetic recognition reagent comprising a nucleic acid or nucleic acid analog backbone comprising three or more ribose, deoxyribose, or nucleic acid analog backbone residues, a sequence of bivalent nucleobases attached to the backbone residues, where the sequence of bivalent nucleobases bind to a unit target sequence, or one or more sequential iterations of a unit target sequence of an expanded repeat of an repeat expansion disease on two nucleic acid strands, and determining if binding and concatenation of the genetic recognition reagent occurs as indicating that the sample comprises a nucleic acid containing an expanded repeat associated with an expanded repeat disease, and, optionally treating the patient for the expanded repeat disease. The nucleic acid or nucleic acid analog backbone comprises a first end and a second end, and further comprises a first concatenating group attached to the first end of the backbone, a second concatenating group attached to the second end, either binding non-covalently or self-ligating with the first concatenating group.

In yet another embodiment but not part of the invention defined in the claims, a method of knocking down expression of a gene containing an expanded repeat associated with an expanded repeat disease in a cell, is provided. The method comprises contacting a nucleic acid, such as an RNA, comprising the expanded repeat with a genetic recognition reagent comprising a nucleic acid or nucleic acid analog backbone comprising three or more ribose, deoxyribose, or nucleic acid analog backbone residues, a sequence of bivalent nucleobases attached to the backbone residues, where the sequence of bivalent nucleobases bind to a unit target sequence, or one or more sequential iterations of a unit target sequence of an expanded repeat of an repeat expansion disease on two nucleic acid strands.

### BRIEF DESCRIPTION OF THE DRAWINGS

Figure 1 (A) (Figure 1, panel (A)) The design of JB-MPγPNA ligands LG 1, LG2, and LG3 for targeting rCAG^{exp}-hairpin structure. K: L-lysine, arrow: N-terminus. (B) A representative binding mode of LG2 in the preferred anti parallel orientation (N-terminus facing 3'-end of the Watson-strand). (C) H-bonding interactions of E, I, and F with the respective C-G, A-A, and G-C base pairs of RNA. (D) The chemical structure of MPγPNA backbone to which J-bases were covalently attached. (E) Supposition of γPNAs and as γPNA-DNA duplex and PNA [as PNA-DNA duplex], determined by NMR and X-ray.
Figure 2, panels A-F provide exemplary structures of nucleic acid analogs.
Figure 3 depicts various examples of amino acid side chains.
Figure 4 provides structures of exemplary nucleobases.
Figures 5A-5C provide structures of exemplary bivalent nucleobases, with R referring to a nucleotide or nucleotide analog backbone monomer or residue.

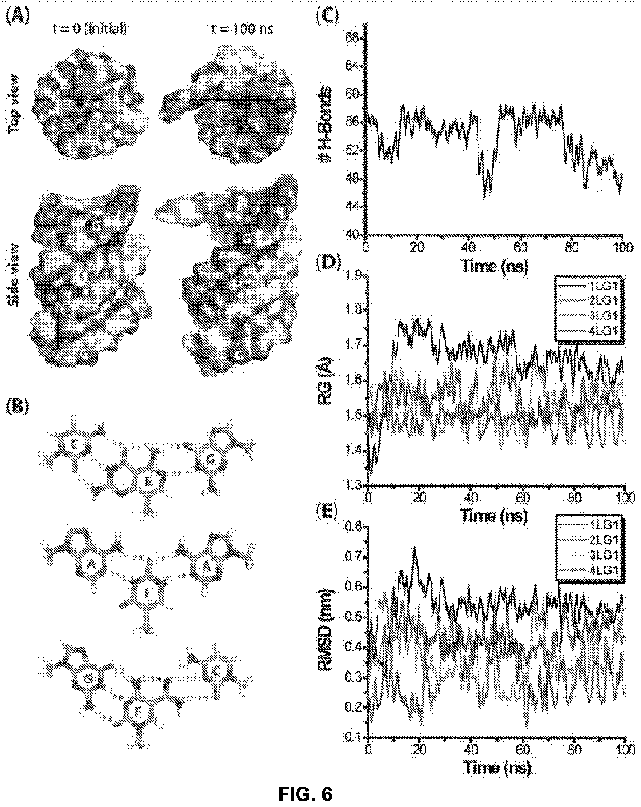

Figure 6. The results of MD simulations of LG 1 binding to an RNA duplex containing the sequence r(CAG)₄/r(CAG)₄. LG1: NH₂-EIF-H, with the γ-sidechain being a methyl group. (A) Surface representation of the bound complex with four separate LG1 ligands and an RNA duplex containing four CAG-repeats before (t=0) and after (t=I00 ns) the simulation. (B) H-bonding interaction of the CEG, AJA, and GFC triads after the simulation. (C) Number of H-bonds per triad between the RNA bases and J-bases of LG1, (D) radius of gyration of LG I-RNA complex, and (E) root-mean square deviation (RMSD) of the LGI-RNA complex with respect to the initial structure.
Figure 7 shows structures of the E, I, and F JB-MPγPNA monomers.
Figures 8 and 9 show synthesis schemes for E (15) and F (26), respectively.
Figure 10 shows a scheme for coupling of E (15), F (26), and I (27) to the MPγPNA backbone.
Figure 11 shows model RNA targets chosen for binding the study described in the example (SEQ ID NO: 1).
Figure 12. CD titration of LG2 with R11A. The concentration of R11A was 0.5 µM and those of LG2 were X: 0, 1.5, 2.5, 3.5, 4.5, 5.5, 6.5, 8.5, and 11.5 µM. After the addition of LG2 to R11A, the mixtures were incubated at 37 °C for 30 min prior to the acquisition of data at 25 °C.
Figure 13. CD spectra of LG2 with the (A) mismatched R11U, (B) single-stranded WS and CS, and (C) double-stranded HP containing a single binding-site, as well as that of (D) LG2P with R11A. CD spectra of RNAs (line) and that of the [RNA+ligand] mixtures (dash).
Figure 14. The effects of ligand orientation (LG2P), mismatched R11U and single-stranded targets (WS+CS). Inset: Fluorescent spectra of the corresponding samples after addition of 2.5 µM of pentamidine.
Figure 15. NMR titration of LG2 with R11A. The concentration of R11A was 0.1 mM. Aliquots of LG2 at the indicated molar ratios were added to R11A and incubated at 37 °C for 15 min prior to data acquisition. Solvent-exchangeable protons are indicated by dashed lines, and nonexchangeable protons by vertical solid lines.
Figure 16 schematically shows native chemical ligation, as described in the examples.
Figure 17. Reaction progress of the parent compound M following the addition of 4MP at 37 °C. (A) An outline of the reaction sequence. The M# and M## intermediates were initially formed, and then converted into the reactive LG2N* intermediate and finally to the cyclic product cLG2N. (B) MALDI-TOF MS spectra of M following the addition of 4MP and incubation at 37 °C for 0, 15, and 60 min. The samples were prepared in 0.1 X PBS buffer.
Figure 18. MALDI-TOF spectra of (A) [LG2N+R11A], (B) [LG2N+R11U], and (C) LG2N alone following the incubation with 2ME at 3 7 °C for 16 h. Prior to MS analysis, the samples were reconstituted with 4 mM guanidinium chloride and heat-denatured at 95 °C for 5 min. The concentrations of LG2N, R11A, R11U, and 2ME were 22, 1, 1, and 500 µM, respectively. In the positive mode, RNA molecules were not observed in the MALDI-TOF spectra. The Y-axes in (B) and (C) are that same as that of (A); they were omitted to save space.
Figure 19. Competitive binding assay. The concentrations of R24A, R127A, and R96U were 100, 18, and 22 nM, with each containing 1.1 µM binding-sites; of LG2N in lanes 2-6, and 8 were 11, 22, 44, 88, 88, and 88 µM; and of 4MP and TCEP were 500 and 100 µM, in that respective order. The ratios of binding-site to ligand were as indicated. The samples were prepared in 0.1X PBS buffer and incubated at 37°C for 24h prior to separation by 2% agarose-gel and stained with SYBR-Gold.

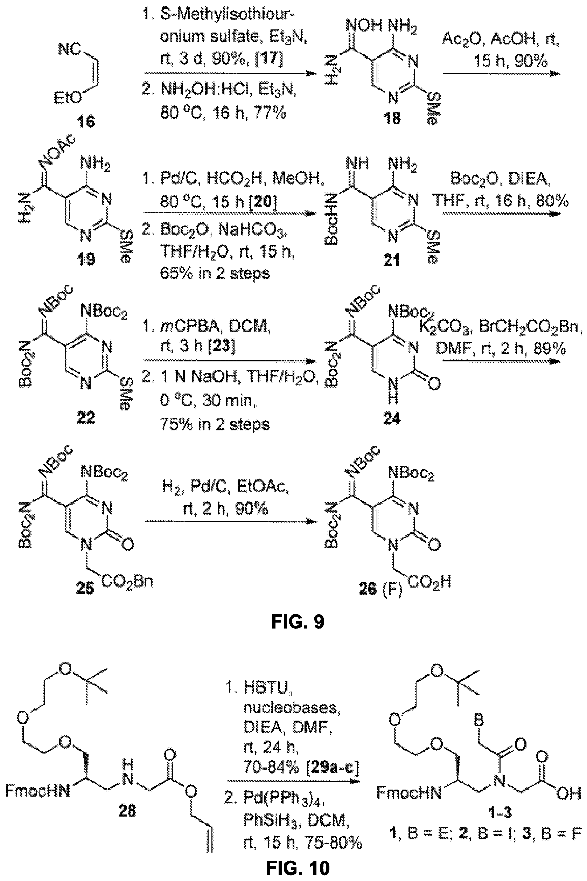

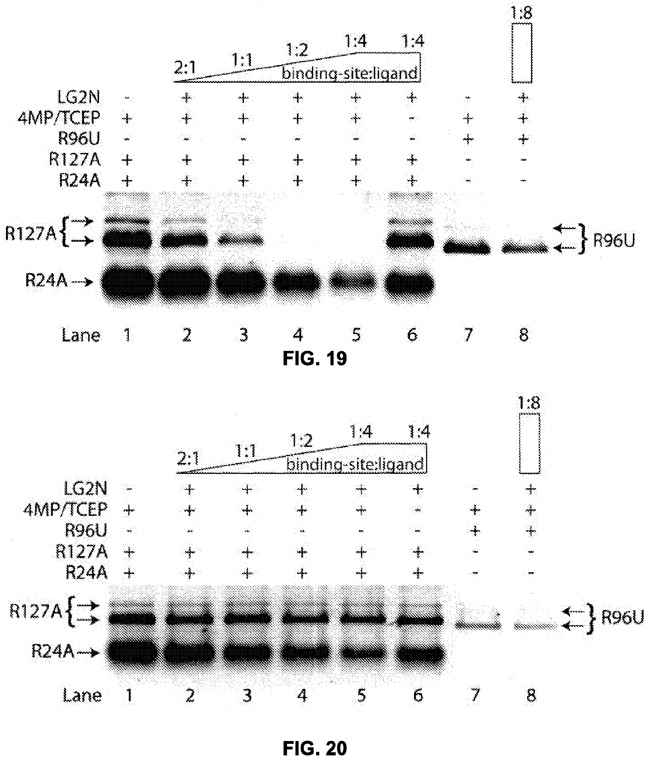

Figure 20. Gel-shift assay at a physiologically simulated ionic strength. The samples were prepared in the same way as that shown in Figure 9 except that the buffer was PS (10 mM NaPi, 150 mM KCI, 2 mM MgCl₂ pH 7.4). The concentrations of R24A, R127A, and R96U were 100, 18, and 22 nM, with each containing 1.1 µM binding-sites; ofLG2N in lanes 2-6, and 8 were 11, 22, 44, 88, 88 and 88 µM; and of 4MP and TCEP were 500 and 100 µM, in that respective order. The ratios of binding-sites to ligand were as indicated. The samples were incubated at 37 °C for 24 h prior to separation by 2% agarose-gel and stained with SYBR-Gold.
Figure 21 shows a general method of synthesizing the ligands described herein.

### DETAILED DESCRIPTION

**The** use of numerical values in the various ranges specified in this application, unless expressly indicated otherwise, are stated as approximations as though the minimum and maximum values within the stated ranges are both preceded by the word "about". In this manner, slight variations above and below the stated ranges can be used to achieve substantially the same results as values within the ranges. Also, unless indicated otherwise, the disclosure of ranges is intended as a continuous range including every value between the minimum and maximum values. As used herein "a" and "an" refer to one or more.

**As** used herein, the term "comprising" is open-ended and may be synonymous with "including", "containing", or "characterized by". The term "consisting essentially of" limits the scope of a claim to the specified materials or steps and those that do not materially affect the basic and novel characteristic(s) of the claimed invention. The term "consisting of" excludes any element, step, or ingredient not specified in the claim. As used herein, embodiments "comprising" one or more stated elements or steps also include but are not limited to embodiments "consisting essentially of" and "consisting of" these stated elements or steps.

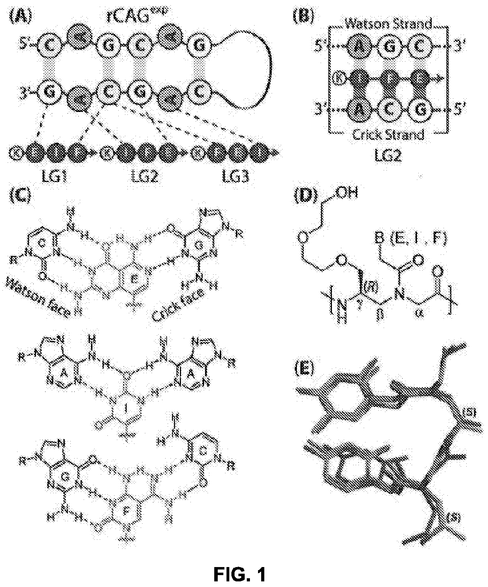

Provided herein are compositions and methods for binding target sequences in nucleic acids, for example for binding repeat expansions associated with diseases involving repeat expansions of nucleic acid sequences. Figure 1 is a schematic diagram illustrating the binding of a non-limiting example of the recognition reagents (genetic recognition reagents, ligands) described herein, targeting CAG repeats in RNA hairpin structures as seen in HD and other PolyQ diseases. Cooperative binding of modules to adjacent modules is facilitated by terminal self-ligating groups or pi-stacking aromatic groups, such as the exemplary self-ligating groups described in the Example, below, and as more broadly disclosed in United States Patent Application Publication No. 2016/0083433. Collectively, self-ligating groups, pi-stacking groups, and similar groups that either covalently link or non-covalently bind adjacent recognition reagents on a strand of a nucleic acid, or between two nucleic acid strands in the case of a recognition reagent comprising bivalent nucleobases, are collectively referred to herein as "concatenating groups". The plurality of recognition reagents bind (hybridize) by Watson-Crick or Watson-Crick-like cooperative base pairing to a template nucleic acid. In a cell a template nucleic acid is an RNA or DNA molecule, though in vitro, a template nucleic acid can be any RNA or DNA, as well as a modified nucleic acid or a nucleic acid analog. Recognition reagents in sufficient proximity, for example binding to adjacent sequences on a template nucleic acid, will self ligate or π-π stack, thus concatenating to essentially form a longer oligomer or polymer. Further details are provided below.

As used herein, a "patient" is an animal, such as a mammal, including a primate (such as a human, a non-human primate, e.g. , a monkey, and a chimpanzee), a non-primate (such as a cow, a pig, a camel, a llama, a horse, a goat, a rabbit, a sheep, a hamster, a guinea pig, a cat, a dog, a rat, a mouse, a horse, and a whale), or a bird (e.g., a duck or a goose).

As used herein, the terms "treating", or "treatment" refer to a beneficial or desired result, such as improving one of more, or symptoms of a disease. The terms "treating" or "treatment" also include, but are not limited to, alleviation or amelioration of one or more symptoms of a repeat expansion disease, such as a PolyQ repeat expansion disease, e.g., Huntington's Disease. "Treatment" can also mean to prolonging survival as compared to expected survival in the absence of treatment.

By "lower" in the context of a disease marker or symptom is meant a clinically-relevant and/or a statistically significant decrease in such level. The decrease can be, for example, at least 10%, at least 20%, at least 30%, at least 40%, or more, down to a level accepted as within the range of normal for an individual without such disorder, or to below the level of detection of the assay. In certain aspects, the decrease is down to a level accepted as within the range of normal for an individual without such disorder which can also be referred to as a normalization of a level. In certain aspects, the reduction is the normalization of the level of a sign or symptom of a disease, a reduction in the difference between the subject level of a sign of the disease and the normal level of the sign for the disease (e.g., to the upper level of normal when the value for the subject must be decreased to reach a normal value, and to the lower level of normal when the value for the subject must be increased to reach a normal level). In certain aspects, the methods include a clinically relevant inhibition of expression of a mRNA of a PolyQ repeat expansion disease, such as Huntington's Disease, e.g. as demonstrated by a clinically relevant outcome after treatment of a subject with a recognition reagent as described herein.

"Therapeutically effective amount," as used herein, is intended to include the amount of a recognition reagent as described herein that, when administered to a subject having a disease, is sufficient to effect treatment of the disease (e.g., by diminishing, ameliorating or maintaining the existing disease or one or more symptoms of disease). The "therapeutically effective amount" may vary depending on the recognition reagent (agent), how the agent is administered, the disease and its severity and the history, age, weight, family history, genetic makeup, the types of preceding or concomitant treatments, if any, and other individual characteristics of the subject to be treated. In the context of the genetic recognition reagent described herein, an exemplary dosage range, per dose, ranges from 0.1 pg to 1 mg.

A "therapeutically-effective amount" also includes an amount of an agent that produces some desired local or systemic effect at a reasonable benefit/risk ratio applicable to any treatment. Recognition reagent agents employed in the methods described herein may be administered in a sufficient amount to produce a reasonable benefit/risk ratio applicable to such treatment.

The phrase "pharmaceutically-acceptable carrier" as used herein means a pharmaceutically-acceptable material, composition or vehicle, such as a liquid or solid filler, diluent, excipient, manufacturing aid (e.g., lubricant, talc magnesium, calcium or zinc stearate, or steric acid), or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the subject being treated. Some examples of materials which can serve as pharmaceutically-acceptable carriers include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) lubricating agents, such as magnesium state, sodium lauryl sulfate and talc; (8) excipients, such as cocoa butter and suppository waxes; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; (16) pyrogen-free water; (17) isotonic saline; (18) Ringer's solution; (19) ethyl alcohol; (20) pH buffered solutions; (21) polyesters, polycarbonates and/or polyanhydrides; (22) bulking agents, such as polypeptides and amino acids (23) serum component, such as serum albumin, HDL and LDL; and (22) other non-toxic compatible substances employed in pharmaceutical formulations.

**As** used herein, the terms "cell" and "cells" refer to any types of cells from any animal, such as, without limitation, rat, mice, monkey, and human. For example and without limitation, cells can be progenitor cells, such as stem cells, or differentiated cells, such as endothelial cells, smooth muscle cells.

By "expression" or "gene expression," it is meant the overall flow of information from a gene (without limitation, a functional genetic unit for producing a gene product, such as RNA or a protein in a cell, or other expression system encoded on a nucleic acid and comprising: a transcriptional promoter and other cis-acting elements, such as response elements and/or enhancers; an expressed sequence that typically encodes a protein (open-reading frame or ORF) or functional/structural RNA, and a polyadenylation sequence), to produce a gene product (typically a protein, optionally post-translationally modified or a functional/structural RNA). By "expression of genes under transcriptional control of," or alternately "subject to control by," a designated sequence, it is meant gene expression from a gene containing the designated sequence operably linked (functionally attached, typically *in cis*) to the gene. The designated sequence may be all or part of the transcriptional elements (without limitation, promoters, enhancers and response elements), and may wholly or partially regulate and/or affect transcription of a gene. A "gene for expression of" a stated gene product is a gene capable of expressing that stated gene product when placed in a suitable environment--that is, for example, when transformed, transfected, transduced, etc. into a cell, and subjected to suitable conditions for expression. In the case of a constitutive promoter "suitable conditions" means that the gene typically need only be introduced into a host cell. In the case of an inducible promoter, "suitable conditions" means when an amount of the respective inducer is administered to the expression system (e.g., cell) effective to cause expression of the gene.

**As** used herein, the term "knockdown" or "knocking down" means that expression of one or more genes in an organism is reduced, typically significantly, with respect to a functional gene, such as to a therapeutically-effective degree. Gene knockdown also includes complete gene silencing. As used herein, "gene silencing" means that expression of a gene is essentially completely prevented. Knockdown and gene silencing may occur either at the transcriptional stage or the translational stage. Use of the described recognition reagents to target an RNA in a cell, such as an mRNA, can modify gene expression, by knocking down or silencing a gene or genes at the post-transcriptional or translational stage.

As used herein, the term "nucleic acid" refers to deoxyribonucleic acids (DNA) and ribonucleic acids (RNA). Nucleic acid analogs include, for example and without limitation: 2'-O-methyl-substituted RNA, locked nucleic acids, unlocked nucleic acids, triazole-linked DNA, peptide nucleic acids, morpholino oligomers, dideoxynucleotide oligomers, glycol nucleic acids, threose nucleic acids and combinations thereof including, optionally ribonucleotide or deoxyribonucleotide residue(s). Herein, "nucleic acid" and "oligonucleotide" which is a short, single-stranded structure made of up nucleotides, in reference to nucleic acids and nucleic acid analogs, are used interchangeably. An oligonucleotide may be referred to by the length (i.e. number of nucleotides) of the strand, through the nomenclature "-mer". For example, an oligonucleotide of 22 nucleotides would be referred to as a 22-mer.

A "nucleic acid analog" is a composition comprising a sequence of nucleobases arranged on a substrate, such as a polymeric backbone, and can bind DNA and/or RNA by hybridization by Watson-Crick, or Watson-Crick-like hydrogen bond base pairing. Non-limiting examples of common nucleic acid analogs include peptide nucleic acids, such as γPNA, morpholino nucleic acids, phosphorothioates, locked nucleic acid (2'-O-4'-C-methylene bridge, including oxy, thio or amino versions thereof), unlocked nucleic acid (the C2'-C3' bond is cleaved), 2'-O-methyl-substituted RNA, threose nucleic acid, glycol nucleic acid, *etc.*

A conformationally preorganized nucleic acid analog is a nucleic acid analog that has a backbone (a preorganized backbone) that forms only either a right-handed helix or a left-handed helix, depending on the structure of the nucleic acid backbone. As shown herein, one example of a conformationally preorganized nucleic acid analog is γPNA, which has a chiral center at the γ carbon, and, depending on, and due to, the chirality of the groups at the γ carbon, forms only a right-handed helix or a left-handed helix. Likewise, locked nucleic acid, comprising a ribose with a bridge between the 2' oxygen and the 4' carbon, that "locks" the ribose into a 3'-endo (North) conformation.

In the context of the present disclosure, a "nucleotide" refers to a monomer comprising at least one nucleobase and a backbone element (backbone moiety or residue), which in a nucleic acid, such as RNA or DNA is ribose or deoxyribose. "Nucleotides" also typically comprise reactive groups that permit polymerization under specific conditions. In native DNA and RNA, those reactive groups are the 5' phosphate and 3' hydroxyl groups. For chemical synthesis of nucleic acids and analogs thereof, the bases and backbone monomers may contain modified groups, such as blocked amines, as are known in the art. A "nucleotide residue" refers to a single nucleotide that is incorporated into an oligonucleotide or polynucleotide. Likewise, a "nucleobase residue" refers to a nucleobase incorporated into a nucleotide or a nucleic acid or analog thereof. A "genetic recognition reagent" refers generically to a nucleic acid or a nucleic acid analog that comprises a sequence of nucleobases that can hybridize (bind) to a complementary nucleic acid or nucleic acid analog sequence on a nucleic acid by cooperative base pairing, *e.g.*, Watson-Crick base pairing or Watson-Crick-like base pairing.

In further detail, nucleotides, for either RNA, DNA, or nucleic acid analogs, have the structure A-B wherein A is a backbone monomer moiety and B is a nucleobase as described herein. The backbone monomer can be any suitable nucleic acid backbone monomer, such as a ribose triphosphate or deoxyribose triphosphate, or a monomer of a nucleic acid analog, such as peptide nucleic acid (PNA), such as a gamma PNA (γPNA). In one example the backbone monomer is a ribose mono-, di-, or tri-phosphate or a deoxyribose mono-, di-, or tri-phosphate, such as a 5' monophosphate, diphosphate, or triphosphate of ribose or deoxyribose. The backbone monomer includes both the structural "residue" component, such as the ribose in RNA, and any active groups that are modified in linking monomers together, such as the 5' triphosphate and 3' hydroxyl groups of a ribonucleotide, which are modified when polymerized into RNA to leave a phosphodiester linkage. Likewise for PNA, the C-terminal carboxyl and N-terminal amine active groups of the N-(2-aminoethyl)glycine backbone monomer are condensed during polymerization to leave a peptide (amide) bond. In another aspect, the active groups are phosphoramidite groups useful for phosphoramidite oligomer synthesis, as is broadly-known in the arts. The nucleotide also optionally comprises one or more protecting groups as are known in the art, such as 4,4'-dimethoxytrityl (DMT), and as described herein. Additional methods of preparing synthetic genetic recognition reagents are known and depend on the backbone structure and particular chemistry of the base addition process. Determination of which active groups to utilize in joining nucleotide monomers and which groups to protect in the bases, and the required steps in preparation of oligomers is well within the abilities of those of ordinary skill in the chemical arts and in the field of nucleic acid and nucleic acid analog oligomer synthesis.

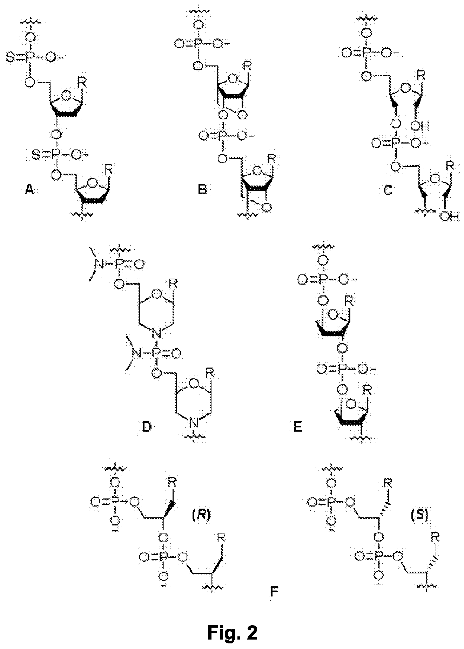

Non-limiting examples of common nucleic acid analogs include peptide nucleic acids, such as γPNA, phosphorothioate (e.g., Figure 2 (A)), locked nucleic acid (2'-O-4'-C-methylene bridge, including oxy, thio or amino versions thereof, e.g., Figure 2(B)), unlocked nucleic acid (the C2'-C3' bond is cleaved, e.g., Figure 2(C)), 2'-O-methyl-substituted RNA, morpholino nucleic acid (e.g., Figure 2(D)), threose nucleic acid (e.g., Figure 2(E)), glycol nucleic acid (e.g., Figure 2(F), showing *R* and *S* Forms), *etc.* Figure 2(A-F) shows monomer structures for various examples of nucleic acid analogs. Figure 2(A-F) each show two monomer residues incorporated into a longer chain as indicated by the wavy lines. Incorporated monomers are referred to herein as "residues" and the part of the nucleic acid or nucleic acid analog excluding the nucleobases is referred to as the "backbone" of the nucleic acid or nucleic acid analog. As an example, for RNA, an exemplary nucleobase is adenine, a corresponding monomer is adenosine triphosphate, and the incorporated residue is an adenosine monophosphate residue. For RNA, the "backbone" consists of ribose subunits linked by phosphates, and thus the backbone monomer is ribose triphosphate prior to incorporation and a ribose monophosphate residue after incorporation. Like γPNA, Locked Nucleic Acid (Figure 2(B)) is conformationally preorganized.

A "moiety" is a part of a molecule, and includes as a class "residues", which are the portion of a compound or monomer that remains in a larger molecule, such as a polymer chain, after incorporation of that compound or monomer into the larger molecule, such as a nucleotide as-incorporated into a nucleic acid or an amino acid as-incorporated into a polypeptide or protein.

The term "polymer composition" is a composition comprising one or more polymers. As a class, "polymers" includes, without limitation, homopolymers, heteropolymers, co-polymers, block polymers, block co-polymers and can be both natural and synthetic. Homopolymers contain one type of building block, or monomer, whereas co-polymers contain more than one type of monomer. An "oligomer" is a polymer that comprises a small number of monomers, such as, for example, from 3 to 100 monomer residues. As such, the term "polymer" includes oligomers. The terms "nucleic acid" and "nucleic acid analog" includes nucleic acid and nucleic acid polymers and oligomers.

A polymer "comprises" or is "derived from" a stated monomer if that monomer is incorporated into the polymer. Thus, the incorporated monomer that the polymer comprises is not the same as the monomer prior to incorporation into a polymer, in that at the very least, certain linking groups are incorporated into the polymer backbone or certain groups are removed in the polymerization process. A polymer is said to comprise a specific type of linkage if that linkage is present in the polymer. An incorporated monomer is a "residue". A typical monomer for a nucleic acid or nucleic acid analog is referred to as a nucleotide.

By "non-reactive", in the context of a chemical constituent, such as a molecule, compound, composition, group, moiety, ion, etc. it is meant that the constituent does not react with other chemical constituents in its intended use to any substantial extent. The non-reactive constituent is selected to not interfere, or to interfere insignificantly, with the intended use of the constituent, moiety, or group as a recognition reagent. In the context of the linker moieties described herein, they are non-reactive in that they do not interfere with the binding of the recognition reagents to a target template, and do not interfere with concatenation of the recognition reagents on the target template.

**As** used herein, "alkyl" refers to straight, branched chain, or cyclic hydrocarbon groups including, for example, from 1 to about 20 carbon atoms, for example and without limitation C₁₋₃, C₁₋₆, C₁₋₁₀ groups, for example and without limitation, straight, branched chain alkyl groups such as methyl, ethyl, propyl, butyl, pentyl, hexyl, heptyl, octyl, nonyl, decyl, undecyl, dodecyl, and the like. "Substituted alkyl" refers to alkyl substituted at 1 or more, e.g., 1, 2, 3, 4, 5, or even 6 positions, which substituents are attached at any available atom to produce a stable compound, with substitution as described herein. "Optionally substituted alkyl" refers to alkyl or substituted alkyl. "Halogen," "halide," and "halo" refers to -F, -Cl, -Br, and/or -I. "Alkylene" and "substituted alkylene" refer to divalent alkyl and divalent substituted alkyl, respectively, including, without limitation, methylene, ethylene, trimethylene, tetramethylene, pentamethylene, hexamethylene, hepamethylene, octamethylene, nona methylene, or decamethylene. "Optionally substituted alkylene" refers to alkylene or substituted alkylene.

"Alkene or alkenyl" refers to straight, branched chain, or cyclic hydrocarbyl groups including, e.g., from 2 to about 20 carbon atoms, such as, without limitation C₁₋₃, C₁₋₆, C₁₋₁₀ groups having one or more, e.g., 1, 2, 3, 4, or 5, carbon-to-carbon double bonds. "Substituted alkene" refers to alkene substituted at 1 or more, e.g., 1, 2, 3, 4, or 5 positions, which substituents are attached at any available atom to produce a stable compound, with substitution as described herein. "Optionally substituted alkene" refers to alkene or substituted alkene. Likewise, "alkenylene" refers to divalent alkene. Examples of alkenylene include without limitation, ethenylene (-CH=CH-) and all stereoisomeric and conformational isomeric forms thereof. "Substituted alkenylene" refers to divalent substituted alkene. "Optionally substituted alkenylene" refers to alkenylene or substituted alkenylene.

"Alkyne or "alkynyl" refers to a straight or branched chain unsaturated hydrocarbon having the indicated number of carbon atoms and at least one triple bond. Examples of a (C₂-C₈)alkynyl group include, but are not limited to, acetylene, propyne, 1-butyne, 2-butyne, 1-pentyne, 2-pentyne, 1-hexyne, 2-hexyne, 3-hexyne, 1-heptyne, 2-heptyne, 3-heptyne, 1-octyne, 2-octyne, 3-octyne and 4-octyne. An alkynyl group can be unsubstituted or optionally substituted with one or more substituents as described herein below. The term "alkynylene" refers to divalent alkyne. Examples of alkynylene include without limitation, ethynylene, propynylene. "Substituted alkynylene" refers to divalent substituted alkyne.

**The** term "alkoxy" refers to an -O-alkyl group having the indicated number of carbon atoms. For example, a (C₁-C₆)alkoxy group includes -O-methyl (methoxy), -O-ethyl (ethoxy), -O-propyl (propoxy), -O-isopropyl (isopropoxy), -O-butyl (butoxy), -O-sec -butyl (sec-butoxy), -O-tert-butyl (tert-butoxy), -O-pentyl (pentoxy), -O-isopentyl (isopentoxy), -O-neopentyl (neopentoxy), -O-hexyl (hexyloxy), -O-isohexyl (isohexyloxy), and -O-neohexyl (neohexyloxy). "Hydroxyalkyl" refers to a (C₁-C₁₀)alkyl group wherein one or more of the alkyl group's hydrogen atoms is replaced with an -OH group. Examples of hydroxyalkyl groups include, but are not limited to, -CH₂OH, -CH₂CH₂OH, -CH₂CH₂CH₂OH, -CH₂CH₂CH₂CH₂OH, - CH₂CH₂CH₂CH₂CH₂OH, -CH₂CH₂CH₂CH₂CH₂CH₂OH, and branched versions thereof. The term "ether" or "oxygen ether" refers to an alkyl group wherein one or more of the alkyl group's carbon atoms is replaced with an -O- group. The term ether includes -CH₂-(OCH₂-CH₂)_{q}OP₁ compounds where P₁ is a protecting group, -H, or a (C₁-C₁₀)alkyl. Exemplary ethers include polyethylene glycol, diethylether, methylhexyl ether and the like.

"Heteroatom" refers to N, O, P and S. Compounds that contain N or S atoms can be optionally oxidized to the corresponding N-oxide, sulfoxide or sulfone compounds. "Hetero-substituted" refers to an organic compound in any embodiment described herein in which one or more carbon atoms are substituted with N, O, P or S.

"Aryl," alone or in combination refers to an aromatic ring system such as phenyl or naphthyl. "Aryl" also includes aromatic ring systems that are optionally fused with a cycloalkyl ring. A "substituted aryl" is an aryl that is independently substituted with one or more substituents attached at any available atom to produce a stable compound, wherein the substituents are as described herein. "Optionally substituted aryl" refers to aryl or substituted aryl. "Arylene" denotes divalent aryl, and "substituted arylene" refers to divalent substituted aryl. "Optionally substituted arylene" refers to arylene or substituted arylene. As used herein, the term "polycyclic aryl group" and related terms, such as "polycyclic aromatic group" means a group composed of at least two fused aromatic rings. "Heteroaryl" or "hetero-substituted aryl" refers to an aryl group substituted with one or more heteroatoms, such as N, O, P, and/or S.

"Cycloalkyl" refer to monocyclic, bicyclic, tricyclic, or polycyclic, 3- to 14-membered ring systems, which are either saturated, unsaturated or aromatic. The cycloalkyl group may be attached via any atom. Cycloalkyl also contemplates fused rings wherein the cycloalkyl is fused to an aryl or hetroaryl ring. Representative examples of cycloalkyl include, but are not limited to cyclopropyl, cyclobutyl, cyclopentyl, and cyclohexyl. A cycloalkyl group can be unsubstituted or optionally substituted with one or more substituents as described herein below. "Cycloalkylene" refers to divalent cycloalkyl. The term "optionally substituted cycloalkylene" refers to cycloalkylene that is substituted with 1, 2 or 3 substituents, attached at any available atom to produce a stable compound, wherein the substituents are as described herein.

"Carboxyl" or "carboxylic" refers to group having the indicated number of carbon atoms, where indicated, and terminating in a -C(O)OH group, thus having the structure -R-C(O)OH, where R is a divalent organic group that includes linear, branched, or cyclic hydrocarbons. Non-limiting examples of these include: C₁₋₈ carboxylic groups, such as ethanoic, propanoic, 2-methylpropanoic, butanoic, 2,2-dimethylpropanoic, pentanoic, *etc.* "Amine" or "amino" refers to group having the indicated number of carbon atoms, where indicated, and terminating in a -NH₂ group, thus having the structure -R-NH₂, where R is a unsubstituted or unsubstituted divalent organic group that, e.g. includes linear, branched, or cyclic hydrocarbons, and optionally comprises one or more heteroatoms.

Terms combining the foregoing refer to any suitable combination of the foregoing, such as arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynylheteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl. As an example, "arylalkylene" refers to a divalent alkylene wherein one or more hydrogen atoms in an alkylene group is replaced by an aryl group, such as a (C₃-C₈)aryl group. Examples of (C₃-C₈)aryl-(C₁-C₆)alkylene groups include without limitation 1-phenylbutylene, phenyl-2-butylene, I-phenyl-2-methylpropylene, phenylmethylene, phenylpropylene, and naphthylethylene. The term "(C₃-C₈)cycloalkyl-(C₁-C₆)alkylene" refers to a divalent alkylene wherein one or more hydrogen atoms in the C₁-C₆ alkylene group is replaced by a (C₃-C₈)cycloalkyl group. Examples of (C₃-C₈)cycloalkyl-(C₁-C₆)alkylene groups include without limitation 1-cycloproylbutylene, cycloproyl-2-butylene, cyclopentyl-1 -phenyl-2-methylpropylene, cyclobutylmethylene and cyclohexylpropylene.

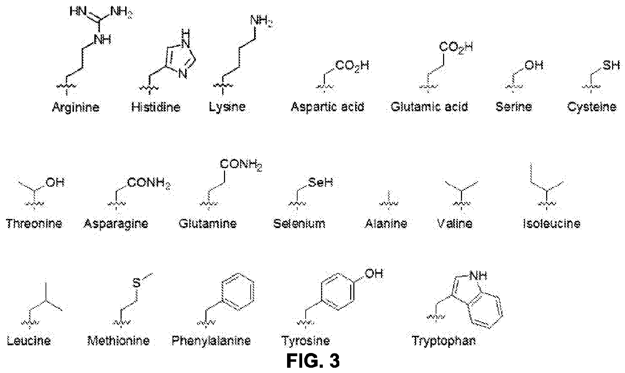

**An** "amino acid" has the structure H₂N-C(R)-C(O)OH, where R is a side chain, such as an amino acid side chain. An "amino acid residue" represents the remainder of an amino acid when incorporated into a chain of amino acids, such as when incorporated into a recognition reagent as discloses herein, e.g., having the structures -NH-C(R)-C(O)-, H₂N-C(R)-C(O)-(when at the N-terminus of a polypeptide), or -NH-C(R)-C(O)OH (when at the C-terminus of a polypeptide). An "amino acid side chain" is a side chain for an amino acid, including proteinogenic or non-proteinogenic amino acids. Amino acids have the structure: 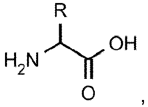 where R is the amino acid side chain. Non-limiting examples of amino acid side chains are shown in Figure 3. Glycine (H₂N-CH₂-C(O)OH) has no side chain.

A "peptide nucleic acid" refers to a nucleic acid analog, or DNA or RNA mimic, in which the sugar phosphodiester backbone of the DNA or RNA is replaced by a N-(2-aminoethyl)glycine unit. A gamma PNA (γPNA) is an oligomer or polymer of gamma-modified N-(2-aminoethyl)glycine monomers of the following structure: 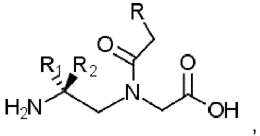 where at least one of R₁ or R₂ attached to the gamma carbon is not a hydrogen, such that the gamma carbon is a chiral center. When R₁ and R₂ are hydrogen (N-(2-aminoethyl)-glycine backbone, or the same, there is no such chirality about the gamma carbon. An incorporated PNA or γPNA monomer, 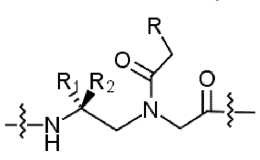 is referred to herein as a PNA or γPNA "residue", in reference to the remaining structure after integration into an oligomer, with each residue having the same or different R group as its base (nucleobase), such as adenine, guanine, cytosine, thymine and uracil bases, or other bases, such as the monovalent and divalent bases described herein, such that the order of bases on the PNA is its "sequence", as with DNA or RNA. A sequence of nucleobases in a nucleic acid or a nucleic acid analog oligomer or polymer, such as a PNA or γPNA oligomer or polymers, binds to a complementary sequence of adenine, guanine, cytosine, thymine and/or uracil residues in a nucleic acid or nucleic acid analog strand by nucleobase pairing, in a Watson-Crick or Watson-Crick-like manner, essentially as with double-stranded DNA or RNA.

A "guanidine" or "guanidinium" group may be added to the recognition reagent to increase solubility and/or bioavailability. Because PNA is produced in a similar manner to synthetic peptides, a simple way to add guanidine groups is to add one or more terminal arginine (Arg) residues to the N-terminal and/or C-terminal ends of the PNA, e.g. γPNA, recognition reagent. Likewise, an arginine side group, 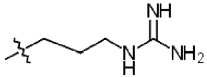 , or a guanidine-containing moiety, such as 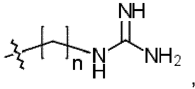 where n, for example and without limitation, ranges from 1-5, or a salt thereof, can be attached to a recognition reagent backbone as described herein. A guanidine-containing group is a group comprising a guanidine moiety, and may have less than 100 atoms, less than 50 atoms, e.g., less than 30 atoms. In one aspect, the guanidine-containing group has the structure: 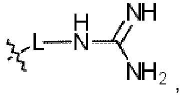 where L is a linker according to any aspect described herein, e.g., a non-reactive aliphatic hydrocarbyl linker, such as a methylene, ethylene, trimethylene, tetramethylene, or pentamethylene linker. In aspects the guanidine-containing group has the structure: 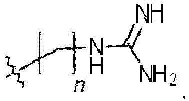 where n is 1-5, e.g., the guanidine group may be arginine.

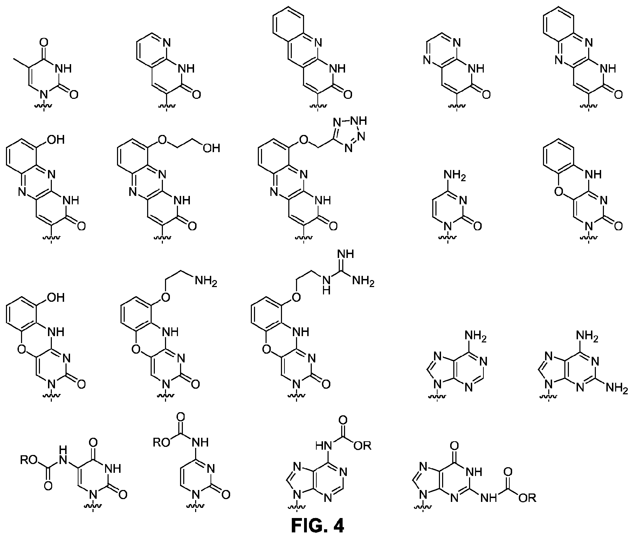

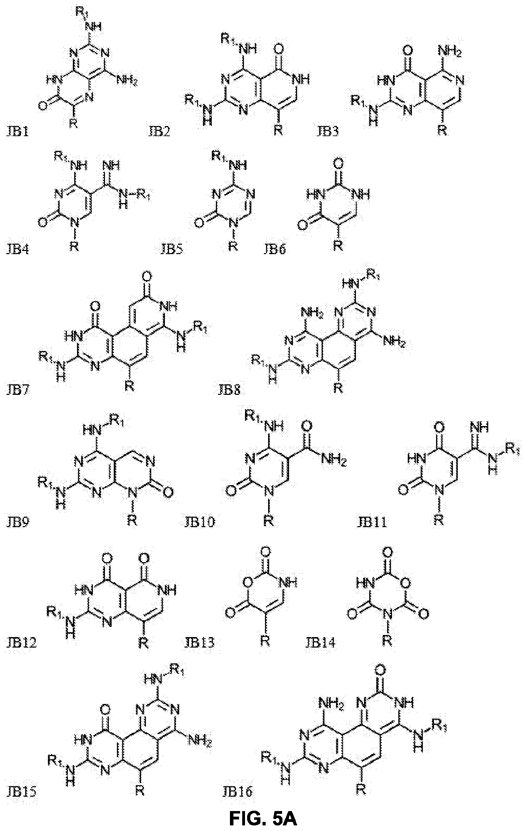

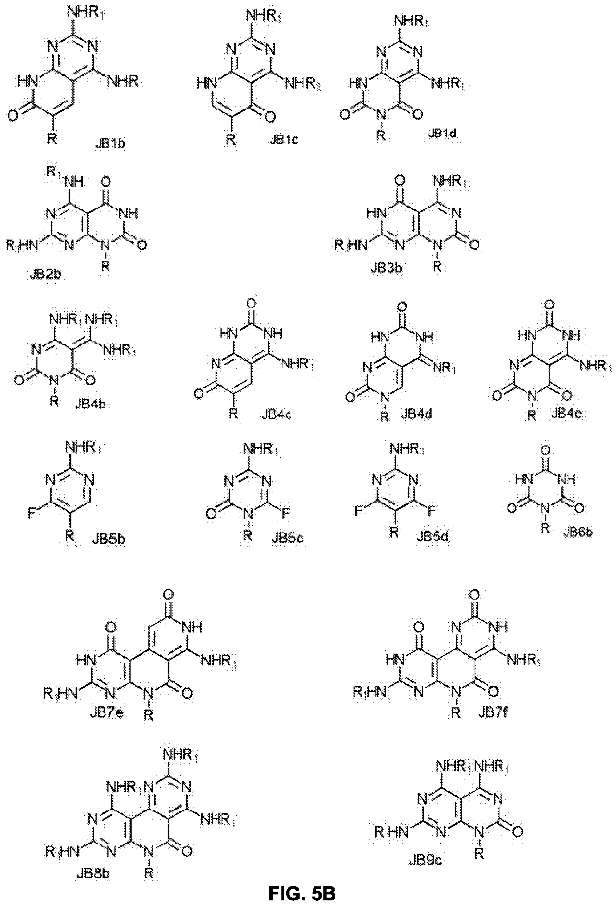

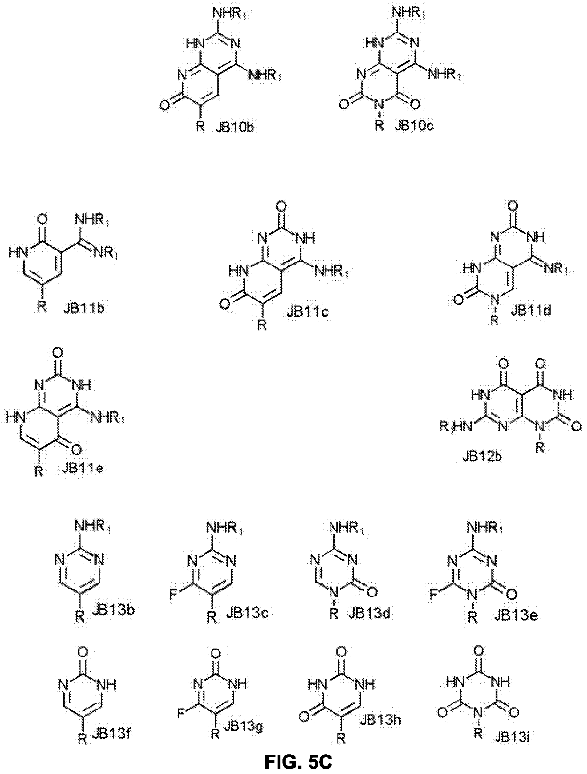

A "nucleobase" includes primary nucleobases: adenine, guanine, thymine, cytosine, and uracil, as well as modified purine and pyrimidine bases, such as, without limitation, hypoxanthine, xanthene, 7-methylguanine, 5, 6, dihydrouracil, 5-methylcytosine, and 5-hydroxymethylcytosine. Figures 4 and 5A-5C also depict non-limiting examples of nucleobases, including monovalent nucleobases (e.g., adenine, cytosine, guanine, thymine or uracil, which bind to one strand of nucleic acid or nucleic acid analogs), and divalent nucleobases (e.g., JB1-JB16 described herein) which bind complementary nucleobases on two strands of nucleic acid simultaneously, and "clamp" nucleobases, such as a "G-clamp," which binds complementary nucleobases with enhanced strength. Additional purine, purine-like, pyrimidine and pyrimidine-like nucleobases are known in the art, for example as disclosed in United States Patent Nos. 8,053,212, 8,389,703, and 8,653,254. For divalent nucleobases JB1-JB16, shown in Figure 5A, Table A shows the specificity of the different nucleobases. Of note, JB1-JB4 series bind complementary bases (C-G, G-C, A-T and T-A), while JB5-JB16 bind mismatches, and thus can be used to bind two strands of matched and/or mismatched bases. Divalent nucleobases are described in further detail in United States Patent Publication No. 20160083434 A1 and International Patent Publication No. WO/2018/058091.

**Table A - Divalent Nucleobases**

| **Nucleobase** | **Bases represented** | | **Nucleobase** | **Bases represented** |
|---|---|---|---|---|
| JB1 | T/D* | | JB9/JB9b | A/C |
| JB2 | D/T | | JB10 | C/A |
| JB3 | G/C | | JB11 | U/G |
| JB4 | C/G | | JB12 | G/U |
| JB5 | C/C | | JB13 | C/U |
| JB6 | U/U | | JB14 | U/C |
| JB7 | G/G | | JB15 | G/D |
| JB8 | D/D | | JB16 | D/G |

| | | | | |
|---|---|---|---|---|
| *diaminopurine, an adenine analog. | | | | |

**As** used herein, "a JB# nucleobase", such as "a JB4 nucleobase," refers to all nucleobases having the JB4 designation, representing C/G (binding G/C), including JB4, JB4b, JB4c, JB4d, and JB4e. Exemplary γPNA structures that are not end-modified with aryl groups in the manner described herein, but which may be, as described herein, are disclosed in International Patent Publication No. WO 2012/138955. A miniPEG γPNA includes one or more groups comprising poly(ethylene glycol) (PEG, polyoxyethylene) moieties, as are shown herein.

Complementary refers to the ability of polynucleotides (nucleic acids) to hybridize (bind) to one another, forming inter-strand base pairs. Base pairs are formed by hydrogen bonding between nucleotide units in antiparallel polynucleotide strands. Complementary polynucleotide strands can base pair (hybridize or bind) in the Watson-Crick manner (e.g., A to T, A to U, C to G), or in any other manner that allows for the formation of duplexes. When using RNA as opposed to DNA, uracil rather than thymine is the base that is complementary to adenosine. Two sequences comprising complementary sequences can hybridize if they form duplexes under specified conditions, such as in water, saline (e.g., normal saline, or 0.9% w/v saline) or phosphate-buffered saline), or under other stringency conditions, such as, for example and without limitation, 0.1X SSC (saline sodium citrate) to 10X SSC, where 1X SSC is 0.15M NaCl and 0.015M sodium citrate in water. Hybridization of complementary sequences is dictated, e.g., by salt concentration and temperature, with the melting temperature (Tm) lowering with increased mismatches and increased stringency. Perfectly matched sequences are said to be "fully complementary", though one sequence (e.g., a target sequence in an mRNA) may be longer than the other, as in the case of the small recognition reagents described herein in relation to the much longer target sequences on which they concatenate, such as mRNAs containing repeat expansions. Two complementary strands of nucleic acid bind in an antiparallel orientation, with one strand in a 5' to 3' orientation, and the other in a 3' to 5' orientation. PNA permits both parallel and antiparallel orientation, though for γPNA antiparallel binding is preferred.

According to one aspect of the invention, a genetic recognition reagent is provided. In aspects, the recognition reagent is self-concatenating, meaning it comprises concatenating groups, that is, end groups that either covalently link or, for non-covalent linkages, e.g. by pi stacking, when hybridized to adjacent sequences on a target nucleic acid. The recognition reagent comprises three or more, e.g., 3-10 or 3-8 contiguous nucleic acid or nucleic acid analog monomer residues, with terminal groups that, e.g., either self-ligate or pi stack, and, for targeting double-stranded RNA hairpin sequences, containing a sequence of nucleobases for hybridizing to (binding) repeat expansion sequences associated with a repeat expansion disease. In the case of polyQ diseases, double stranded sequences of hairpins formed from rCAG^{exp} sequences are the target sequences, and the reagents have a sequence of bivalent nucleobases chosen from the following: EIF or (EIF)n where n is an integer of two more, such as 2, 3, 4, or 5, such as EIFEIF; IFE or (IFE)n where n is an integer of two more, such as 2, 3, 4, or 5, such as IFEIFE; or FEI, or (FEI)n where n is an integer of two more, such as 2, 3, 4, or 5, such as FEIFEI, where E is a bivalent nucleobase, such as JB3 or JB3b, binding C/G (Watson strand/Crick strand), F is a bivalent nucleobase, such as JB4, JB4b, JB4c, JB4d, or JB4e, binding G/C, and I is a bivalent nucleobase, such as JB6 or JB6b, binding A/A, thus being able to bind both hybridized strands of a hairpin formed from CAG repeats, as depicted in Figure 1.

The recognition reagents described herein combine the features of small molecules, for example, low molecular weight, ease of large-scale production, low production cost, cell permeability, and desired pharmacokinetics, with the sequence-specific recognition of oligonucleotides via Watson-Crick base-pairings. The concatenation of the oligomer recognition reagents has been demonstrated for both self-ligating and pi-stacking concatenation methods and is described in accordance with examples, which are intended to be illustrative in all aspects rather than restrictive.

Examples of applications for the recognition reagents described herein is in the treatment of genetic diseases with repeat expansion of small sequences, such as those listed in Table B.

**Table B - Genetic diseases associated with expanded repeats**

| Disease | Repeat Unit | Gene Name | Normal Repeat Length | Pathogenic Repeat Length |
|---|---|---|---|---|
| FRDA | (GAA)n | *FRDA (frataxin)* | 6-32 | 200-1,700 |
| FRAXA | (CGG)n | *FMR1* (FMRP) | 6-60 | >200 |
| FRAXE | (CCG)n | *FMR2* (FMR2) | 4-39 | 200-900 |
| SCA1 | (CAG)n | *SCA1* (ataxin 1) | 6-39 | 40-82 |
| SCA2 | (CAG)n | *SCA2* (ataxin 2) | 15-24 | 32-200 |
| SCA3 (MJD) | (CAG)n | SCA3 (ataxin 3) | 13-36 | 61-84 |
| SCA6 | (CAG)n | *CACNA1A* | 4-20 | 20-29 |
| SCA7 | (CAG)n | SCA7 (ataxin 7) | 4-35 | 37-306 |
| SCA17 | (CAG)n | *SCA17* (TBP) | 25-42 | 47-63 |
| DRPLA | (CAG)n | *DRPLA* (atrophin 1) | 7-34 | 49-88 |
| SBMA | (CAG)n | AR (androgen receptor) | 9-36 | 38-62 |
| HD | (CAG)n | *HD* (huntingtin) | 11-34 | 40-121 |
| MD1 | (CTG)n | *DMPK* (DMPK) | 5-37 | 50-1,000 |
| MD2 | (CCTG)n | *ZNF9* (ZNF9) | 10-26 | 75-11,000 |
| FXTAS | (CGG)n | *FMR1* (FMRP) | 6-60 | 60-200 |
| SCA8 | (CTG)n | *SCA8* | 16-34 | >74 |
| SCA10 | (ATTCT)n | Unknown | 10-20 | 500-4,500 |
| SCA12 | (CAG)n | *PPP2R2B* | 7-45 | 55-78 |
| HDL2 | (CTG)n | *JPH3* | 7-28 | 66-78 |
| ALS | (GGGGCC)n | *C9ORF72* | 20-50 | >100 |

Based on Table B, exemplary sequences of bivalent nucleobases for recognition reagents that would target the described gene products include the sequences in a 5' to 3' direction with respect to the sense strand are provided in Table C. It should be noted that, depending on their sequence, not all repeated sequences will form a hairpin structure under normal conditions, but can be induced into a triplex "hairpin" structure by the listed recognition reagent. Also, the sequences listed in Table C are merely exemplary, and depending on the alignment of the folded sequences in the native hairpin structure or a hairpin structure induced by the recognition reagent, other sequences would be expected to form a triplex structure. Also, the repeated nature of the sequences dictate that for a three-base repeat, three different frameshifts are useful for each sequence (see Table C, third column for (GAA)n), and for a four-base repeat, four different frameshifts are useful. Therefore, for a three-base sequence, permutations of duplex binding alignment, and the ability to shift the frame of the recognition reagent within each alignment (e.g. EIF, IFE, and FEI for the alignment within the depicted rCAG^{exp} sequence in Figure 1, yields nine permutations of the unit recognition reagent, which may be repeated in the recognition reagent. For example, for the sequence (GAA)ₙ, no folded alignment would be expected to form a hairpin duplex under normal conditions, but three different sequences are expected to be able to form a triplex structure. In Table C, the only repeated sequence showing all permutations is the sequences for (GAA)ₙ, for other repeats, only exemplary sequences are provided. Of note, due to the repeated structures, any of the sequences provided in Table C may be repeated, e.g., from 2-5X, for example, G/A-A/A-A/G, also refers to G/A-A/A-A/G-G/A-A/A-A/G, and 13-6-14 (JB13 series-JB6 series-JB14 series) also includes 13-6-14-13-6-14. In Table C, nucleobase sequences are first identified by the target nucleobases they bind (e.g. JB4 binds G/C), and also are identified by "JB" reference number, e.g., "4" refers to "JB4", and includes JB4, JB4b, JB4c, JB4d, and JB4e.

**Table C - Examples of recognition reagents**

| Repeat Unit | Exemplary Unit Recognition Reagent Target Sequence (bases bound) | Exemplary Unit Recognition Reagent Sequence (JB#) |
|---|---|---|
| (GAA)ₙ | G/A-A/A-A/G | 13-6-14 |
| | A/A-A/G-G/A | 6-14-13 |
| | A/G-G/A-A/A | 14-13-6 |
| | A/G-A/A-G/A | 14-6-13 |
| | A/A-G/A-A/G | 6-13-14 |
| | G/A-A/G-A/A | 13-14-6 |
| (CGG)ₙ | C/G-G/G-G/C | 3-5-4 |
| | G/G-G/C-C/G | 5-4-3 |
| | G/C-C/G- G/G | 4-3-5 |
| | G/C-G/G-C/G | 4-5-3 |
| | G/G-C/G-G/C | 5-3-4 |
| | C/G-G/C-G/G | 3-4-5 |
| (CCG)ₙ | C/G-C/C-G/C | 3-7-4 |
| | C/C-G/C-C/G | 7-4-3 |
| | G/C-C/G-C/C | 4-3-7 |
| | G/C-C/C-C/G | 4-7-3 |
| | C/C-C/G-G/C | 7-3-4 |
| | C/G-G/C-C/C | 3-4-7 |
| (CAG)ₙ | C/G-A/A-G/C | 3-6-4 |
| | A/A-G/C-C/G | 6-4-3 |
| | G/C-C/G-A/A | 4-3-6 |
| | G/C-A/A-C/G | 4-6-3 |
| | A/A-C/G-G/C | 6-3-4 |
| | C/G-G/C-A/A | 3-4-6 |
| (CTG)ₙ | C/G-U/U-G/C | 3-8-4 |
| | U/U-G/C-C/G | 8-4-3 |
| | G/C-C/G-U/U | 4-3-8 |
| | G/C-U/U-C/G | 4-8-3 |
| | U/U-C/G-G/C | 8-3-4 |
| | C/G-G/C-U/U | 3-4-8 |
| (CCTG)ₙ | C/G-C/U-U/C-G/C | 3-15-16-4 |
| | C/U-U/C-G/C-C/G | 15-16-4-3 |
| | U/C-G/C-C/G-C/U | 16-4-3-15 |
| | G/C-C/G-C/U-U/C | 4-3-15-16 |
| | G/C-U/C-C/U-C/G | 4-16-15-3 |
| | U/C-C/U-C/G-G/C | 16-15-3-4 |
| | C/U-C/G-G/C-U/C | 15-3-4-16 |
| | C/G-G/C-U/C-C/U | 3-4-16-15 |
| (ATTCT)ₙ | A/U-U/C-U/U-C/U-U/A | 1-16-8-15-2 |
| | U/C-U/U-C/U-U/A-A/U | 16-8-15-2-1 |
| | U/U-C/U-U/A-A/U-U/C | 8-15-2-1-16 |
| | C/U-U/A-A/U-U/C-U/U | 15-2-1-16-8 |
| | U/A-A/U-U/C-U/U-C/U | 2-1-16-8-15 |
| | U/A-C/U-U/U-U/C-A/U | 2-15-8-16-1 |
| | C/U-U/U-U/C-A/U-U/A | 15-8-16-1-2 |
| | U/U-U/C-A/U-U/A-C/A | 8-16-1-2-15 |
| | U/C-A/U-U/A-C/A-U/U | 16-1-2-15-8 |
| | A/U-U/A-C/A-U/U-U/C | 1-2-15-8-16 |
| (GGGGCC)ₙ | G/C-G/C-G/G-G/G-C/G-C/G | 4-4-5-5-3-3 |
| | G/C-G/G-G/G-C/G-C/G-G/C | 4-5-5-3-3-4 |
| | G/G-G/G-C/G-C/G-G/C-G/C | 5-5-3-3-4-4 |
| | G/G-C/G-C/G-G/C-G/C-G/G | 5-3-3-4-4-5 |
| | C/G-C/G-G/C-G/C-G/G-G/G | 3-3-4-4-5-5 |
| | C/G-C/G-G/G-G/G-G/C-G/C | 3-3-5-5-4-4 |
| | C/G-G/G-G/G-G/C-G/C-C/G | 3-5-5-4-4-3 |
| | G/G-G/G-G/C-G/C-C/G-C/G | 5-5-4-4-3-3 |
| | G/G-G/C-G/C-C/G-C/G-G/G | 5-4-4-3-3-5 |
| | G/C-G/C-C/G-C/G-G/G-G/G | 4-4-3-3-5-5 |
| | G/C-C/G-C/G-G/G-G/G-G/C | 4-3-3-5-5-4 |
| | C/G-C/G-G/G-G/G-G/C-G/C | 3-3-5-5-4-4 |

In Table C, the Unit Recognition Reagent Target Sequence (bases bound) lists each base pair of the two strands bound by a single bivalent nucleobase of the recognition reagent as "B₁/B₂", with B₁ being a base from a first strand, and B₂ being a base from the second strand, as bound by a bivalent nucleobase of the recognition reagent. As such, for the sequence (CAG)ₙ, the sequence of the unit target sequence is C/G-A/A-G/C, referring to an antiparallel arrangement of the sequence 5'-CAG-3', and for the sequence, (CCG)ₙ, the sequence of the second alignment of the target sequence, C/G-G/C-C/C, referring to alignment of 5'-CGC-3' in antiparallel with 5'-CCG-3', while the sequence C/G-C/C-G/C refers to an antiparallel arrangement of the sequence 5'-CCG-3'.

A "unit target sequence" in the context of binding of a recognition reagent is the shortest repeated sequence found in a repeated nucleobase sequence in a target nucleotide sequence, or a repeated double-stranded nucleobase sequence, including mismatches, in a double-stranded sequence, or two strands of the same or a different nucleic acid molecule, aligned when bound in a triplex structure by a bivalent recognition reagent, e.g., as described herein.

In one aspect, a recognition reagent is provided, comprising: a peptide nucleic acid or γ peptide nucleic acid backbone, having a first end and a second end, prepared from three or more, e.g. from 3 to 10, or 3, 4, 5, 6, 7, 8, 9, or 10, or from 3-8, PNA or γPNA backbone residues; a sequence of nucleobases attached or linked in a sequence to a plurality of the nucleic acid or nucleic acid analog backbone residues; an -SH, -OH, or -S-S-Lg moiety at the first end of the backbone, and an -C(O)-S-Lg or -C(O)-O-Lg moiety (a leaving group, Lg, linked to the backbone via an ester or thioester linkage) at the second end of the backbone, where the sequence of nucleobases is complementary to a unit target sequence or two or more sequential iterations (two or more direct repeats of the specified sequence, without gaps) of a unit target sequence within a target sequence in a nucleic acid, such that when a plurality of recognition modules bind to adjacent sequences of the target sequence they ligate to each other. Lg in one aspect is biocompatible and/or non-toxic. Non-limiting examples of Lg include, substituted or unsubstituted (C₁-C₈)alkyl, substituted or unsubstituted (C₃-C₈)aryl, (C₃-C₈)aryl(C₁-C₆)alkylene, (C₁-C₈) carboxylic, optionally substituted with an amino acid side chain, or a guanidine-containing group, or 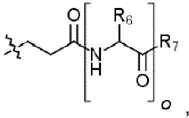 where o is 1-20, each instance of R₆ is, independently, an amino acid side chain, and R₇ is -OH or -NH₂.

A recognition reagent is provided, comprising: a nucleic acid analog backbone, having a first end and a second end, prepared from three or more, e.g. from 3 to 10, or 3, 4, 5, 6, 7, 8, 9, or 10, or from 3-8, nucleic acid or nucleic acid analog backbone residues, that is optionally conformationally preorganized; a sequence of nucleobases attached or linked in a sequence to a plurality of the nucleic acid or nucleic acid analog backbone residues; a first aryl moiety linked to the first end of the nucleic acid or nucleic acid backbone; and a second aryl moiety that is optionally the same as the first aryl moiety, attached to the second end of the of the nucleic acid or nucleic acid backbone. The aryl moieties are, independently, a two- to five-ring fused polycyclic aromatic moiety that is xanthene, riboflavin, mangostin, or mangiferin that stack with an aryl moiety of an adjacent recognition reagent when recognition reagents are hybridized to adjacent sequences of a target nucleic acid. The recognition reagent is able to bind both strands of a hairpin formed from an expanded repeat, e.g., associated with an expanded repeat disease, e.g., as listed in Table B, such as a PolyQ disease. In one aspect, for use in binding CAG repeats, as described above, having the sequence EIF or (EIF)n where n is an integer of two more, such as 2, 3, 4, or 5, such as EIFEIF; IFE or (IFE)n where n is an integer of two more, such as 2, 3, 4, or 5, such as IFEIFE; or FEI, or (FEl)n where n is an integer of two more, such as 2, 3, 4, or 5, such as FEIFEI.

In one aspect, the recognition reagent is self-ligating, and has the structure: 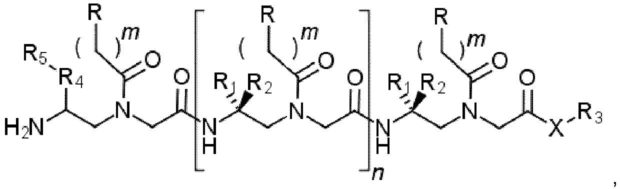
where, X is S or O; n is an integer from 1 to 6, inclusive; m is an integer from 0 to 4, inclusive;
R₁ and R₂ are each, independently: H; a guanidine-containing group such as 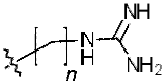 where n=1, 2, 3, 4, or 5; an amino acid side chain, such as: 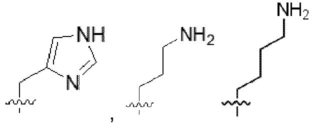 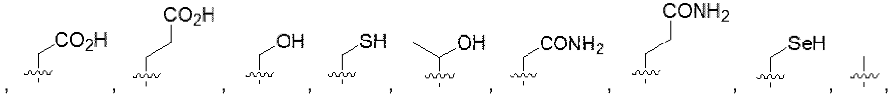 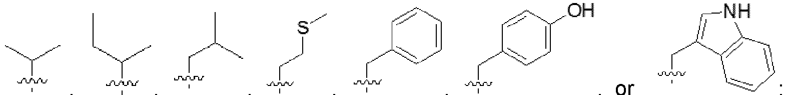 linear or branched (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)ary(C₁-C₆)alkylene, (C₃-C₈)cycloalkyl(C₁-C₆)alkylene, optionally substituted with an ethylene glycol unit comprising from 1 to 50 ethylene glycol moieties; -CH₂-(OCH₂-CH₂)_{q}OP₁; -CH₂-(OCH₂-CH₂)_{q}-NHP₁; -CH₂-(SCH₂-CH₂)_{q}-SP₁; -CH₂-(OCH₂-CH₂)ᵣ-OH; - CH₂-(OCH₂-CH₂)ᵣ-NH₂; -CH₂-(OCH₂-CH₂)ᵣ-NHC(NH)NH₂; or -CH₂-(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50, inclusive; and r and s are each independently integers from 1 to 50, inclusive;
R₃ is H or a leaving group, and in one aspect is biocompatible and/or non-toxic, such as for example, substituted or unsubstituted (C₁-C₈)alkyl, substituted or unsubstituted (C₃-C₈)aryl, (C₃-C₈)aryl(C₁-C₆)alkylene, (C₁-C₈) carboxylic, optionally substituted with an amino acid side chain, or a guanidine-containing group, or 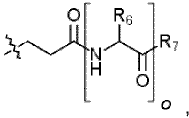 where o is 1-20, each instance of R₆ is, independently, an amino acid side chain, and R₇ is -OH or -NH₂; R₄ is (C₁-C₁₀) divalent hydrocarbon or (C₁-C₁₀) divalent hydrocarbon substituted with one or more N or O moieties, such as -O-, -OH, -C(O)-, -NH-, -NH₂, -C(O)NH-; R₅ is -OH, -SH or a disulfide protecting group; and each instance of R is, independently, a nucleobase, producing a sequence of nucleobases complementary to a target sequence of nucleobases in a target nucleic acid so that each of the plurality of recognition modules bind to the target sequence of nucleobases on the template nucleic acid and ligate to each other. In one aspect, R₅ has the structure -SH, -OH, or -S-S-R₈, where R₈ is one or more amino acid residues, an amino acid side chain, linear, branched or hetero-substituted (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)ary(C₁-C₆)alkylene, (C₃-C₈)cycloalkyl(C₁-C₆)alkylene. In one aspect, R₁ or R₂ is an amino acid side chain or a guanidine-containing group, such as 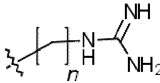 where n=1, 2, 3, 4, or 5. In one aspect R₁ and R₂ are different, R₁ is H and R₂ is not H, or R₂ is H and R₁ is not H. For binding to natural nucleic acids, such as RNA or DNA, R₁ may be H and R₂ is not H, thereby forming "right-handed" L-γPNA. "Left-handed" D-γPNA, in which, e.g., R₂ is H and R₁ is not H, does not bind natural nucleic acids. In one aspect, the recognition reagent is substituted with one or more guanidine-containing group, such as 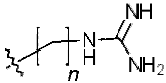 where n=1, 2, 3, 4, or 5.

In one aspect, R₁ or R₂ is (C₁-C₆)alkyl substituted with -(OCH₂-CH₂)_{q}OP₁; -(OCH₂-CH₂)_{q}-NHP₁; -(SCH₂-CH₂)_{q}-SP₁; -(OCH₂-CH₂)ᵣ-OH; -(OCH₂-CH₂)ᵣ-NH₂; -(OCH₂-CH₂)ᵣ-NHC(NH)NH₂; or -(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50; r is an integer from 1 to 50, and s is an integer from 1 to 50.

Recognition reagents terminating in aryl groups have the ability to concatenate as a result of non-covalent binding, e.g. pi-stacking, of aryl groups of immediately adjacent hybridizing recognition reagents. Therefore, in another aspect, a recognition reagent (recognition module) is provided having the structure: 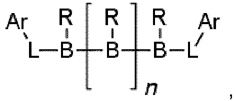
where R are independently, nucleobases, and each instance of R can be the same, or a different nucleobase;
n is an integer ranging from 1 to 6, such as 1, 2, 3, 4, 5, or 6;
each B is independently a ribose-5-phosphate residue, deoxyribose-5-phosphate residue, or a nucleic acid analog backbone residue, and in one aspect, is a backbone residue of a conformationally preorganized nucleic acid analog, such as γPNA or LNA;
L are, independently, linkers, e.g., a non-reactive linker or a non-reactive, non-bulky linker, and each instance of L can be the same or different; and
each instance of Ar are, independently, two- to five-ring fused polycyclic aromatic moieties, e.g., substituted or unsubstituted aryl or heteroaryl moieties having from two to five fused rings, for example and without limitation, unsubstituted or substituted pentalene, indene, naphthalene, azulene, heptalene, biphenylene, as-indacene, s-indacene, acenaphthylene, fluorene, phenalene, phenanthrene, anthracene, fluoranthene, acephenanthrylene, aceanthrylene, triphenylene, pyrene, chrysene, naphthacene/tetracene, pleiadene, picene, or perylene, optionally substituted with one or more hetero atoms such as O, N, and/or S, for example, xanthene, riboflavin (vitamin B2), mangostin, or mangiferin, and may be the same or different, and in aspects are the same. In some aspects, each Ar stacks with an Ar group of an adjacent recognition reagent when recognition reagents are hybridized to adjacent sequences of a target nucleic acid.

**A** moiety in a compound, such as an aryl moiety or a nucleobase is covalently attached to the recognition reagent backbone, and thus is said to be "linked" to the backbone. Depending on the chemistry used to prepare the compound, the linkage may be direct, or through a "linker" which is a moiety that covalently attaches two other moieties or groups. In one aspect, terminal aromatic (aryl) groups are attached to the recognition reagent via a linker. The linker is a non-reactive moiety that links the aromatic group to the backbone of the recognition reagent, and, in aspects includes from 1-10 carbon atoms (C₁-C₁₀), optionally substituted with a hetero-atom, such as a N, S, or O, or a non-reactive linkage, such as an amide linkage (peptide bond) formed by reacting an amine with a carboxyl group. Examples of C₁-C₁₀ alkylenes are linear or branched, alkylene (bivalent) moieties optionally comprising a cyclic moiety, such as a methylene, ethylene, trimethylene, tetramethylene, pentamethylene, hexamethylene, hepamethylene, octamethylene, nonamethylene, or decamethylene moiety (that is, -CH₂-[CH₂]ₙ-, where n=1 to 9), optionally comprising an amide linkage. The linkers are non-bulky in that they do not sterically hinder or otherwise interfere to any substantial extent with the binding of the recognition reagents to a target nucleic acid, and do not interfere with concatenation of the recognition reagents on the target nucleic acid. The linker is the remaining moiety resulting from the linking of the aromatic group and the backbone of the recognition reagent, e.g. 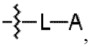 , or, in one non-limiting example, 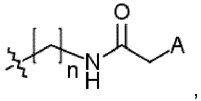 resulting from the linkage of an acetic acid-substituted aryl compound (A), such as pyrene-1-acetic acid, to a Dab (n=1), Orn (n=2), or Lys (n=3) residue.

In further aspects, the linker or linking group is an organic moiety that connects two parts of a compound, e.g., covalently attaches two parts of a compound, such as, for example and without limitation in context of the present disclosure, connection of the aromatic groups to the backbone of the recognition reagent, connection of a nucleobase to the nucleic acid or nucleic acid analog backbone, and/or connection of a guanidium group to the recognition reagent. Linkers typically comprise a direct bond or an atom such as oxygen or sulfur, a unit such as, C(O), C(O)NH, SO, SO₂, SO₂NH, or a chain of atoms, such as, but not limited to, substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynyl heteroarylalkyl, alkynylheteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl, in which one or more carbons, e.g., methylenes or methylidynes (-CH=) is optionally interrupted or terminated by a hetero atom, such as O, S, or N, substituted or unsubstituted aryl, substituted or unsubstituted heteroaryl, substituted or unsubstituted heterocyclic. In one aspect, the linker comprises between about 5 to 25 atoms, e.g., 5-20, 5-10, e.g., 5, 6, 7, 8, 9, 10, 11, 12, 13, 14, 15, 16, 17, 18, 19, or 20 atoms, or a total of from 1 to 10, e.g., 1, 2, 3, 4, 5, 6, 7, 8, 9, or 10 C and heteroatoms, e.g., O, P, N, or S atoms.

For linkage to a PNA, such as a γPNA, an expedient and available linker is one that reacts an amine with a carboxyl group to form an amide linkage, e.g., using well-known peptide synthesis chemistries to add amino acids to the recognition reagent, where the amino acids may be pre-modified with a chemical moiety, such as an aryl moiety, or a guanidine group, as shown in the Example, below, with the addition of an aryl-modified amino acid to link the pyrene aryl moiety to the recognition reagent, and use of arginine to provide the guanidine groups. Linking to non-peptide nucleic acid analogs can be achieved using any suitable linking chemistry as are broadly-known, such as by using carbodiimide chemistry, e.g., EDC (EDAC, 1-Ethyl-3-[3-dimethylaminopropyl] carbodiimide hydrochloride) to link amine-modified aromatic moieties to the recognition reagent, e.g., via terminal phosphates.

In linking the aromatic group to the backbone of the recognition reagent, the linker is of an appropriate size or length to place the aromatic group in position for π- π stacking during concatenation of the recognition reagents on a target nucleic acid sequence as described herein.

In a further aspect, the recognition reagent has the structure: 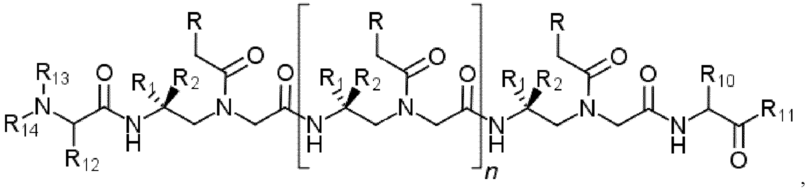 where,
each instance of R is, independently, a nucleobase, and each instance of R can be the same, or a different nucleobase;
n is an integer ranging from 1 to 6, such as 1, 2, 3, 4, 5, or 6;
R₁ and R₂ are each, independently: H, a guanidine-containing group such as 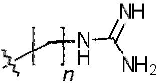
where n=1, 2, 3, 4, or 5, an amino acid side chain, such as: 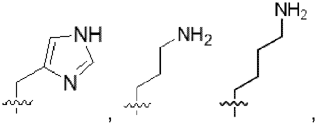 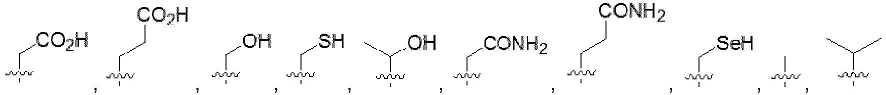 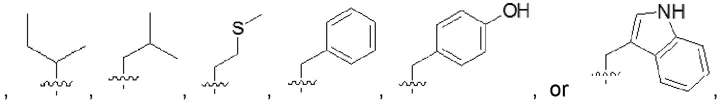 unsubstituted or substituted (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)aryl(C₁-C₆)alkylene, or (C₃-C₈)cycloalkyl(C₁-C₆)alkylene; -CH₂-(OCH₂-CH₂)_{q}OP₁; -CH₂-(OCH₂-CH₂)_{q}-NHP₁; -CH₂-(OCH₂-CH₂-0)_{q}-SP₁; -CH₂-(SCH₂-CH₂)_{q}-SP₁, -CH₂-(OCH₂-CH₂)ᵣ-OH; -CH₂-(OCH₂-CH₂)ᵣ-NH₂; -CH₂-(OCH₂-CH₂)ᵣ-NHC(NH)NH₂; or -CH₂-(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50; r is an integer from 1 to 50, and s is an integer from 1 to 50, inclusive; and in one aspect R₁ and R₂ are different, R₁ is H and R₂ is not H, or R₂ is H and R₁ is not H;
one of R₁₀ or R₁₁, and one of R₁₂, R₁₃, or R₁₄ are -L- R₃, where each instance of R₃ is, independently, two- to five-ring fused polycyclic aromatic moieties, e.g., substituted or unsubstituted aryl or heteroaryl moieties having from two to five fused rings, for example and without limitation, unsubstituted or substituted pentalene, indene, naphthalene, azulene, heptalene, biphenylene, as-indacene, s-indacene, acenaphthylene, fluorene, phenalene, phenanthrene, anthracene, fluoranthene, acephenanthrylene, aceanthrylene, triphenylene, pyrene, chrysene, naphthacene/tetracene, pleiadene, picene, or perylene, optionally substituted with one or more hetero atoms such as O, N, and/or S, for example, xanthene, riboflavin (vitamin B2), mangostin, or mangiferin, and may be the same or different, and in aspects are the same, and in some instances stacks with an R₃ group of an adjacent recognition reagent when recognition reagents are hybridized to adjacent sequences of a target nucleic acid, and where L is a linker, e.g. a non-reactive linker or a non-reactive, non-bulky linker, and each instance of L can be the same or different, and may comprise an amino acid residue, or substituted or unsubstituted alkyl, substituted or unsubstituted alkenyl, substituted or unsubstituted alkynyl, arylalkyl, arylalkenyl, arylalkynyl, heteroarylalkyl, heteroarylalkenyl, heteroarylalkynyl, heterocyclylalkyl, heterocyclylalkenyl, heterocyclylalkynyl, aryl, heteroaryl, heterocyclyl, cycloalkyl, cycloalkenyl, alkylarylalkyl, alkylarylalkenyl, alkylarylalkynyl, alkenylarylalkyl, alkenylarylalkenyl, alkenylarylalkynyl, alkynylarylalkyl, alkynylarylalkenyl, alkynylarylalkynyl, alkylheteroarylalkyl, alkylheteroarylalkenyl, alkylheteroarylalkynyl, alkenylheteroarylalkyl, alkenylheteroarylalkenyl, alkenylheteroarylalkynyl, alkynylheteroarylalkyl, alkynyl heteroarylalkenyl, alkynylheteroarylalkynyl, alkylheterocyclylalkyl, alkylheterocyclylalkenyl, alkylhererocyclylalkynyl, alkenylheterocyclylalkyl, alkenylheterocyclylalkenyl, alkenylheterocyclylalkynyl, alkynylheterocyclylalkyl, alkynylheterocyclylalkenyl, alkynylheterocyclylalkynyl, alkylaryl, alkenylaryl, alkynylaryl, alkylheteroaryl, alkenylheteroaryl, alkynylhereroaryl, in which one or more carbons, e.g., methylenes or methylidynes (-CH=) is optionally interrupted or terminated by a hetero atom, such as O, S, or N, substituted or unsubstituted aryl, substituted or unsubstituted heteroaryl, substituted or unsubstituted heterocyclic, and the remainder of R₁₀, R₁₁, R₁₂, R₁₃, and R₁₄ are each, independently: H; one or more contiguous amino acid residues; a guanidine-containing group; an amino acid side chain; linear or branched (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)aryl(C₁-C₆)alkylene, (C₃-C₈)cycloalkyl(C₁-C₆)alkylene, optionally substituted with an ethylene glycol unit comprising from 1 to 50 ethylene glycol moieties; -CH₂-(OCH₂-CH₂)_{q}OP₁; -CH₂-(OCH₂-CH₂)_{q}-NHP₁; -CH₂-(SCH₂-CH₂)_{q}-SP₁; -CH₂-(OCH₂-CH₂)ᵣ-OH; -CH₂-(OCH₂-CH₂)ᵣ-NH₂; -CH₂-(OCH₂-CH₂)ᵣ-NHC(NH)NH₂; or -CH₂-(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50; r is an integer from 1 to 50, and s is an integer from 1 to 50.

In one aspect R₄ and R₇ are -L- R₃, and in another aspect, R₄ and R₇ are -L- R₃ and R₁₁ and R₁₄ are Arg. In one aspect, the linker comprises between about 5 to 25 atoms, e.g., 5-20, 5-10, e.g., 5, 6, 7, 8, 9, 10, 11, 12, 13, 14, 15, 16, 17, 18, 19, or 20 atoms, or a total of from 1 to 10, e.g., 1, 2, 3, 4, 5, 6, 7, 8, 9, or 10 C and heteroatoms, e.g., O, P, N, or S atoms. In another aspect, one or more of R₁, R₂, R₁₀, R₁₁, R₁₂, R₁₃, or R₁₄ is (C₁-C₆)alkyl substituted with -(OCH₂-CH₂)_{q}OP₁; -(OCH₂-CH₂)_{q}-NHP₁; -(SCH₂-CH₂)_{q}-SP₁; -(OCH₂-CH₂)ᵣ-OH; -(OCH₂-CH₂)ᵣ-NH₂; -(OCH₂-CH₂)ᵣ-NHC(NH)NH₂; or -(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50; r is an integer from 1 to 50, and s is an integer from 1 to 50.

In yet another aspect, the recognition reagent comprises a PNA backbone, and thus has the structure: 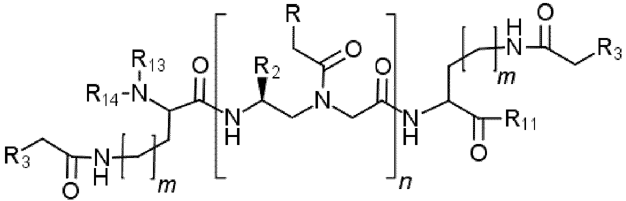 where,
n is an integer ranging from 1 to 8, including 1, 2, 3, 4, 5, 6, ,7, or 8;
m is an integer ranging from 1 to 5, such as from 1-3, including 1, 2, 3, 4, or 5;
R₂ is a guanidine-containing group such as 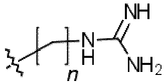 where n=1, 2, 3, 4, or 5, an amino acid side chain, such as: 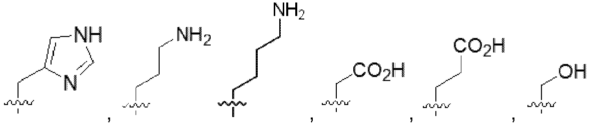 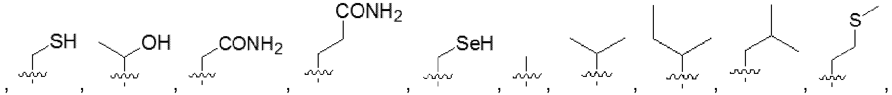 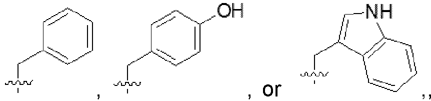 unsubstituted or substituted (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)aryl(C₁-C₆)alkylene, or (C₃-C₈)cycloalkyl(C₁-C₆)alkylene; -CH₂-(OCH₂-CH₂)_{q}OP₁; -CH₂-(OCH₂-CH₂)_{q}-NHP₁; -CH₂-(OCH₂-CH₂-0)_{q}-SP₁; -CH₂-(SCH₂-CH₂)_{q}-SP₁, -CH₂-(OCH₂-CH₂)ᵣ-OH; -CH₂-(OCH₂-CH₂)ᵣ-NH₂; -CH₂-(OCH₂-CH₂)ᵣ-NHC(NH)NH₂; or -CH₂-(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50, inclusive; r and s are each independently integers from 1 to 50, inclusive;
R₃ is an unsubstituted fused-ring polycyclic aromatic moiety, such as pentalene, indene, naphthalene, azulene, heptalene, biphenylene, as-indacene, s-indacene, acenaphthylene, fluorene, phenalene, phenanthrene, anthracene, fluoranthene, acephenanthrylene, aceanthrylene, triphenylene, pyrene, chrysene, naphthacene/tetracene, pleiadene, picene, or perylene; and
each of R₁₁, R₁₃, and R₁₄ are, independently H, a guanidine-containing group such as 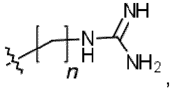 where n=1, 2, 3, 4, or 5, an amino acid side chain, or one or more contiguous amino acid residues, such as one or more Arg residue. In one aspect, R₃ is pyrene.

In another aspect, R₂ is -CH₂-(OCH₂-CH₂)ᵣ-OH, where r is an integer ranging from 1-50, e.g., 1-10, such as 1, 2, 3, 4, 5, 6, 7, 8, 9, or 10, and in one example 2. In another aspect, one or more of R₂, R₁₁, R₁₃, or R₁₄ is (C₁-C₆)alkyl substituted with -(OCH₂-CH₂)_{q}OP₁; -(OCH₂-CH₂)_{q}-NHP₁; -(SCH₂-CH₂)_{q}-SP₁; -(OCH₂-CH₂)ᵣ-OH; -(OCH₂-CH₂)ᵣ-NH₂; -(OCH₂-CH₂)ᵣ-NHC(NH)NH₂; or -(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50; r is an integer from 1 to 50, and s is an integer from 1 to 50.

While certain of the preceding PNA-based recognition reagents are shown without chirality, in one example, the gamma carbons (to which R₁ and R₂ are attached), are in an (R) orientation, where R₂ is H and R₁ is not H, or the gamma carbons are in an (S) orientation, where, e.g., R₁ is H and R₂ is not H. Further, in one example, when present, in the R₁₀, R₁₁, R₁₂, R₁₃, and/or R₁₄ positions, one or more, or all, chiral amino acid residues may be L-amino acids. In another example, when present, in the R₁₀, R₁₁, R₁₂, R₁₃, and/or R₁₄ positions, one or more, or all, chiral amino acid residues may be D-amino acids.

Pharmaceutically-acceptable salts of any of the preceding also are provided.

Pharmaceutically acceptable salts of any of the compounds described herein also may be used in the methods described herein. Pharmaceutically acceptable salt forms of the compounds described herein may be prepared by conventional methods known in the pharmaceutical arts, and include as a class veterinarily acceptable salts. For example and without limitation, where a compound comprises a carboxylic acid group, a suitable salt thereof may be formed by reacting the compound with an appropriate base to provide the corresponding base addition salt. Non-limiting examples include: alkali metal hydroxides, such as potassium hydroxide, sodium hydroxide and lithium hydroxide; alkaline earth metal hydroxides, such as barium hydroxide and calcium hydroxide; alkali metal alkoxides, such as potassium ethanolate and sodium propanolate; and various organic bases such as piperidine, diethanolamine, and N-methylglutamine.

Non-limiting examples of pharmaceutically-acceptable base salts include: aluminum, ammonium, calcium, copper, ferric, ferrous, lithium, magnesium, manganic, manganous, potassium, sodium, and zinc salts. Salts derived from pharmaceutically acceptable organic non-toxic bases include, without limitation: salts of primary, secondary, and tertiary amines, substituted amines including naturally occurring substituted amines, cyclic amines, and basic ion exchange resins, such as arginine, betaine, caffeine, chloroprocaine, choline, N,N'-dibenzylethylenediamine (benzathine), dicyclohexylamine, diethanolamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lidocaine, lysine, meglumine, N-methyl-D-glucamine, morpholine, piperazine, piperidine, polyamine resins, procaine, purines, theobromine, triethanolamine, triethylamine, trimethylamine, tripropylamine, and tris-(hydroxymethyl)-methylamine (tromethamine).

Non-limiting examples of pharmaceutically-acceptable acid salts include: acetate, adipate, alginate, arginate, aspartate, benzoate, besylate (benzenesulfonate), bisulfate, bisulfite, bromide, butyrate, camphorate, camphorsulfonate, caprylate, chloride, chlorobenzoate, citrate, cyclopentanepropionate, digluconate, dihydrogenphosphate, dinitrobenzoate, dodecylsulfate, ethanesulfonate, fumarate, galacterate, galacturonate, glucoheptanoate, gluconate, glutamate, glycerophosphate, hemisuccinate, hemisulfate, heptanoate, hexanoate, hippurate, hydrochloride, hydrobromide, hydroiodide, 2-hydroxyethanesulfonate, iodide, isethionate, iso-butyrate, lactate, lactobionate, malate, maleate, malonate, mandelate, metaphosphate, methanesulfonate, methylbenzoate, monohydrogenphosphate, 2-naphthalenesulfonate, nicotinate, nitrate, oxalate, oleate, pamoate, pectinate, persulfate, phenylacetate, 3-phenylpropionate, phosphate, phosphonate, and phthalate.

Multiple salts forms are also considered to be pharmaceutically-acceptable salts. Common, non-limiting examples of multiple salt forms include: bitartrate, diacetate, difumarate, dimeglumine, diphosphate, disodium, and trihydrochloride.

**As** such, "pharmaceutically acceptable salt" as used herein is intended to mean an active ingredient (drug) comprising a salt form of any compound as described herein. The salt form preferably confers to the improved and/or desirable pharmacokinetic/pharmodynamic properties of the compounds described herein.

**The** compositions described herein can be administered by any effective route. Examples of delivery routes include, without limitation: topical, for example, epicutaneous, inhalational, enema, ocular, otic and intranasal delivery; enteral, for example, orally, by gastric feeding tube and rectally; and parenteral, such as, intravenous, intraarterial, intramuscular, intracardiac, subcutaneous, intraosseous, intradermal, intrathecal, intraperitoneal, transdermal, iontophoretic, transmucosal, epidural and intravitreal, with oral, intravenous, intramuscular and transdermal approaches being preferred in many instances. Suitable dosage forms may include single-dose, or multiple-dose vials or other containers, such as medical syringes, containing a composition comprising an active ingredient useful for treatment of a repeat expansion disease, as described herein.

Dosage regimens may be adjusted to provide the optimum desired response (e.g., a therapeutic or prophylactic response). For example, a single bolus may be administered, several divided doses may be administered overtime, or the composition may be administered continuously or in a pulsed fashion with doses or partial doses being administered at regular intervals, for example, every 10, 15, 20, 30, 45, 60, 90, or 120 minutes, every 2 through 12 hours daily, or every other day, *etc.,* be proportionally reduced or increased as indicated by the exigencies of the therapeutic situation. In some instances, it may be especially advantageous to formulate compositions, such as parenteral or inhaled compositions, in dosage unit form for ease of administration and uniformity of dosage. The specification for the dosage unit forms are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

Useful dosage forms include: intravenous, intramuscular, or intraperitoneal solutions, oral tablets or liquids, topical ointments or creams and transdermal devices (e.g., patches). In one aspect, the compound is a sterile solution comprising the active ingredient (drug, or compound), and a solvent, such as water, saline, lactated Ringer's solution, or phosphate-buffered saline (PBS). Additional excipients, such as polyethylene glycol, emulsifiers, salts and buffers may be included in the solution.

Therapeutic/pharmaceutical compositions are prepared in accordance with acceptable pharmaceutical procedures, such as described in Remington: The Science and Practice of Pharmacy, 21st edition, ed. Paul Beringer *et al.,* Lippincott, Williams & Wilkins, Baltimore, MD Easton, Pa. (2005) (see, e.g., Chapters 37, 39, 41, 42 and 45 for examples of powder, liquid, parenteral, intravenous and oral solid formulations and methods of making such formulations).

Dosage regimens may be adjusted to provide the optimum desired response (e.g., a therapeutic or prophylactic response). For example, a single bolus may be administered, several divided doses may be administered over time, or the composition may be administered continuously or in a pulsed fashion with doses or partial doses being administered at regular intervals, for example, every 10, 15, 20, 30, 45, 60, 90, or 120 minutes, every 2 through 12 hours daily, or every other day, etc., be proportionally reduced or increased as indicated by the exigencies of the therapeutic situation. In some instances, it may be especially advantageous to formulate compositions, such as parenteral or inhaled compositions, in dosage unit form for ease of administration and uniformity of dosage. The specification for the dosage unit forms are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

Useful dosage forms include: intravenous, intramuscular, or intraperitoneal solutions, oral tablets or liquids, topical ointments or creams and transdermal devices (e.g., patches). In one aspect, the compound is a sterile solution comprising the active ingredient (drug, or compound), and a solvent, such as water, saline, lactated Ringer's solution, or phosphate-buffered saline (PBS). Additional excipients, such as polyethylene glycol, emulsifiers, salts and buffers may be included in the solution.

In addition to the genetic recognition reagents, also provided in aspects of the disclosure, is a method of binding a nucleic acid containing an expanded repeat associated with an expanded repeat disease, comprising contacting a nucleic acid comprising the expanded repeat with any aspect of the genetic recognition reagent described herein. In one aspect, the method is conducted in vitro or ex vivo. In another aspect, the method is conducted in vivo by administering a suitable pharmaceutical formulation comprising the genetic recognition reagent to a patient in an amount effective to knock down expression of a gene having an expanded repeat, or to treat the patient.

In another aspect, a method of identifying the presence of a nucleic acid containing an expanded repeat associated with an expanded repeat disease in a sample obtained from a patient, is provided, such as (CAG)ₙ. The method comprises contacting a nucleic acid sample obtained from a patient with the genetic recognition reagent according to any aspect as described herein. Next, binding and concatenation of the genetic recognition reagent occurs in the presence of the target sequence in a nucleic acid in the sample, such that binding and concatenation is indicative of the presence of the target sequence in a nucleic acid in the sample. Binding and concatenation of the genetic recognition reagent may be detected and/or quantified using any effective method. Presence of the nucleic acid comprising expanded repeats in the target sequence is associated with an expanded repeat disease, at which point, the patient may be treated for the expanded repeat disease, for example, by administration of a pharmaceutical dosage form to the patient comprising a genetic recognition reagent as described herein.

As such, a method of treating a patient having a disease associated with an expanded repeat of a nucleotide sequence is provided. Examples of the diseases are those listed in Table B, or a PolyQ disease such as Huntington's disease, DRPLA (dentatorubral-pallidoluysian atrophy), SBMA (Spinal and bulbar muscular atrophy), SCA1 (Spinocerebellar ataxia Type 1), SCA2 (Spinocerebellar ataxia Type 2), SCA3 (Spinocerebellar ataxia Type 3 or Machado-Joseph disease), SCA6 (Spinocerebellar ataxia Type 6), SCA7 (Spinocerebellar ataxia Type 7), and SCA17 (Spinocerebellar ataxia Type 17). The disease is treated by administration of a composition or pharmaceutical dosage form to the patient comprising a genetic recognition reagent as described herein that is selected to bind the characteristic repeated sequence of the particular disease, such as (CAG)ₙ for a polyQ disease such as Huntington's disease. The composition of pharmaceutical dosage form is administered to the patient in an amount and in a dosage regimen effective to treat the disease. As such as use for a composition comprising a genetic recognition reagent as described herein also is provided, for treatment of a disease associated with an expanded repeat of a nucleotide sequence, such as a PolyQ disease such as Huntington's disease, DRPLA (dentatorubral-pallidoluysian atrophy), SBMA (Spinal and bulbar muscular atrophy), SCA1 (Spinocerebellar ataxia Type 1), SCA2 (Spinocerebellar ataxia Type 2), SCA3 (Spinocerebellar ataxia Type 3 or Machado-Joseph disease), SCA6 (Spinocerebellar ataxia Type 6), SCA7 (Spinocerebellar ataxia Type 7), and SCA17 (Spinocerebellar ataxia Type 17).

In some aspects, the disclosure provides a compound comprising: a) a series of peptide nucleic acid (PNA) units, wherein the series of units comprises i) a first unit; ii) a last unit; and iii) at least one middle unit between the first unit and the last unit, wherein each unit in the series of units comprises: A) a backbone portion, wherein: 1) the backbone portion of the first unit is covalently bound to the backbone portion of one other unit; 2) the backbone portion of the last unit is covalently bound to the backbone portion of one other unit; and 3) the backbone portion of each middle unit is covalently bound to the backbone portion of two other units; and B) a divalent nucleobase covalently bound to the backbone portion; b) a first aryl moiety covalently linked to the first unit; and c) a last aryl moiety covalently linked to the last unit.

In some aspects, the disclosure provides a compound comprising: a) a series of PNA units, wherein the series of units comprises i) a first unit; ii) a last unit; and iii) at least one middle unit between the first unit and the last unit, wherein each unit in the series of units comprises: A) a backbone portion, wherein: 1) the backbone portion of the first unit is covalently bound to the backbone portion of one other unit; 2) the backbone portion of the last unit is covalently bound to the backbone portion of one other unit; and 3) the backbone portion of each middle unit is covalently bound to the backbone portion of two other units; and B) a divalent nucleobase covalently bound to the backbone portion; b) two aryl moieties, one covalently linked to the first unit, and another covalently linked to the last unit.

In some aspects, the disclosure provides a compound wherein the first aryl moiety is covalently linked to the first unit by a first linker moiety, and the last aryl moiety is covalently linked to the last unit by a last linker moiety. In some embodiments, the disclosure provides a compound wherein the first linker moiety and the last linker moiety each independently comprise a guanidine group. In some embodiments, the disclosure provides a compound wherein the first linker moiety and the last linker moiety each independently comprise an amino acid residue. In some embodiments, the disclosure provides a compound wherein the first linker moiety and the last linker moiety each independently comprise three guanidine-containing amino acid residues.

In some aspects, the disclosure provides a compound wherein the first linker moiety and the last linker moiety each independently comprise three contiguous arginine residues. In some embodiments, the disclosure provides a compound wherein the series of units is 3 to 8 units. In some embodiments, the disclosure provides a compound wherein the series of units is a gamma-PNA. In some embodiments, the disclosure provides a compound wherein the first aryl moiety and the last aryl moiety are each independently a two- to five-ring fused polycyclic aromatic moiety, for example, a polyaromatic hydrocarbon such as pyrene. In some embodiments, the disclosure provides a compound wherein the first aryl moiety and the last aryl moiety are the same. In some embodiments, the disclosure provides a compound that further comprises an ethylene glycol unit, for example, a diethylene glycol unit.

In some aspects, the disclosure provides a compound wherein the compound presents the divalent nucleobases in an order that is complementary to a target nucleic acid sequence. In some embodiments, the disclosure provides a compound wherein the target nucleic acid sequence is associated with a repeat expansion disease. In some embodiments, the disclosure provides a compound wherein the repeat expansion disease is myotonic dystrophy type 1 (DM1) or myotonic dystrophy type 2 (DM2). In some embodiments, the disclosure provides a compound wherein, when two of the compounds hybridize to a nucleic acid, the first aryl moiety of one compound and the last aryl moiety of the other compound stack.

In some aspects, the disclosure provides a compound of the formula: H-^{L}Arg-CAGCAG-^{L}Arg-NH₂ (P1); H-^{L}Arg-^{L}Dab(Pyr)-CAGCAG-^{L}Orn(Pyr)-^{L}Arg-NH₂ (P2); H-^{L}Arg-^{L}Orn(Pyr)-CAGCAG-^{L}Orn(Pyr)-^{L}Arg-NH₂ (P3); H-^{L}Arg-^{L}Lys(Pyr)-CAGCAG-^{L}Lys(Pyr)-^{L}Arg-NH₂ (P4); H-^{L}Arg-^{L}Lys(Pyr)-CATCAG-^{L}Lys(Pyr)-^{L}Arg-NH₂ (P5); or H-^{L}Arg-^{L}Lys(Pyr)-CTGCTG-^{L}Lys(Pyr)-^{L}Arg-NH₂ (P6), wherein Orn is ornithine, Dab is diamino butyric acid, and Pyr is a carboxyl-functionalized aromatic compound, for example, a pyrene such as pyrene-1-carboxylic acid, pyrene-2-carboxylic acid, pyrene-4-carboxylic acid, pyrene-1-acetic acid, pyrene-2-acetic acid, or pyrene-4-acetic acid.

In some aspects, the disclosure provides a method of binding a nucleic acid, the method comprising contacting the nucleic acid with a compound of the disclosure, wherein the compound binds to the nucleic acid upon contact. In some embodiments, the disclosure provides a method of knocking down expression of a mRNA in a cell, the method comprising contacting the cell with a compound of the disclosure, wherein the compound knocks down the expression of the mRNA in the cell upon binding a DNA sequence in the cell that corresponds to the mRNA.

In some aspects, the disclosure provides a genetic recognition reagent comprising: a nucleic acid or nucleic acid analog backbone, having a first end and a second end, and having from three to eight ribose-5-phosphate, deoxyribose-5-phosphate, or nucleic acid analog backbone residues; divalent nucleobases, that may be the same or different, linked to a plurality of the ribose-5-phosphate, deoxyribose-5-phosphate, or nucleic acid analog backbone residues; a first aryl moiety linked by a linker to the first end of the nucleic acid or nucleic acid analog backbone; and a second aryl moiety that is optionally the same as the first aryl moiety, linked by a linker to the second end of the of the nucleic acid or nucleic acid analog backbone.

In some aspects, the present disclosure provides a method of detection comprising a) hybridizing a first probe nucleic acid comprising one or more divalent nucleobases, a first end connected to a first emitter moiety, and a second end connected to a second emitter moiety to a first repeat portion of a target nucleic acid; and b) hybridizing a second probe nucleic acid comprising one or more divalent nucleobases, a first end connected to a third emitter moiety, and a second end connected to a fourth emitter moiety to a second repeat portion of said target nucleic acid; wherein i) the first and second repeat portions of the target nucleic acid are associated with a repeat expansion disorder; ii) binding of the first probe nucleic acid and the second probe nucleic acid to the target presents the first or second emitter moiety in close proximity to the third or fourth emitter moiety; and iii) the presence of the first or second emitter moiety in close proximity to the third or fourth emitter moiety causes a change in emission wavelength of the emitter moieties that are in close proximity.

In some aspects, the method of detection further comprises detecting the change in the emission wavelength of the emitter moieties that are in close proximity. In some embodiments hybridizing the first probe nucleic acid to the first repeat portion increases an affinity of the second probe nucleic acid to the second repeat portion. In some embodiments the presence of the first or second emitter moiety in close proximity to the third or fourth emitter moiety causes a pi-pi stacking interaction between the first or second emitter moieties and third or fourth emitter moieties. In some embodiments, the target nucleic acid is obtained directly from a biological sample.

### Example 1

NMR, X-ray, and biochemical studies revealed that the rCAG-hairpin structures are relatively dynamic compared to that of the canonical RNA duplex, with the A-A internal bulge exhibiting large amplitude of motion. Such a molecular scaffold, comprising an internal A-A mismatch at every two canonical G-C/C-G base pairs (Figure 1 (A)), akin to a 'pothole' in the road, presents a distinct and viable receptor-like binding site for exogenous ligands. Relatively short nucleic acid ligands for targeting rCAG-hairpin structures were developed (Figure 1 (B)). Janus bases (J-bases, or JBs), E, I, and F (Figure 1 (C)) were used, which are capable of forming bivalent H-bonding interactions with nucleobases in both strands of the RNA double helix, with the conformationally-preorganized MPyPNA backbone (Figure 1 (D and E)). The J-bases described herein are part of a larger set of bifacial nucleic acid recognition elements, 16 in total, designed to bind to all 16 possible RNA basepair combinations (Figures 5A-5C). They differ from the other J-bases, including 'Janus-wedges,' in that they are uniform in shapes, sizes, and chemical functionalities and, as such, they can be combined in a modular format to recognize any combinations of RNA basepairs.

**Molecular dynamic (MD) simulations.** To assess the feasibility of the bivalent recognition design concept, we carried out MD simulations of ligand LG1 bound to an RNA duplex containing four rCAG-repeats. The C-terminal lysine residue was omitted and the MP-sidechain was replaced with a methyl group (MeγPNA) to simplify the computational modeling. The CEO, AIA, and GFC triads were built and optimized by 6-31 basis set and grafted onto the respective RNA and MeγPNA backbone. The structure of the bound RNA-LGI-RNA complex was created using NAB module of Ambertools. The final structure was solvated with water molecules and ions, energy minimized, and simulated for 100 ns. The resulting complex remained fairly stable throughout the simulation, with the four separate LG 1 ligands fitting snuggly in between the two RNA strands (Figure 6 (A)). The number of H-bonds, five for each of the CEO and GFC triads and four for AIA (Figure 6 (B)), remained intact throughout the simulation (Figure 6 (C)). Attempts to simulate binding with fewer than four LG 1 ligands, however, were unsuccessful. The complexes unraveled, with some forming contorted structures, due to fraying of the terminal base pairs (Figure 6 (D and E)).

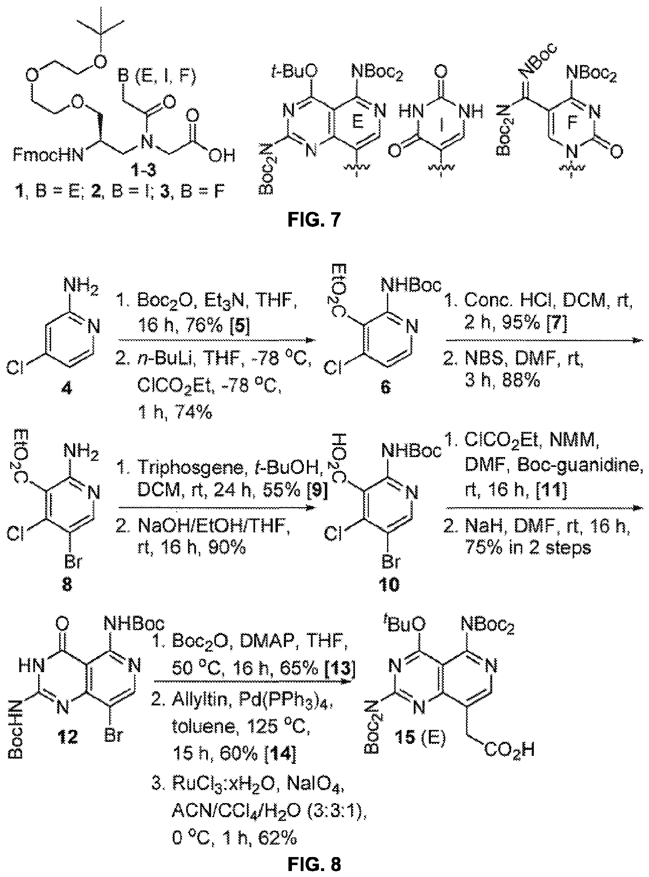

**Synthesis of chemical building blocks and ligands.** We synthesized J-bases E and F, along with the corresponding JB-MPγPNA monomers **1** through **3** (Figure 7). J-base I was not prepared since it was commercially available. E and F were synthesized according to Scheme 1 (Figure 8) and Scheme 2 (Figure 9), respectively. The Boc protection/deprotection sequence in compounds **5** through **9** was found to be preferable for improving the chemical yield of NBS reaction and in suppressing side-reactions in the subsequent condensation and cyclization steps. It was discovered that further protection of **12** was preferable for Stille coupling to proceed smoothly. This was accomplished with Boc-anhydride, carried out at an elevated temperature. Despite the apparent bulkiness, we had no issue coupling E to the MPγPNA backbone or in the assembly of the corresponding monomer on MBHA-resin. The core structure of F, 4-amino-2-(methylthio)pyridine-5-carbonitrile **(17),** was prepared according to the published protocols. Subsequent conversion of the cyano group to amidine followed by Boc-protection yielded **21.** Attempts to fully protect the exocyclic amines with a large excess of Boc-anhydride under various conditions, however, were unsuccessful-as this led to the formation of multiple spots on TLC with different numbers of Boc-groups that were difficult to separate by column chromatography. To address this challenge, we carried out Boc-protection in two steps. Subjecting **22** to oxidation and hydrolysis, followed by alkylation and hydrogenolysis yielded F. Once prepared, E **(15),** F **(26),** and I **(27)** were coupled to the MPγPNA backbone (Figure 10, Scheme 3). Removal of the Alloc-protecting group yielded the desired monomers **1** through **3.** Ligands LG1, LG2, and LG3, along with LG2P, which is in opposite (parallel) orientation of LG2, were prepared on MBHA-resin using PAL-linker and HBTU as a coupling reagent. LG2P was included as a negative control, since the parent LG2 ligand showed the most promise for binding rCAG-repeats. A lysine residue was incorporated at the C-terminus to improve water solubility. Upon completion of the last monomer coupling, ligands were cleaved from resin, precipitated with diethyl ether, purified by RP-HPLC, and confirmed by MALDI-TOF mass spectrometry (Table D).

**Table D: MWs of LG1, LG2, LG3, LG2P, and the parent compound M.**

| Ligand | Sequence | Calc. m/z | Obs. m/z |
|---|---|---|---|
| LGI | H-FIE-Lys-NH2 | 1361.6 | 1365.1 |
| LG2 | H-EFI-Lys-NH2 | 1361.6 | 1362.7 |
| LG3 | H-IEF-Lys-NH2 | 1384.6* | 1384.8* |
| LG2P | H-IFE-Lys-NH2 | 1361.6 | 1363.2 |
| LG2N (M) | H-Cys(SEt )-EFI-X-Lys-NH2 | 1698.7 | 1701.4 |

| | | | |
|---|---|---|---|
| *With Na, | | | |

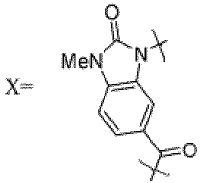

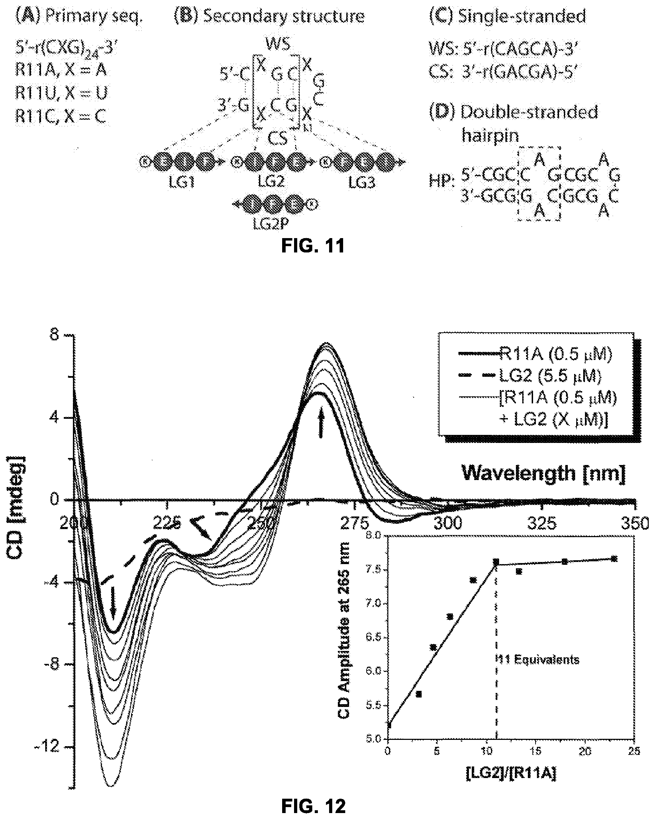

**Target selection and sample preparations.** A series of model RNA targets were chosen for binding study (Figure 11). The R11X series comprised 24 rCXG-repeats (Figure 11 (A)), which, upon adopting a secondary hairpin structure, provided 11 binding sites for ligands (Figure 11(B)). R11A contained the perfect-match (A-A), while R11U and R11C contained the respective U-U and C-C mismatches for ligand binding. R11G (X=G) was not ascertained since it has shown to be capable of forming G-quadruplex as well as hairpin structures, which could confound the interpretation of the experimental results. Single-stranded RNA targets, WS (Watson Strand) and CS (Crick Strand), with the LG2 binding-site underlined (Figure 11 (C)), as well as double-stranded hairpin HP containing a single ligand binding-site (Figure 11 (D)), were also examined. The samples were prepared by incubating the pre-annealed RNAs with respective ligands in 0.1X PBS buffer (1 mM NaPi, 13.7 mM NaCl, 0.27 mM KCl; pH 7.4) at 37 °C. This particular buffer was chosen based on our initial screen, in which we found that R11A adopted a stable hairpin structure and that ligands were able to bind.

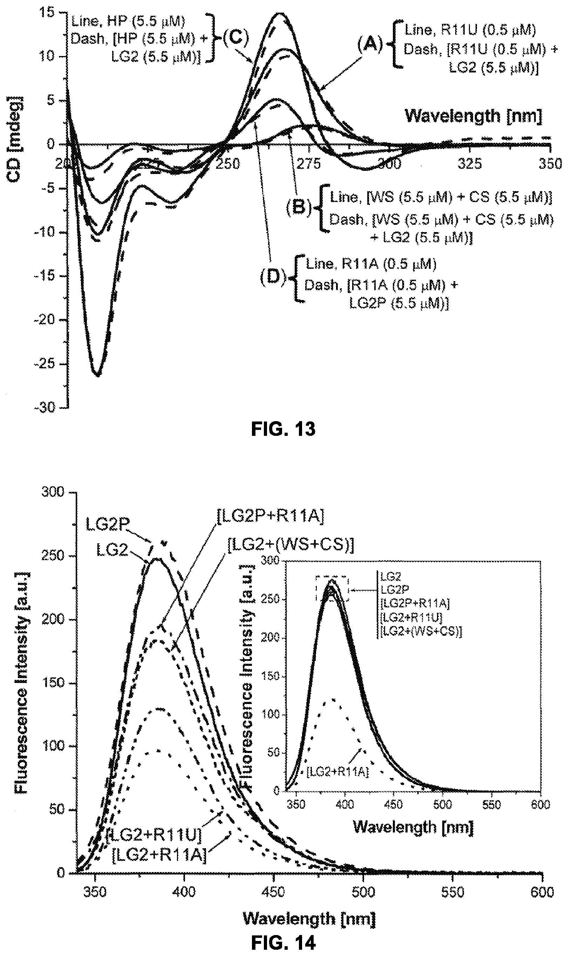

**Spectroscopic characterization of ligand binding.** A combination of UV-Vis and circular dichroism (CD) was employed to determine the binding properties of ligands. UV-Vis measurements revealed that all three ligands were able to bind R11A. The evidence for their interaction can be gleaned from the hypochromicity and bathochromic shift in the absorption of [R11A+ligand] at 252 and 330 nm. The UV-absorption in the 275-375 nm regimes correspond to the π-π* transition of E-base. This finding was corroborated by CD data, which revealed a marked increase and red-shift in the signals at ~270 nm. Among this series, LG2 exhibited the largest differential in CD amplitude. CD titration of LG2 with R11A reached a saturation point at 11:1 ratio, consistent with the predicted number of ligand binding-sites of R11A (Figure 12). We made the presumption that R11A adopted a uniform hairpin structure, since it was thermally annealed prior to the addition of ligands; however, other combinations of intra- and inter-molecular folds are also possible. In contrast, no significant differences in the CD signals were observed following the incubation of LG2 with the mismatched R11U (Figure 13 (A)) and R11C (data not shown), single-stranded WS and CS (Figure 13 (B)), double-stranded HP containing a single binding-site (Figure 13 (C)), or the mismatched-orientation LG2P with R11A (Figure 13 (D)). Since the results of the R11U and R11C mismatches were nearly identical in every respect, all the discussions on base-mismatch from this point forward are centered on R11U. Taken together, these results show that the interactions between ligand and RNA occurred in a sequence- and orientation-specific manner, with a preference for double-stranded over single-stranded RNA target, and in favor of one with multiple consecutive binding-sites over one in isolation.

**Confirmation of ligand binding by fluorescent measurements.** To further corroborate the UV-Vis and CD findings, we measured the fluorescent signals of ligands with and without RNA targets following an excitation at 330 nm (λₘₐₓ of E-base). We observed a significant fluorescent quenching of LG3 compared to that of LG1 and LG2. Such a drastic reduction in the fluorescent signals could be due to photo-induced electron transfer quenching, which is expected to be most efficient for LG3 since the fluorophore (E) is stacked between two other J-bases. This interpretation is consistent with the observation that the fluorescent signals of LG3 were fully recovered upon heating (data not shown). With all three ligands, the emission signals were further reduced upon the addition of R11A, but most dramatically with LG2, 60% reduction with LG2 as compared to 35 and 33% with LG1 and LG3, respectively. We observed a similar fluorescent quenching pattern upon the incubation of LG2 with R11U, [WS+CS], HP, and LG2P with R11A (Figure 14)-although less dramatic as compared to that of LG2 with R11A. A similar observation was made with a related class of bicyclic pyrimidine analogues, indicating that the fluorescent yields of these fused-ring fluorophores are highly sensitive to the local environments. To test the hypothesis that the nonspecific binding of ligand to RNA can lead to fluorescent quenching, we incubated the respective RNA targets with pentamidine prior to adding LG2 and vice versa. The data showed that the fluorescent signals of LG2 with R11U and [WS+CS], as well as that of LG2P with R11A, were fully recovered, except in the case of LG2 with R11A, which remained suppressed (Figure 14, *Inset).* This result is consistent with LG2 binding R11A through a different mode from that of pentamidine, presumably *via* the prescribed bivalent H-bonding interactions (Figure 1B and C).

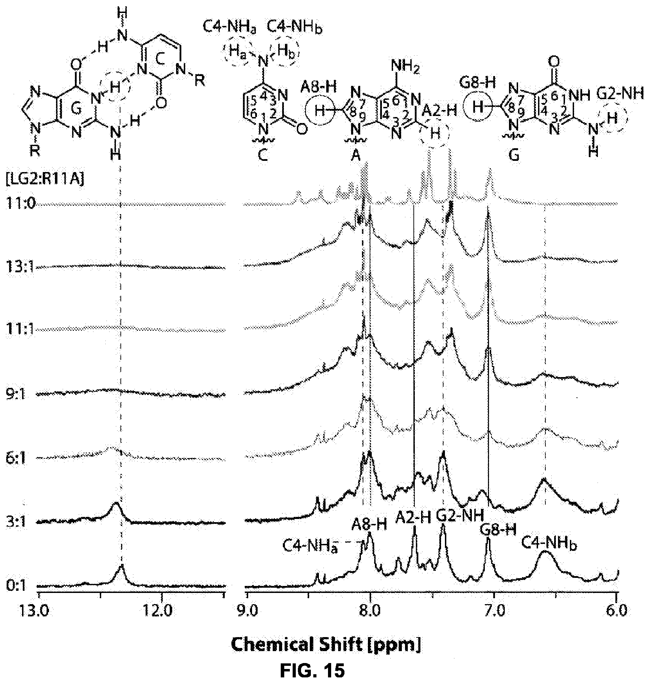

**NMR titration.** To gain insight into the binding mode of LG2, we performed a series of multinuclear and multidimensional NMR experiments with LG2, R11A, and in combination. The samples were prepared in the same 0.1X PBS buffer as before, containing a 9:1 volume ratio of H₂O:D₂O The NOESY data were collected at 300, 200, and 100 ms mixing times to obtain proton-proton distance, while COSY experiments were performed to map the proton spin-system of each residue. The expectation was that H-bonding interactions between LG2 and R11A would result in line-broadening and downfield chemical shift of the imino proton signals of the canonical G-C/C-G pairs due to base-pair opening, and in an emergence of a new set of imino proton signals due to the formation of H-bonds between ligand and RNA. Consistent with the CD data, ¹H-NMR experiments revealed that R11A adopted a stable hairpin structure in 0.1X PBS buffer, as evident by a sharp imino proton signal at 12.35 ppm. Variable-temperature measurements further supported this chemical shift assignment, showing a gradual decay in peak intensity with increasing temperature while that of the non-exchangeable protons remained fairly constant. Partial assignments of the nucleobase protons were made by NOESY experiments. As predicted, the imino proton signal of R11A was gradually broadened and downfield-shifted upon the addition of LG2 (Figure 15). Moreover, the chemical shifts of C4-NH and G2-NH were significantly affected, an indication of their interactions with ligand. Despite efforts, we were not able to assign and thus monitor the chemical shift of A6-NH upon titrating LG2 to R11A due to the degeneracy in the repeated sequence. Nonetheless, the result indicates that the G-C/C-G base pair openings are mediated by ligand. However, we did not observe any new imino proton signals in the 10-20 ppm regimes, as would be expected for H-bond formation between ligand and RNA.

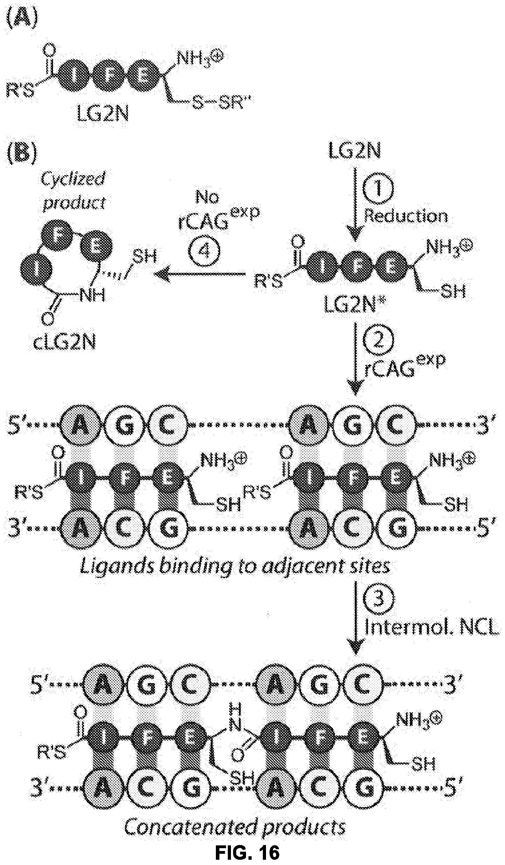

**Template-directed native chemical ligation (NCL).** The weak and transient interactions of ligand with RNA, as observed with LG2 and R11A, are unlikely to produce a meaningful biological response. To further improve the binding affinity of LG2, we synthesized a second LG2 derivative, LG2N (Figure 16 (A)), which contained a C-terminal thioester and N-terminal cystine. This ligand was prepared using a second-generation N-acylurea linker. The dual-functional probe design exploits the conformational preorganization of MPγPNA in preventing the two functional groups from spontaneously reacting with each other upon the reduction of disulfide-bond. The cystine group was employed to provide a greater control in probe handling. Prior studies revealed that a ligand of the same length comprising all natural nucleobases has a reduced (acyclic) half-life of ~1 hr at a physiological temperature (37 °C). We expected a similar half-life for LG2N*. Figure 16 (B) depicts the reaction pathways of LG2N following the reduction of disulfide-bond, the expected chemical state of LG2N in the reducing intracellular environments. In the presence of RNA target, ligand was expected to form transient bivalent H-bonding interactions with nucleobases of the RNA target next to one another as the result of intermolecular base-stacking (Step 2). Upon the cleavage of disulfide-bond, ligand would undergo template-directed NCL to form concatenated products that bind an RNA template more tightly (Step 3). In the absence of the RNA target, ligand would self-deactivate by undergoing intramolecular NCL reaction to form cyclic products (Step 4).

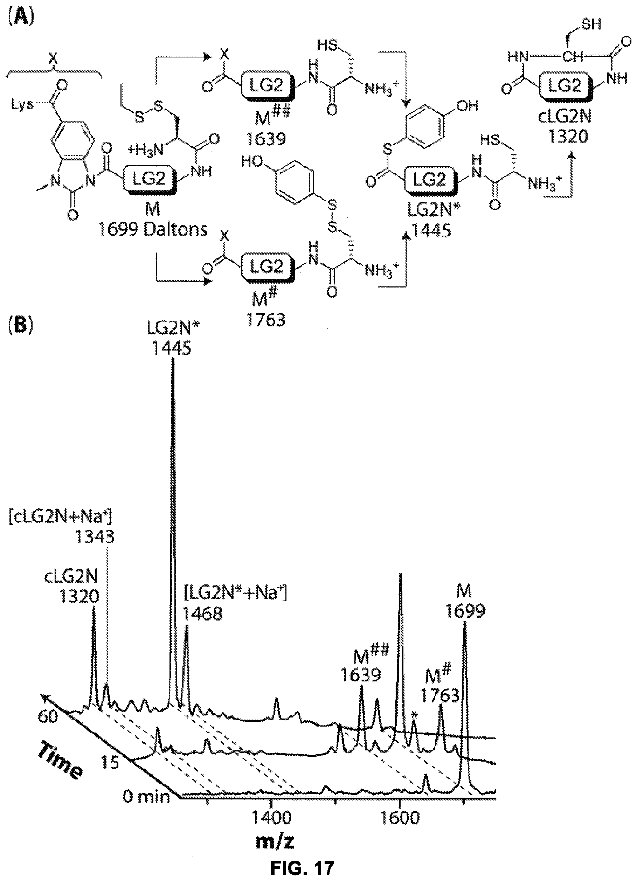

To confirm the prediction that LG2N would remain stable for a finite period following the reduction of disulfide-bond before undergoing a cyclization reaction, we monitored the reaction progress of the parent compound M from which LG2N* was derived in-*situ* (Figure 17 (A)). We selected MALDI-TOF over HPLC and other analytical techniques in the quantification of the reaction products because of the relatively short time-scale of intramolecular NCL, which can be accomplished with the former in less than 5 min. Attempts were made to quench the reaction with an electrophile prior to HPLC analysis; however, such an effort was not successful due to the rapid intramolecular reaction of ligand. 4-Mercaptophenol (4MP) and 2-mercaptoethanol (2ME) were investigated as possible reducing agents. Both yielded similar kinetic profiles for the parent compound M; however, the former yielded an intermediate with an overlapping mass-to-charge ratio (m/z) as that of the hydrolyzed parent compound, making it difficult to differentiate one from the other. For this reason, we selected 4MP in the reaction monitoring by MALDI-TOF and in the gel-shift assay, while 2ME was employed in the subsequent melting experiments due to its transparency in the nucleobase absorption regions. Our data revealed that upon the addition of 4MP to the parent compound M, two reactive species corresponding to that of the transesterification (M#) and the C-terminal thioester (M##) were initially formed (Figure 17 (A)). They persisted for <15 min before converting into the reactive LG2N* intermediate, and finally into the cyclic product

(cLG2N) (Figure 17 (B)). LG2N* was stable at a physiological temperature. It existed as a major product at a 1-h reduction time-point, accounting for~ 75% of the total products in the mixture, with the remaining 25% being mostly cLG2N. LG2N* has a half-life of~ 3 h, roughly three times longer than that of the natural counterpart. We attributed the chemical stability of LG2N* to the expanded aromatic ring-size of E-base, which provides better base-stacking interaction and, thus, makes the ligand less conformationally flexible.

Next, we performed UV-melting experiments to determine the effect of template-directed NCL on the thermal stability of RNA. The samples were prepared in a 0.1X PBS buffer at a 2:1 ratio of LG2N to the number of binding-sites of R11A and incubated at 37 °C for 16 h prior to carrying out UV-melting analyses. As expected, without the reducing agent, LG2N had minimal effect on the melting transition (Tₘ) of R11A, with ΔTₘ of ~+1°C at most. A similar finding was made with LG2, which neither contained a C-terminal thioester nor an N-terminal cysteine. However, in the presence of 2ME, whether the incubation of [LG2N+R11A] was made at an ambient temperature or at 37 °C, the results were similar. A significant enhancement in the Tₘ of R11A appeared in both cases, in the range of 59-68 °C. The derivative of the melting curves showed broad sigmoidal distributions, suggesting the presence of a range of concatenated products being formed and bound to RNA template. This finding was expected for template-directed synthesis. Despite the similarity in the Tₘ, the two melting curves are distinct in pattern, with the one incubated at an elevated temperature showing an inverse optical absorption in the 25-50°C range. This melting behavior is characteristic of hydrophobic/aromatic interaction, a phenomenon that has been previously observed with thermophilic foldamers and other aromatic systems, such as perylene and pyrene. We attributed the inverse intensity distribution to the interactions of J-bases of the concatenated products, which, presumably, were formed in greater quantities at 37 °C than at an ambient temperature. Upon heating, the hydrophobic interaction became more pronounce up to a certain point (~50 °C), beyond which repulsion ensued. The enhancement in the thermal stability of R11A is consistent with the formation of NCL products and their binding to RNA template.

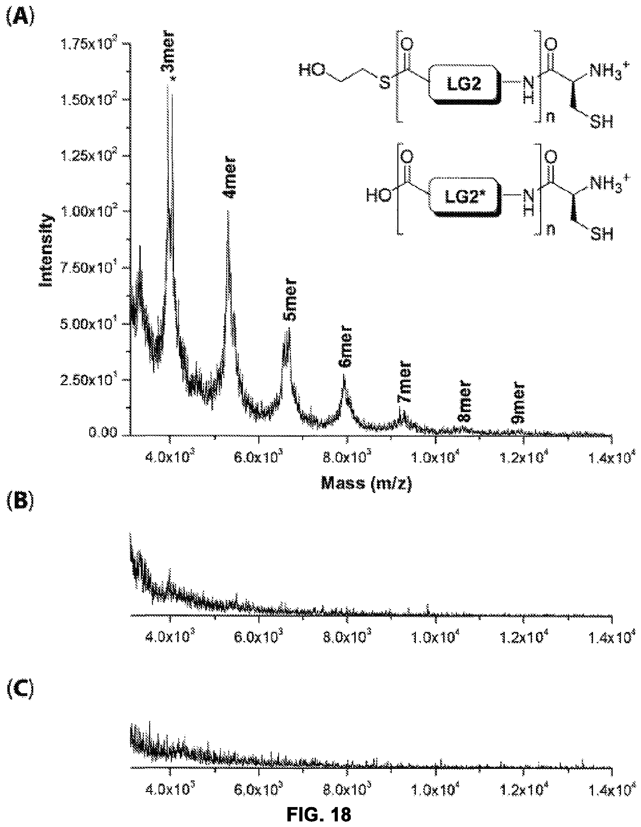

To confirm that the enhancement in the Tₘ of R11A was due to template-directed concatenation and binding of the resultant products to RNA template, we carried out MALDI-TOF analyses of the UV-melting samples prior to heating. Formation of the concatenated products is apparent from the emergence of new peaks with progressively larger m/z values with a step size of ~1319 Daltons, corresponding to the mass of ligand (Figure 18 (A)). These concatenated products, however, were not observed with the mismatched R11U (Figure 18 (B)) or LG2N alone without the target (Figure 18 (C)). Likewise, there was no evidence of the concatenated products being formed with single-stranded [WS+CS] or double-stranded HP (data not shown). This result is consistent with the occurrence of template-directed synthesis, mediated by the R11A hairpin structure.

Selective binding of the concatenated ligand. To determine whether LG2N can discriminate rCAG^{exp} from the normal repeats, we carried out a competitive binding assay. Two RNA targets containing r(CAG)ₙ-repeats in the normal (R24A, n=24, the same as R11A) and in the pathogenic (R127A, n=127) range, along with the mismatched r(CUG)₉₆ control (R96U) were employed. Upon adopting the respective hairpin motif, these RNA transcripts would provide 11, 62, and 47 binding-sites for LG2N. Two sets of samples were prepared, one in 0.1X PBS and another in a physiologically relevant buffer (10 mM NaPi, 150 mM KCI, 2 mM MgCl₂; pH 7.4). In both sets, equimolar mixtures ofR24A (100 nM strand-concentration, 1.1 µM binding-sites) and R127 A (18 nM strand-concentration, 1.1 µM binding-sites) were incubated with various concentrations of LG2N, along with 4 MP and TCEP at 37 °C for 24 h. TCEP was included to ensure that the disulfide bond was completely reduced. The reaction mixtures were analyzed by agarose-gel and stained with SYBR-Gold for visualization. Inspection of Figure 19 revealed that LG2N bound preferentially to R127A over R24A, as evident by the faster rate of disappearance of the top two bands compared to that of the bottom band in lanes 2 through 5. Such a binding event only occurred in the presence of reducing agents and with the perfectly matched RNA target, since no evidence of binding was observed in the absence of 4MP and TCEP (compare lane 6 to lane 1) and with the mismatched R96U (compare lane 8 to lane 7). We did not observe any shifted bands being formed, as would be expected for the complexation of ligand with RNA-suggesting that SYBRGold was not able to intercalate the RNA-ligand complex. This observation was not surprising considering that ligand binding is known to cause a dramatic change in the conformation of RNA (Figure 6). In contrast, we did not observe any evidence of ligand binding taking place at a physiologically relevant ionic strength (Figure 20), indicating that RNA hairpin was not able to mediate template-directed ligand oligomerization under such a condition.

Many neuromuscular disorders, more than 20 in counting, including HD, myotonic dystrophy type 1 (DMI) and type 2 (DM2), spinocerebella ataxias (SCAs), fragile X syndrome (FXS), Friedreich's ataxia (FRDA), and a subpopulation of amyotrophic lateral sclerosis (ALS, or Lou Gehrig's disease), manifest as a result of unstable repeat expansions. An expansion in the coding region of a gene can lead to an altered protein function, whereas that occurring in the noncoding region can cause a disease without interfering with a protein sequence through toxic-gain of RNA function and/or inadvertent production of deleterious polypeptides *via* RAN-translation. Many of these genetic disorders are autosomal dominant, requiring an expansion (or mutation) in only one allele to cause a disease. As such, one way to interfere with such a disease pathway would be to target the affected allele or the corresponding gene transcript. Between the two, the latter provides greater accessibility for exogenous molecules and greater recognition specificity for bivalent ligands, such as JB-MPyPNAs, because of its propensity to adopt an imperfect hairpin structure.

We concentrated our effort on rCAG-repeats because of their expansive genetic penetrance in HD, as well as in other neurodegenerative disorders, including but not limited to, MJD, SBMA, DRPLA, and SCAs. A molecule that selectively binds the mutant *htt* transcript and interferes with the aforementioned disease pathways can be used to treat not only HD, but also other related genetic disorders. The nucleic acid ligands described herein differ from the conventional antisense agents in several respects. First, they bind the CAG-RNA hairpin motif through bivalent H-bonding interactions. Second, they are relatively small in size, a single triplet-repeating unit in length. As such, they are easier to chemically synthesize, structurally modify, and scale-up, *via* solution- as opposed to solid-phase chemistry. Third, with molecular weight on the outer fringe of small molecules, such a 'millamolecular' system generally possesses more favorable pharmacokinetic property than a typical antisense agent. Fourth, J-base recognition is more sequence-specific and selective. It is more specific because a single-base mismatch that would normally occur on one face of a natural nucleobase would occur on both faces of a J-base; and it is more selective, favoring double-stranded over single-stranded RNA target, because of the large differential in binding free energy of the two resultant products. However, unlike small molecules, or riboswitches, recognition of J-bases follows a defined set of rules, *via* bivalent Watson-and-Crick H-bonding interactions, as opposed to a more diverse combination of attractive forces. Furthermore, JB-ligand design is modular. As such, ligands could be prepared and modified to bind to any sequence of RNA-repeats. The weak and transient interactions of JB-ligands with normal-length rCAG-repeats at low salt concentrations is desirable at the initial design stage, as this will allow for further improvement in the recognition selectivity by tuning the binding affinity of ligands and by adjusting the ionic strength of buffers.

### MATERIALS AND METHODS

**UV-melting analyses.** All UV-melting samples were prepared by mixing ligands with RNA targets at the indicated concentrations in 0.1X PBS buffer, and annealed by incubation at 90 °C for 5 min followed by a gradual cooling to room temperature. UV melting curves were collected using Agilent Cary UV-Vis 300 spectrometer equipped with a thermoelectrically controlled multi-cell holder. UV-melting spectra were collected by monitoring UV-absorption at 260 nm from 25 to 95 °C in the heating runs, and from 95 to 25 °C in the cooling runs, both at the rate of 1 °C per min. The cooling and heating curves were nearly identical, indicating that hybridization process was reversible. The recorded spectra were smoothed using a 20-point adjacent averaging algorithm. The first derivatives of the melting curves were taken to determine the melting temperatures of the complex.

**CD analyses.** The samples were prepared in 0.1X PBS buffer. All spectra represent an average of at least fifteen scans collected at 100 nm/min rate between 200 and 375 nm, in a 1-cm path-length cuvette at 25 °C. CD spectrum from buffer solution was subtracted from the sample spectra, which were then smoothed via a five-point adjacent averaging algorithm. Steady-State fluorescent measurements. All steady-state fluorescence samples were prepared by mixing ligands with RNA at the indicated concentrations in 0.1IX PBS buffer, and annealed by incubation at 90 °C for 5 min followed by a gradual cooling to 37 °C. The samples were incubated at 37 °C for 1 hr before the measurements. Steady-state fluorescence data were collected at 25 °C by Cary Eclipse Fluorescence Spectrometer. (εₑₓ= 330 nm, εₑₘ= 340-600 nm) with slit size.

**Competitive binding assays.** R24A was purchased from IDT. R127A [r(CAG)₁₂₇] and R96U [r(CUG)₉₆] were prepared as previous described. R24A, R126A, and R96U were prepared in 0.1X PBS buffer and annealed by heating to 90 °C for 5 min, followed by a gradual cooling to room temperature. Ligand and RNA were mixed at the indicated concentrations and incubated at 37 °C for 24 h. The samples were then loaded onto 2% agarose-gel with 1X Tris-borate buffer and electrophoretically separated at 100 V for 25 min. The gel was stained with SYBR-Gold and visualized by UV-Transilluminator.

### General Techniques

All starting materials and chemicals were purchased from commercial suppliers and used without further purification unless otherwise stated. All reactions were carried out under a nitrogen atmosphere with dried solvents under anhydrous conditions unless otherwise noted. Removal of solvent in vacuo refers to distillation using a rotary evaporator attached to a high vacuum pump. Products obtained as solids, syrups, or liquids were dried under high vacuum. Analytical thin-layer chromatography was performed on precoated silica plates (60F-254, 0.25 mm thickness); compounds were visualized by UV-light or by staining with ninhydrin or iodine, or heat as developing agents. NMR spectra were recorded either on a 300 or 500 MHz with CDCl₃, DMSO, and D₂O as the solvents and TMS as an internal standard. The following abbreviations were used to explain the multiplicities: s=singlet, d=doublet, t=triplet, q=quaiiet, m=multiplet, ddt=doublet of doublet of triplet, dt=doublet of triplet, td=triplet of doublet, ABq=ABquartet, dd=doublet of doublet, tt=triplet of triplet, br=broad, wtc. High-resolution mass spectroscopy (HRMS) was performed using an ESI-ESI-TOF mass analyzer and DART analyzer. Low-resolution mass spectroscopy (LRMS) was performed using an ESI-Ion Trap-MS mass analyzer. Unless otherwise stated, a C18 (15 cm x 2.1mm, 5 µm particles) column was used with a linear elution gradient from 95% H₂O (0.01% TFA) to 95% MeCN (0.01 % TFA) in 40 min (method A) at a flow rate of 1.0 mL/min for oligomer purification on RP-HPLC. The MALDI spectra were measured using TOF/TOF spectrometer. Absorption profiles were recorded on UV-Vis spectrophotometer. Circular dichroism (CD) spectra were recorded on Spectropolarimeter. Fluorescence (emission) was recorded on Fluorescence Spectrophotometer.

### MD simulations

The C-terminal lysine residue was omitted from the γPNA and the MP-side chain was replaced with a methyl group (MeγPNA, published X-ray crystal structure, PDB-ID 3PA0). The CEG, AIA, and GFC triads were built using chimera1 and optimized by HF/6-31G* basis set in Gaussian. The helical structure of the bound RNA-HD1-RNA complex was created from the triads using NAB module of Ambertools and the modified MeγPNA backbone from the crystal structure was grafted onto the helical structure (as shown in Figure 2A, initial structure). Four such RNA-HD1-RNA complexes were generated where RNA was a duplex containing four rCAG-repeats and the number of HD1 ligand bound to RNA was varied from one to four. Each RNA-(HDI)ₙ-RNA complex was solvated with TIP3P⁴ water molecules, and ions were added to neutralize the charges. The systems were energy minimized and then heated to 300K under a harmonic restraint of 25 Kcal/mol/Å on the atoms of RNA and HD1 ligand. The restraints were then gradually released in a series of six short simulations and finally unrestrained NPT simulations were run at 300K and 1 bar pressure for 100 ns using Nose-Hoover thermostat and Parinello-Rahman barostat, respectively. Electrostatic interactions were treated using particle mesh Ewald method and all the simulations were done using GROMACS Amber99-parmbsc0 force field with χOL3 modifications was used for RNA and the general amber force field was used to create the force field parameters for HD1 ligand.

### Resin loading [(MP)(PAL)]

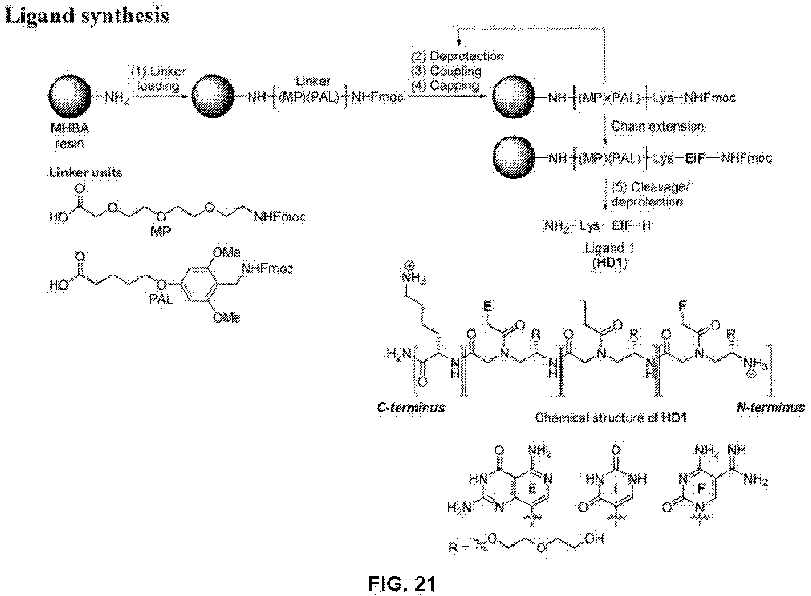

(1) MP loading. 1 g of MBHA resin (1 mmol/g, Peptide International, RMB-2100-PI) was soaked in DCM in a reaction vessel for 1 h, then washed with DCM (3X) and 5% DIEA in DCM (3X), see Figure 21. Upon confirmation by Kaiser-test that the amine groups were neutralized (blue), in a 15-mL canonical tube were added the following solutions in sequential order: 3.5 mL of NMP, 450 µL of Solution A, 460 µL of Solution B, and 550 µL of Solution C. The mixture was vortexed for 10 sec and allowed to stand for 3 min before adding to resin in the reaction vessel. The reaction was allowed to proceed for 1 h with gentle shaking. The reaction mixture was discarded with positive air pressure, and resin was washed with DMF (3X), DCM (3X), 5% DIEA in DCM (1X), and DCM (3X). Resin was capped by adding the Capping Solution to the reaction vessel and gently agitated the vessel for 45 min (2X). After discarding the last capping solution, resin was washed with DCM (3X), and completion of capping was confirmed by Kaiser-test (pale yellow).
   Solution A: 43 mg Fmoc-MP in 500 µL NMP (0.200 M)
   Solution B: 87 µL DIEA in 913 µL pyridine (0.500 M)
   Solution C: 55 mg HATU in 750 µL NMP (0.201 M)
   Capping Solution (Ac₂O/NMP/pyridine: 112/2): 2 mL Ac₂O, 4 mL NMP, and 4 mL pyridine
(2) PAL and Lys loading (1 g of resin from above). Fmoc-protecting group was removed by treating resin with a 20%-piperidine solution in DMF (2X) for 7 min each, followed by washes with DMF (3X) and DCM (3X). Upon confirmation that Fmoc was successfully removed by Kaiser-test (blue), the coupling solution was prepared by mixing the following materials: 3 mL of 0.2 M Monomer Solution, 1.5 mL of 0.52 M DIEA Solution, and 1.5 mL of 0.39 M HBTU Solution. The mixture was activated for 3 min before adding to resin. The reaction was allowed to proceed for 30 min with gentle agitation of the reaction vessel. Completion of the reaction was confirmed by Kaiser-test (pale yellow). Resin was washed with DMF (3X), 5% DIEA solution (1X), and DCM (1X), and the unreacted amines were capped by adding the Capping Solution to resin and gently agitated the reaction vessel for 30 min. Upon removal of the capping solution, resin was washed with 20% piperidine (2X), DMF (2X), and DCM (2X).
   Monomer Solution: 303 mg Fmoc-PAL in 3 mL NMP (0.200 M) 281 mg Fmoc-Lys(Boc)-OH in 3 mL NMP (0.200 M)
   DIEA Solution: 364 µL DIEA in 3.638 mL DMF (0.52 M)
   HBTU Solution: 740 mg HBTU in 5 mL DMF (0.39 M)
   Capping Solution: (Ac₂O /NMP/pyridine: 1/25/25 vol.)

**Fmoc-deprotection.** Fmoc-protecting group was removed by treating resin with a 20% piperidine (0.5 mL for 50 mg Resin) solution in DMF (2X) for 7 min each, followed by washes with DMF (3X) and DCM (3X). The Fmoc deprotection was confirmed by Kaiser-test (Blue).

Monomer coupling. Upon confirmation that Fmoc was successfully removed by Kaiser-test (blue), resin was washed with DMF ( 4X) and DCM (5X). The coupling solution was prepared by mixing the following materials: 150 µL of 0.2 M Monomer Solution, 75 µL of 0.52 M DIEA Solution, and 75 µL of 0.39 M HBTU Solution. The mixture was activated for 10 min. Resin was washed with pyridine (1X) before monomer solution was added to resin. The reaction was allowed to proceed for 2-4 h with gentle agitation of the reaction vessel. Completion of the reaction was confirmed by Kaiser-test (pale yellow). Resin was washed with DMF (3X) and DCM (3X).

**Capping.** The unreacted amines were capped with freshly prepared capping solution. Capping solution (1 :25:25; acetic anhydride:NMP:Py) was added to resin, and the reaction vessel was agitated for 4 min. Resin was washed with DMF (4X) and DCM (5X).

**Cleavage.** After the final Fmoc-deprotection step, resin was washed with DMF (5X) and DCM (8X). Resin was dried under vacuum for 15 min. To the dried resin, freshly prepared 95% TFA:5%m-cresol (0.6 mL for 50 mg resin) was added and the reaction vessel was kept at a standby mode. After 1 h at room temperature, the TFA solution was collected in centrifuge canonical tube. Again, cleavage solution (0.5 mL for 50 mg resin) was added to resin, and the reaction vessel was allowed to stand for 30 min. The TFA solution was combined with the previously collected solution.

**Precipitation.** To the collected TFA solution, cold dry diethyl ether (-60 °C, 14 mL) was added and shaken. The precipitation occurred within 30 min. The precipitated oligomer was collected by centrifugation and washed with cold diethyl ether (2x).

**Purification.** The crude oligomer was dissolved in 0.5 mL of 95% water, 5% acetonitrile, and 0.1 % of TFA. The resultant solution was purified by reverse phase analytical column.

### Example 2 - Monomer synthesis

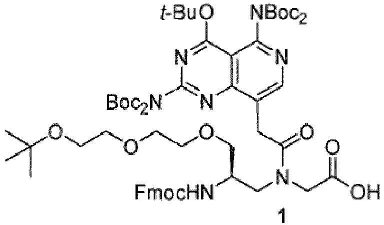

**Monomer E (1):** Palladiumtetrakis(triphenylphosphine) (28.22 mg, 24.42 µmol) was added to a mixture of phenyl silane (0.12 mL, 0.98 mmol) and allyl monomer 29a (0.60 g, 0.49 mmol) in anhydrous dichloromethane (10 mL) at room temperature. The complete conversion of starting material to the corresponding acid was observed by TLC (Eluents: EA:Hex (SO:SO); Rf for 29a 0.6; R_{f} for product= 0.0 (dragging)) over a period of 15 h. To the reaction mixture, silica gel was added at room temperature and solvents were removed on a rotary evaporator. The crude silica gel absorbed product was purified by flash silica gel chromatography to get the desired product. Yield: 0.44 g, 7S%. ¹H NMR (S00.13 MHz, DMSO-d6, rotamer): δ 1.08/1.09 (s/s, 9H), 1.25-1.28 (m, 18H), 1.37-1.38 (m, 18H), 1.41/1.6S (s/s, 9H), 3.12 -3.64 (m, 13H), 3.77 -3.90 (m, 2H), 3.90-4.00 (m, 2H), 3.99- 4.4.34 (m, 4H), 7.21 - 7.49 (m, 5H), 7.54 - 7.79 (m, 2H), 7.89 (d, J = 7.2 Hz, 2H), 8.55/8.64 (s/s, 1H); ¹³C NMR (125.77 MHz, DMSO-d6, rotamer): δ 27.2-27.6 (18C, ^{t}Bu), 35.8, 46.7, 54.9, 59.7, 60.6/60.6 (2C), 65.6/65.6, 69.6/69.6, 69.8/69.9, 70.4/70.4, 72.2/72.2, 82.1 (3C), 82.2, 83.2, 83.4, 86.2, 108.1, 120.1 (2C), 125.2, 127.0(4C), 127.6(4C), 140.7 (2C), 143.8/143.8, 147.9, 149.2/149.3, 149.3, 149.5, 149.7, 155.0/155.9, 156.3/156.5, 162.3, 166.6/166.6; HRMS: calculated m/z for.[C₆₁H₈₅N7O₁₇+H]: 1188.6080, observed: 1188.6071.

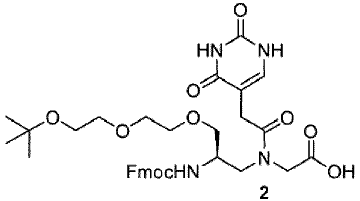

**Monomer I (2):** This compound was prepared using the above-mentioned procedure for compound 1, starting with compound **29b** (3.10 g, 4.39 mmol) as the starting material. TLC (Eluents: MeOH:DCM (5.95); Rr for **29b** = 0.2; R_{f} for **2** = 0.0).Yield: 2.22 g, 76 %. ¹H NMR (300.13 MHz, DMSO-d₆): δ 1.09 (bs, 9H), 3.11 - 3.52 (m, 15H), 3.87 - 3.96 (m, 2H), 4.24 (dd, J = 13.4, 5.4 Hz, 4H), 7.19-7.44 (m, SH), 7.69 (d, J = 7.5 Hz, 2H), 7.89 (d, J = 7.4 Hz, 2H), 10.71-10.75 (m, 1H), 11.06 (bs, 1H), 12.46 (bs, 1H); ¹³C NMR (126 MHz, DMSO-d₆, rotamers): δ 27.8(3C), 47.2/47.2, 61.1(3C), 65.6/66.0, 70.1 (2C), 70.3/70.4, 70.9 (3C), 72.7, 107.8, 120.6-128.1 (10C), 139.7/140.1, 141.2 (2C), 144.3, 151.8, 156.3, 164.6, 170.9; HRMS: calculated m/z for [C₃₄H₄₂N₄O₁₀+H]: 667.2979, observed: 667.2988.

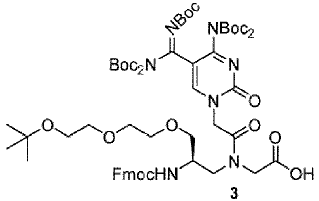

**Monomer F (3):** This compound was prepared using the above-mentioned procedure for compound **1,** starting with compound **29c** (2.50 g, 2.00 mmol) as the starting material. TLC (Eluents: EA:Hex (50:50); R_{f} for **29c** =0.6; R_{f} for 3=0.0 (dragging)). Yield: 1.94 g, 80 %. ¹H NMR (300.13 MHz, DMSO-d6, rotamer): δ 1.08 (s, 9H), 1.35 (s, 9H), 1.36 (s, 9H), 1.41/1.42 (s/s, 18H), 3.20- 3.62 (m, 20H), 3.77 - 4.15 (m, 4H), 4.18 - 4.34 (m, 4H), 4.82 - 5.35 (m, 2H), 7.17 - 7.46 (m, 5H), 7.70 (d, J = 7.0 Hz, 2H), 7.88 (d, J = 7.5 Hz, 2H), 8.93/8.95 (s/s, 1H); ¹³C NMR (125.77 MHz, DMSO-d6, rotamer): δ 27.3-27.5 (18C, ^{t}Bu), 46.7/46.7, 60.6 (2C), 60.7 (2C), 69.6 (2C), 69.7, 69.9, 70.4 (3C), 72.2, 82.1, 82.1, 82.9, 82.9, 83.3, 83.4, 108.0, 110.8, 118.4, 120.1 (2C), 123.1, 125.2/125.3, 127.0 (4C), 127.6 (4C), 140.7 (2C), 143.8, 146.9, 149.5, 156.6, 157.1, 162.4/162.4. HRMS: calculated m/z for [C₆₀H₈₅N₇O₁₉+H]: 1208.5980, observed: 1208.5959.

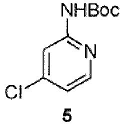

**(4-chloro-pyridin-2-yl)-carbamic acid tert-butyl ester (5):** To a stirred solution of 2-amino-4-chloro pyridine (1) (50.00 g, 0.39 mol) in 556 mL of anhydrous THF in an ice bath was added Et₃N (81.37 mL, 0.58 mol) drop-wise in an inert atmosphere over a period of 10 min. After stirring for additional 10 min at the same temperature, Boc₂O (84.88 g, 0.39 mol) in THF (100 mL) was added drop-wise over the course of 30 min followed by catalytic amount of DMAP (4. 75 g, 0.04 mol). After stirring overnight at room temperature, the reaction mixture was diluted with ethyl acetate (200 mL). The combined organic layer was filtered through a sintered funnel and the filtrate was washed with saturated solution of NaHCO₃ and dried over Na₂SO₄. Solvents were removed on a rotary evaporator to get the pure product. Yield: 67.59 g, 76 %. ¹H NMR (300.13 MHz, CDCl₃): δ 1.54 (s, 9H), 6.95 (dd, J = 5.5, 1.8 Hz, 1H), 8.12 (d, J = 1.9 Hz, 1H), 8.24 (d, J = 5.5 Hz, 1H), 9.96 (s, 1H); ¹³C NMR (125.77 MHz, CDCl₃): δ 28.5 (3C), 81.5, 112.9, 118.7, 146.2, 148.3, 152.6, 153.8; ESI-MS: calculated m/z for [C₁₀H₁₃ClN₂O₂+H]: 229.1, observed: 229.2.

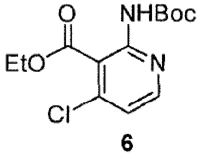

**Ethyl 2-((tert-butoxycarbonyl)amino)-4-chloronicotinate (6):** To a stirred solution of (4-chloro-pyridin-2-yl)carbamic acid tert-butyl ester (30.50 g, 0.13 mol) in 534 mL of anhydrous THF in a dry-ice-acetone bath (-78 °C) was added n-BuLi (24.86 mL, 0.27 mol, 11M) drop-wise in an inert atmosphere over a period of 1 h. After stirring for an additional 30 min at -78 °C, the ClCO₂Et (13.93 mL, 0.15 mol) was added drop-wise over the course of 10 min. The reaction progress was checked with TLC; and after complete consumption of the starting material, the reaction mixture was slowly quenched with 1 % HCl (200 mL). The resultant reaction mixture was transferred to separating funnel containing 10% HCl (50 mL) and EA (200 mL). The crude product was extracted with EA from aq. layer and the combined organic layer was dried over Na₂SO₄. The resultant solvents were removed on a rotary evaporator and the obtained crude was purified by column chromatography to get Ethyl 2-((tert-butoxycarbonyl)amino)-4-chloronicotinate as a faint yellow solid. Yield: 29.68 g, 74%. ₁H NMR (500.13 MHz, CDCl₃): δ 1.40 (t, J = 7.2 Hz, 3H), 1.50 (s, 9H), 4.41(q, J 7.2 Hz, 2H), 7.08 (d, J = 5.2 Hz, 1H), 8.31 (d, J = 5.2 Hz, 1H), 8.62 (s, 1H); ¹³C NMR (125.77 MHz, CDCl₃) δ 14.1, 28.3(3C), 62.3, 81.8, 120.8, 144.7, 149.9, 150.0, 151.3, 151.8, 164.9; HRMS: calculated m/z for [C₁₃H₁₇ClN₂O₄+Na]: 323.0774, observed: 323.0777.

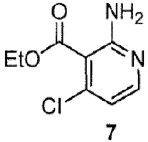

**Ethyl 2-amino-4-chloronicotinate (7):** Ethy 1 2-((tert-butoxycarbonyl)amino)-4- chloronicotinate (29.00 g, 0.10 mol) was added to a mixture of concentrated HCl (52.60 mL, 0.58 mol) and dichloromethane (64 mL) at room temperature (slow addition). After 2 h at room temperature, completion of starting material was monitored by TLC and then the reaction mixture was cooled to 0 °C and neutralized with 2N NaOH solution. The reaction mixture was transferred to a separating funnel containing 100 mL of dichloromethane and the compound was extracted into the organic layer from aq. layer. The combined dichloromethane layer was dried over anhydrous Na₂SO₄ and concentrated. The crude material was purified by silica gel column chromatography to get the pure ethyl 2-amino-4-chloronicotinate as a white solid. Yield: 18.38 g, 95%. ¹H NMR (500.13 MHz, CDCl₃): δ 1.41(t, J=7.1Hz,3H), 4.42 (q, J = 7.1 Hz,2H), 6.13 (bs, 2H), 6.68 (d, J = 5.3 Hz, 1H), 7.97 (d, J = 5.4 Hz, 1H); ¹³C NMR (125.77 MHz, CDCCl₃): δ 14.2, 61.8, 108.3, 115.9, 145.6, 151.2, 159.9, 166.4; HRMS: calculated m/z for [C₈H₉ClN₂O₂+H]: 201.0431, observed: 201.0422.

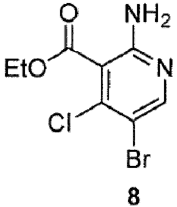

**Ethyl 2-amino-4-chloro-5-bromonicotinate (8):** To a solution of 7 (18.00g, 0.09 mol) in anhydrous DMF was added NBS (18.36 g, 0.10 mol) at room temperature portion-wise. Analysis by TLC of the resulting solution showed that 8 had been completely consumed after 4 h. The resulting mixture was quenched with water (100 mL) and extracted with ethyl acetate (EA, 2 x 100 mL). The combined organic layers were washed with brine, dried over anhydrous sodium sulfate (Na2SO₄), filtered, and concentrated. The resultant residue was purified by column chromatography on silica gel (100-200 mesh) to afford exclusively ethyl 2-amino-4-chloro-5-bromonicotinate **(8)** as a light yellow solid. Yield: 22.07g, 88 %. ¹H NMR (500.13 MHz, CDCl₃): δ 1.41 (t, J = 7.1 Hz, 3H), 4.43 (q, J = 7.1 Hz, 2H), 5.86 (s, 2H), 8.24 (s, 1H); ¹³C NMR (125.77 MHz, CDCl₃): δ 14.2, 62.3, 109.7, 110.4, 144.4, 152.8, 158.0, 165.9. HRMS: calculated m/z for [C₈H₈BrClN₂O₂+H]: 278.9536, observed: 278.9541.

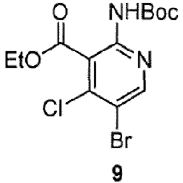

**Ethyl 2-((tert-butoxycarbonyl)amino)-4-chloro-5-Bromonicotinate (9):** To an ice-cold solution of **8** (20.00 g, 71.55 mmol) in anhydrous dichloromethane (CH₂Cl₂) was added diisopropylethylamine (DIEA, 27.42 mL, 157.41 mmol) followed by triphosgene (7.64 g, 25.76 mmol). After 1 h at 0 °C, tert-butanol (33.96 mL, 357.76 mmol) was added over the course of 5 min and the ice bath was removed. The progress of the reaction was monitored by TLC and after 24 h at room temperature, the starting material had been completely consumed. The resulting mixture was quenched with water and extracted with dichloromethane (CH₂Cl₂, 2 x 100 mL). The collected organic layers were washed with brine, saturated solution of sodium bicarbonate (NaHCO₃), dried over anhydrous sodium sulfate, filtered, and concentrated. The obtained crude material was purified by silica gel column chromatography (100-200 mesh) to give Ethyl 2-((tert-butoxycarbonyl)amino)-4-chloro-5-Bromonicotinate **(5)** as a white solid. Yield: 13.58 g, 50 %. ¹H NMR (500.13 MHz, CDCl₃): δ 1.41 (t, J=7.2 Hz, 3H), 1.50 (s, 9H), 4.42 (q, J 7.2 Hz, 2H), 8.37 (s, 1H), 8.57 (s, 1H); ¹³C NMR (125.77 MHz, CDCl₃): δ 14.1, 28.3(3C), 62.6, 82.2, 113.9, 116.3, 119.9, 144.0, 149.5, 151.7, 164.2; HRMS: calculated m/z for [C₁₃H₁₆BrClN₂O₄+H]: 379.0060, observed: 379.0063.

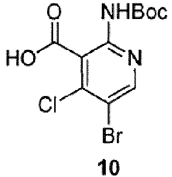

**5-Bromo-2-((tert-butoxycarbonyl)amino)-4-chloronicotinic acid (10):** 41.41 mL of 1M sodium hydroxide (118.53 mmol) was added to a mixture of **9** (10.00 g, 26.34 mmol) in ethanol:THF (3:1, 76 mL) at 0 °C over a period of 15 min. After complete consumption of the starting material as monitored by TLC, the reaction mixture was concentrated. The obtained crude material was transferred into a separating funnel containing water (200 mL) and extracted with EA (1 x 100 mL), and the organic layer was discarded. To the aqueous layer, 10 % HCl (200 mL) and EA (100 mL) were carefully added (pH~3-5). The compound was extracted in EA layer and dried over anhydrous sodium sulphate and concentrated to afford pure 5-Bromo-2-((tert-butoxycarbonyl)amino)-4-chloronicotinic acid **(10)** as a light yellow solid. Yield: 6.02 g, 65 %. ¹H NMR (500.13 MHz, DMSO-d₆): δ 1.43 (s, 9H), 8.69 (s, 1H), 9.88 (s, 1H); ¹³C NMR (125.77 MHz, DMSO-d₆): δ 27.9 (3C), 80.1, 116.3, 124.5, 141.2, 148.8, 150.8, 152.6, 164.1; HRMS: calculated m/z for [C₁₁H₁₂BrClN₂O₄+H]: 350.9747, observed: 350.9712.

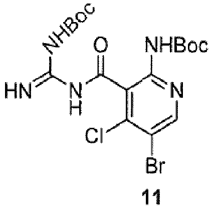

**(11)** Purification by column chromatography for this compound was difficult. So we proceeded to the next step without any purification or analysis. HRMS: calculated m/z for [C₁₇H₂₃BrClN₅O₅+H]: 492.0649, observed: 492.0655.

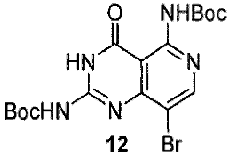

**Di-tert-butyl(8-bromo-4-oxo-3,4-dihydropyrido[4,3-d]pyrimidine-2,5-diyl)dicarbamate (12):** To an ice-cold solution of **10** (5.00 g, 11.07 mmol) in anhydrous dimethylformamide (DMF, 28 mL) was added N-methylmorpholine (NMM, 1.64 mL, 14.94 mmol) followed by chloroethylformate (1.63 mL, 13.84 mmol). After 1 h at 0 °C, Boc-guanidine (2.38 g, 14.94 mmol) was added in one portion and the ice bath was removed. The progress of the reaction was monitored by TLC; and after 24 h at room temperature, the starting material had been completely consumed. The resulting mixture was concentrated to dryness to afford Boc-guanidine adduct as a crude mixture, to which water was added and stirred for 10 min at room temperature. The obtained solid was filtered through a filter paper and dried overnight under vacuum. To the ice-cold solution of the resultant solid in anhydrous DMF was added NaH (60%, 1.77 g, 44.28 mmol) carefully. After 24 h, DMF was removed under vacuum and water was added carefully. The resultant solid was collected by filtration and dried in vacuum. The obtained crude was purified by silica gel column chromatography (100-200 mesh) to give **12** as a yellow solid. Yield: 3.64 g, 72 % (over two steps). ¹H NMR (500.13 MHz, DMSO-d₆): δ 1.48 (s, 9H), 1.50 (s, 9H), 8.54 (s, 1H), 10.85 (s, 1H), 11.58 (s, 1H), 11.78 (s, 1H); ¹³C NMR (125.77 MHz, DMSO-d₆): δ 27.7(3C), 27.8(3C), 80.3, 83.2, 103.2, 109.2, 149.0, 150.4, 152.0, 152.6, 153.7, 154.2, 161.7; HRMS: calculated m/z for [C₁₇H₂₂BrN₅O₅+H]: 456.0883, observed: 456.0890.

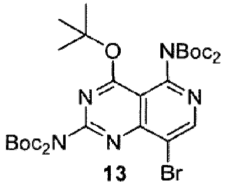

**Di-tert-butyl (8-bromo-4-( tert-butoxy)pyrido[4,3-d]pyrimidine-2,5-diyl)bis((tert-butoxycarbonyl)-carbamate) (13):** To a solution of **12** (3.10 g, 6.79 mmol) in anhydrous THF was added DIEA (3.50 mL, 20.38 mmol) and DMAP (0.17 g, 1.36 mmol), followed by Boc₂O (5.93 g, 27.18 mmol) at room temperature. After 4 h at room temperature, Boc₂O (2.97 g, 13.59 mmol) was added and the reaction mixture was warmed to 50 °C. The consumption of starting material was monitored by TLC and after 24 h, the reaction mixture was then diluted with a saturated solution of NaHCO₃ and EA. The organic layer was separated from the aqueous layer and dried over sodium sulfate. The solvents were removed under vacuum and obtained crude material was purified by silica gel column chromatography to get pure 13 as a white solid. Yield: 3.15 g, 65%. ¹H NMR (500.13 MHz, DMSO-d₆): δ 1.27 (s, 18H), 1.45 (s, 18H), 1.65 (s, 9H), 9.06 (s, 1H); ¹³C NMR (125.77 MHz, DMSO-d₆): δ 27.2(6C), 27.4(6C), 27.5(3C), 82.7(2C), 83.8(2C), 87.6, 109.7, 118.2, 148.7, 148.8(2C), 149.6(2C), 150.8, 155.0, 156.4, 166.5; HRMS: calculated m/z for [C₃₁H₄₆BrN₅O₉+H]: 712.2557, observed: 712.2540.

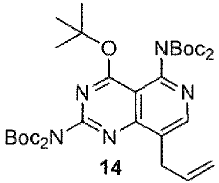

**Di-tert-butyl (8-allyl-4-(tert-butoxy)pyridor-4,3-dlpyrimidine-2,5-diyl)bis((tert-butoxy carbonyl) carbamate) (14):** To a stirred and degassed solution of **13** (3.00 g, 4.21 mmol) in anhydrous toluene (21 mL) was added allyltributyltin (2.79 g, 8.42 mmol) followed by tetrakistriphenylphosphine palladium (0) (0.49 g, 0.42 mmol) at room temperature. The resultant solution was flushed with argon to make an inert atmosphere and refluxed the solution for 15 h at 125 °C. Resulting mixture was cooled to room temperature, silica gel was added, solvents were removed under vacuum, and the slurry was loaded on silica gel column chromatography to yield 14 as a light yellow solid. Yield: 1.70 g, 60%. ¹H NMR (500.13 MHz, CDCl₃): δ 1.32 (s, 18H), 1.47 (s, 18H), 1.71 (s, 9H), 3.78 (dq, J = 6.6, 1.3 Hz, 2H), 4.94 - 5.15 (m, 2H), 6.09 (ddt, J = 16.7, 10.1, 6.4 Hz, 1H), 8.49 (s, 1H); ¹³C NMR (125.77 MHz, CDCl₃): δ 27.8(6C), 27.9(6C), 28.2(3C), 32.4, 82.4(2C), 83 .3(2C), 86.3, 108.8, 116.5, 131.4, 136.0, 148.1(2C), 149.8(2C), 150.3(2C), 155.6, 156.8, 167.1; HRMS: calculated m/z for [C₃₄H₅₁N₅O₉+H]: 674.3765, observed: 674.3746.

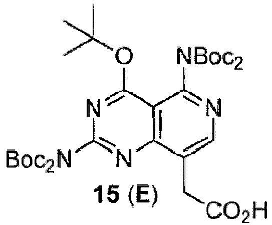

**2-(2,5-bis(bis(tert-butoxycarbonyl)amino)-4-(tert-butoxy)pyrido[4,3-d]pyrimidin-8-yl)acetic acid (15, E):** To an ice-cold stirred solution of **14** (1.00 g, 1.48 mmol) in CH₃CN:CCl₄:H₂O (23:23:34 mL) was added NalO₄ (2.54 g, 11.87 mmol) followed by ruthenium(III) chloride hydrate (0.07 g, 0.30 mmol). After 2 h at 0 °C, the reaction mixture was filtered through a bed of celite and concentrated under vacuum at 35 °C to ¾ volumes. To this mixture, water (20 mL) and EA (20 mL) were added at 25 °C. The organic layer was separated from the aqueous layer and dried over sodium sulfate. The resultant solvents were removed under vacuum and the obtained crude material was purified by silica gel column chromatography to give **15 (E)** as a light brown solid. Yield: 0.51 g 50%. ¹H NMR (300.13 MHz, CDCl₃): δ 1.34 (s, 18H), 1.56 (s, 18H), 1.71 (s, 9H), 4.03 (s, 2H), 8.62 (s, 1H); ¹³C NMR (125.77 MHz, CDCl₃): δ 28.0(6C), 28.1(6C), 28.3(3C), 37.1, 83.2(2C), 85.1(2C), 87.7, 109.0, 124.5, 149.6, 149.9(2C), 150.1, 155.9(2C), 156.5(2C), 167.4, 170.4; HRMS: calculated m/z for [C₃₃H₄₉N₅O₁₁+H]: 692.3507, observed: 692.3491.

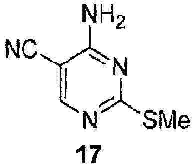

**4-amino-2-(methylthio)pyrimidine-5-carbonitrile (17):** A mixture of 2-(ethoxymethylene)malononitrile (100.00 g, 0.82 mol), S-methylisothiourea hemisulfate salt (73.81 g, 0.82 mol), and triethylamine (370.92 mL, 2.66 mol) in absolute ethanol (819 mL) was stirred at room temperature. After 2 d, solvents were removed under vacuum and the resultant residue was washed with water to yield the desired compound as a white solid. Yield: 122.48 g, 90%. ¹H NMR (500.13 MHz, DMSO-d₆): δ 2.45 (s, 3H), 7.87 (s, 2H), 8.43 (s, 1H); ¹³C NMR (125.77 MHz, DMSO-d₆): δ 13.4, 85.3, 115.6, 160.5, 161.4, 174.6; ESI-MS: calculated m/z for [C₆H₆N₄S+H]: 167.0, observed: 167.1.

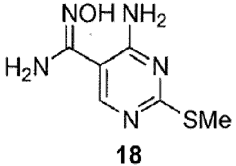

**4-amino-N'-hydroxy-2-(methylthio)pyrimidine-5-carboximidamide (18):** The mixture of **17** (50.00 g, 0.30 mol), hydroxylamine hydrochloride (24.04 g, 0.35 mol), and triethylamine (83.92 mL, 0.60 mol) in methanol (602 mL) was refluxed at 75 °C. After 2 d, the reaction mixture was cooled down to room temperature and the precipitated solid was collected through filtration and washed with methanol and hexane to afford 18 as a light yellow solid. Yield: 46.15 g, 77%. ¹H NMR (500.13 MHz, DMSO-d₆): δ 2.43 (s, 3H), 5.96 (s, 2H), 7.59 (s, 1H), 8.10 (s, 1H), 8.35 (s, 1H), 9.87 (s, 1H); ¹³C NMR (125.77 MHz, DMSO-d₆): δ 13.2, 103.6, 149.9, 152.8, 159.6, 169.3; ESI-MS: calculated m/z for [C₆H₉N₅OS+H]: 200.1, observed: 200.0.

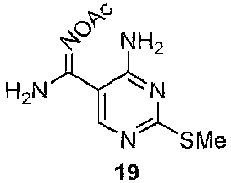

**(E)-N'-acetoxy-4-amino-2-(methylthio)pyrimidine-5-carboximidamide (19):** To a solution of **18** (31.00 g, 0.16 mol) in glacial acetic acid was added acetic anhydride (51.48 mL, 0.55 mol) at room temperature. Analysis by TLC of the resulting solution showed that 18 had been completely consumed after 15 h. Acetic acid was removed under vacuum and to this crude material, saturated sodium bicarbonate solution and EA (each 200 mL) were added (pH-8- 9). Aqueous layer was extracted twice with EA, and the organic layer was dried on sodium sulfate. EA was removed under vacuum to give pure **19** as a white solid. Yield: 28.02 g, 90%. ¹H NMR (500.13 MHz, DMSO-d₆): δ 2.15 (s, 3H), 2.45 (s, 3H), 6.89 (s, 2H), 7.78 (s, 1H), 7.96 (s, 1H), 8.40 (s, 1H); ¹³C NMR (125.77 MHz, DMSO-d₆): δ 13.3, 19.5, 102.4, 154.5, 154.6, 159.8, 167.9, 171.3; HRMS: calculated m/z for [C₈H₁₁N₅O₂S+H]: 242.0712, observed: 242.0718.

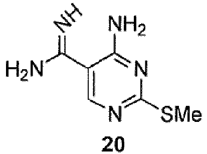

**4-amino-2-(methylthio)pyrimidine-5-carboximidamide (20):** A mixture of **19** (25.00 g, 0.10 mol), formic acid (97.73 mL, 2.59 mol), and palladium on charcoal (10%, 5.51 g, 5.18 mmol) in methanol (130 mL) was refluxed at 80 °C. After 15 h, the reaction mixture was cooled to room temperature and solvents were removed under vacuum to afford the desired product as formic acid salt. The yield of the product as formic acid salt: 29.96 g, 80%. (For NMR analysis: To the resultant crude (1g), methanol (10 mL) and TEA (5 mL, pH-9-10) was added and stirred for 15 min. The resultant solid was collected through filtration and washed with hexane.) ¹H NMR (500.13 MHz, DMSO-d₆): δ 2.43 (s, 3H), 5.96 (s, 2H), 7.59 (s, 1H), 8.11 (s, 1H), 8.36 (s, 1H), 9.87 (s, 1H); 13C NMR (125.77 MHz, DMSO-d6): δ 13.3, 103.6, 149.9, 152.9, 159.6, 169.4; HRMS: calculated m/z for [C₆H₉N₅S+H]: 184.0657, observed: 184.0664.

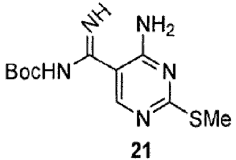

**tert-Butyl ((4-amino-2-(methylthio)pyrimidin-5-yl)(imino)methyl) carbamate (21):** To an ice-cold stirred solution of **20** as formic acid salt (22.00 g, 68.47 mmol) in THF:water (100:125 mL) was added sodium bicarbonate in portion wise (34.51 mL, 0.41 mol). When the pH of the resultant solution reached 8-9, Boc-anhydride (17.19 g, 78.74 mmol) in 25 ml of THF was slowly added. Analysis by TLC of the resulting solution showed that 20 had been completely consumed after 24 h. The reaction mixture was transferred into a separating funnel containing 100 mL of water and 200 mL of EA. The aqueous layer was extracted using EA (2X) and the combined EA layer was dried over sodium sulfate. EA was removed under vacuum and the mixture was purified by silica gel column chromatography to give pure **21** as a white solid. Yield: 15.71g, 81%. ¹H NMR (300.13 MHz, CDCl₃): δ 1.51(s,9H), 2.50 (s, 3H), 7.61 (s, 2H), 8.42 (s, 1H); ¹³C NMR (125.77 MHz, CDCl₃): δ 13.9, 28.1(3C), 79.6, 104.6, 155.2(2C), 161.1(2C), 173.9; HRMS: calculated m/z for [C₁₁H₁₇N₅O₂S+H]: 284.1181, observed: 284.1189.

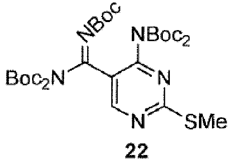

**tert-butyl(Z)-((4-(bis(tert-butoxycarbonyl)amino)-2-(methylthio)pyrimidin-5-yl)((tert-butoxycarbonyl)imino)methyl)(tert-butoxycarbonyl)carbamate (22).** To an ice-cold stirred solution of **21** (15.00 g, 52.94 mmol) in THF (106 mL) was added DIPEA in one portion (46.11 mL, 264.69 mmol) and DMAP (1.29 g, 10.59 mmol), followed by Boc-anhydride (57.77 g, 264.69 mmol) in 25 ml of THF (slowly added). The progress of reaction was monitored by TLC, which showed that 21 had been completely consumed after 24 h. The reaction mixture was transferred to a separating funnel containing 200 mL of water and 100 mL of EA. The mixture was extracted EA (2X), and the combined EA layer was dried over sodium sulfate. EA was removed under vacuum, and the crude mixture was purified by silica gel column chromatography to give pure 22 as a white solid. Yield: 28.96 g, 80%. ¹H NMR (500.13 MHz, CDCl₃): δ 1.44 (s, I 8H), 1.45 (s, 18H), 1.51 (s, 9H), 2.61 (s, 3H), 8.69 (s, 1H); 13C NMR (125.77 MHz, CDCl₃): δ 14.6, 28.0(6C), 28.1(6C), 28.1(3C), 83.2, 83.7(2C), 84.9(2C), 119.2, 148.3, 150.1, 157.3(2C), 158.8, 159.5(2C), 160.4, 176.1; HRMS: calculated m/z for [C₃₁H₅₀N₅O₁₀S+H]: 684.3278, observed: 684.3275.

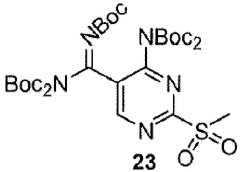

**Compound (23):** To an ice-cold stirred solution of **22** (15.00 g, 21.94 mmol) in DCM (55 mL) was added mCPBA in portion-wise (10.60 g, 61.42 mmol). The progress of the reaction was analyzed by TLC, which showed that **22** had been completely consumed after 3 h. The reaction mixture was transferred to a separating funnel containing 200 mL of saturated solution of sodium bicarbonate and 100 mL of DCM. The mixture was extracted with DCM (2X), and the combined DCM layer was dried over sodium sulfate. DCM was removed under vacuum, and the crude mixture was used in next step without further purification.

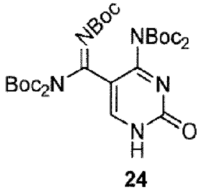

**tert-butyl (Z)-((4-(bis(tert-butoxycarbonyl)amino)-2-oxo-1,2-dihydro-pyrimidin-5-yl)((tert-butoxycarbonyl)imino)methyl)(tert-butoxycarbonyl)carbamate (24):** To an ice-cold stirred solution of crude **23** (15.00 g) in THF (55 mL) was slowly added 2N NaOH (54.84 mL, 109.68 mmol). The starting material consumption was monitored by TLC. After 30 min the reaction mixture was transferred to a separating funnel containing 200 mL of 1 % HCl and 100 mL of EA. The aqueous layer was extracted with EA (2X), and the combined EA layer was dried on sodium sulfate. EA was removed under vacuum, and the crude material was purified by silica gel column chromatography. Yield: 10.75 g, 75% over two steps. ¹H NMR (300.13 MHz, DMSO-d6): δ 1.37 (s, 18H), 1.39 (s, 18H), 1.41 (s, 9H), 8.65 (s, 1H), 12.80 (s, 1H); ¹³C NMR (125.77 MHz, DMSO-d6): δ 27.4 (15C), 82.0, 82.9 (2C), 83.5 (2C), 108.0, 145.2, 147.5 (2C), 149.1 (2C), 152.8, 155.0, 157.0, 162.9; HRMS: calculated m/z for [C₃₀H₄₇N₅O₁₁+H]: 654.3350, observed: 654.3359.

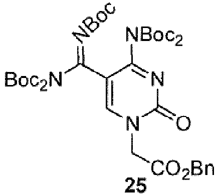

**Benzyl (Z)-2-(4-(bis(tert-butoxycarbonyl)amino)-2-oxo-5-(N,N,N'-tris(tert-butoxycarbonyl) carbamimidoyl)pyrimidin-1(2H)-yl)acetate (25):** To a mixture of compound **24** (10.00 g, 15.30 mmol) and K₂CO₃ (4.23 g, 30.59 mmol) in anhydrous DMF (75 mL) was added drop-wise benzyl-2-bromo acetate (2.42 mL, 15.30 mmol) over a period of 10 min. The progress of reaction was monitored by TLC, which was found to be complete after 4 h. The reaction mixture was filtered through a sintered funnel and DMF was evaporated under vacuum. To the resultant crude material, water was added and stirred for 5 min. The obtained precipitated was collected by filtration and washed with water and hexane to give pure compound **25** as a white solid. (Note: If the compound is not precipitated well, then extraction is an alternative way to get the compound, and if necessary, do a short bed column to get pure compound). Yield: 10.92 g, 89%. ¹H NMR (500.13 MHz, CDCl₃): δ 1.46 (s, 18H), 1.48 (s, 18H), 1.50 (s, 9H), 4. 75 (s, 2H), 5.22 (s, 2H), 7.27 - 7.47 (m, 5H), 7.95 (s, 1H); ¹³C NMR (125.77 MHz, CDCl₃): δ 27.8 (6C), 27.9 (6C), 28.0 (3C), 51.0, 68.2, 83.0, 83.7 (2C), 85.2 (2C), 110.6, 128.6 (3C), 128.7 (2C), 128.8, 134.5, 148.2, 149.5 (2C), 152.0 (2C), 154.4, 157.1, 163.9, 166.1; HRMS: calculated m/z for [C₃₉H₅₅N₅O₁₃+H]: 802.3875, observed: 802.3882.

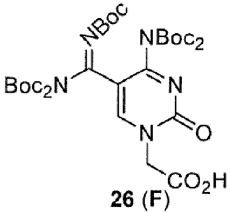

**(Z)-2-(4-(bis(tert-butoxycarbonyl)amino)-2-oxo-5-(N,N,N'-tris(tert-butoxycarbonyl)carbamimidoyl) pyrimidi-1(2H)-yl)acetic acid (26,** F): Palladium on charcoal (10% loading, 0.66 g, 0.62 mmol) was carefully added to a solution of **25** (5.00 g, 6.24 mmol) in EA (50 mL) under argon balloon. After 5 min, argon balloon was replaced with hydrogen balloon and the reaction mixture was stirred for another 1 h under the same atmosphere. The complete depletion of starting material was evaluated by TLC. The resultant reaction mixture was carefully filtered through a bed of celite and the filtrate was evaporated on rotary evaporator to give pure compound **26** as a white solid. Yield: 3.99 g, 90% ¹H NMR (500.13 MHz, DMSO-d6): δ 1.36 (s, 18H), 1.41 (s, 18H), 1.42 (s, 9H), 4.85 (s, 2H), 9.10 (s, 1H), 13.45 (s, 1H); ¹³C NMR (125.77 MHz, DMSO-d6): 027.4 (6C), 27.4 (6C), 27.5 (3C), 50.9, 82.2, 83.0 (2C), 83.3 (2C), 108.3, 145.1, 146.9 (2C), 149.4 (2C), 154.1, 156.1, 157.0, 162.5, 168.4; HRMS: calculated m/z for [C₃₂H₄₉N₅O₁₃+H]: 712.3405, observed: 712.3386.

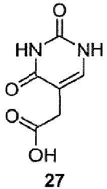

**2-(2,4-dioxo-1,2,3,4-tetrahydropyrimidin-5-yl)acetic acid (27):** This compound was purchased from a commercially available source.

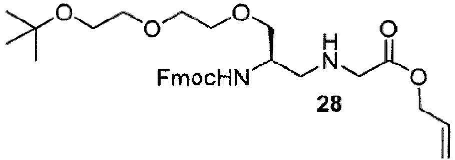

**Allyl (R)-(2-((((9H-fluoren-9-yl)methoxy)carbonyl)amino)-3-(2-(2-(tert-butoxy)ethoxy) ethoxy)propyl) glycinate (28):** To a chilled solution of N-Fmoc(O-MP(^{t}butyl)-L-serinol (5.00 g, 10.93 mmol) in saturated DCM:water (28 mL) was added Dess-Martin Periodinane (9.96 g, 23.49 mmol) portion wise. The reaction mixture was stirred at the same temperature for another 30 min and at room temperature for 1 h to get complete conversion of alcohol to aldehyde. The reaction mixture was diluted with ether, saturated solution of sodium bicarbonate, 10% sodium thiosulfate, and stirred for 5 min. The overall reaction mixture was transferred to a separating funnel and the organic compounds were extracted in ether (2x). The ether layer was dried over sodium sulfate and removed on rotary evaporator to give the crude aldehyde. The aldehyde was used in next step without further purification. To the aldehyde in anhydrous DCM (44 mL) was added allyl glycinate PTSA salt (6.28 g, 21.86 mmol) and DIEA (5.90 mL, 33.89 mmol), and allowed to react for 1 h. NaB(OAc)₃H (5.79 g, 27.33 mmol) was added in one portion to the reaction mixture. After stirring overnight at room temperature, the reaction mixture was diluted with DCM (100 mL) and saturated solution of sodium bicarbonate. The reaction mixture was transferred to a separating funnel and the layer was separated from the aqueous layer. The combined organic layers were dried over sodium sulfate and removed on rotary evaporator, and the crude mixture was purified by flash silica gel column chromatography. Yield: 4.85 g, 85%. ¹HNMR (500.13 MHz, CDCl₃): δ 1.19 (s, 9H), 2.77 -2.92 (m, 2H), 3.49-3.69 (m, 13H), 3.88 (bs, 1H), 4.23 (t, J = 6.9 Hz, 1H), 4.39 (d, J = 7.5 Hz, 2H), 4.64 (dt, J = 5.9, 1.4 Hz, 2H), 5.23 - 5.36 (m, 2H), 5.73 (d, J = 8.4 Hz, 1H), 5.92 (ddt, J = 17.3, 10.4, 5.8 Hz, 1H), 7.32 (td, J = 7.5, 1.2 Hz, 2H), 7.36- 7.43 (m, 2H), 7.60- 7.68 (m, 2H), 7.74- 7.79 (m, 2H); ¹³C NMR (125.77 MHz, CDCl₃): δ 27.5 (3C), 47.3, 50.3, 50.5, 50.6, 50.7, 61.2, 65.5, 66.7, 70.5, 70.8, 71.2, 71.4, 73 .1, 118.7, 119.9, 120.0, 120.0, 125.2, 127.0, 127.6, 127.7, 131.8, 141.3, 144.0, 144.0, 156.4, 171.8, 174.3; ESI-MS: calculated m/z for [C₃₁H₄₂N₂O₇1+H]: 555.3, observed: 555.3.

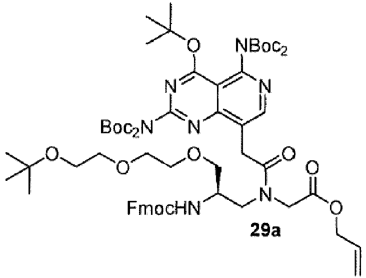

**Allyl Monomer (29a):** To a mixture of **15 (E)** (0.50 g, 0.72mmol) and DIEA (163.67 µL, 0.94 mmol) in anhydrous DMF (6 mL) was added HBTU (0.32 g, 0.83mmol) in one portion at room temperature. After 10 min at room temperature, backbone **28** (0.60 g, 1.08 mmol) in DMF (4 mL) was added to the above reaction mixture. The progress of reaction as monitored by TLC (Eluents: EA:Hex (50:50); R_{f} for backbone= 0.2; R_{f} for product= 0.6; R_{f} for **E** = 0.1). After complete consumption of the starting material, DMF was removed under vacuum at 45 °C. To the resultant crude material, 1 % HCl (20 ml) and EA (20 ml) were added and the organic layers (2x) were separated from water layer using separating funnel. The combined organic layer was dried, removed, and the acquired crude was purified by flash silica gel column chromatography. Yield: 0.62 g, 70%. ¹H NMR (500.13 MHz, DMSO-d6, rotamer): δ 1.08/1.09 (s, 9H), 1.21 1.33 (m, 18H), 1.36/1.38 (s, 18H), 1.41/1.65 (s, 9H), 3.36-3.70 (m, 10H), 3.82 -4.27 (m, 8H), 4.32 (bs, 2H), 4.41 - 4.71 (m, 2H), 5.13 - 5.41 (m, 2H), 5.81 - 6.01 (m, 1H), 7.30 - 7.57 (m, 5H), 7.71/7.73(d, J = 9.7 Hz, 2H), 7.89/7.99 (d, J = 7.5 Hz, 2H), 8.56/8.60 (s, 1H); ¹³C NMR (125.77 MHz, DMSO-d6, rotamer): δ 27.2-27.6 (18C), 46.7, 59.7, 60.6/60.6, 64.7, 65.3, 65.5, 69.6, 69.6, 69.8, 70.0, 70.4, 70.4, 72.2, 81.8-86.3 (6C), 108.1, 117.7, 118.2, 120.I (2C), 125.1, 127.0 (2C), 127.3, 127.6 (2C), 127.8/127.9, 132.1, 132.3, 140.7, 143.8, 147.9, 149.3, 149.5, 149.6, 155.0/155.1, 155.9, 156.5, 166.6, 168.8, 169.2, 170.2; HRMS: calculated m/z for [C₆₄H₈₉N₇O₁₇+H]: 1228.6393, observed: 1228.6398.

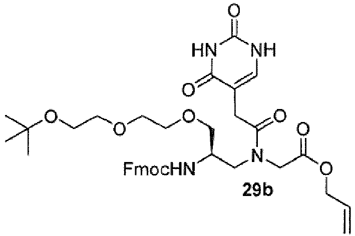

**Allyl Monomer (29b):** This compound was prepared using the above-mentioned procedure for compound **29a** using compound **27** (1.00 g, 5.88 mmol) and backbone **28** (4.08 g, 7.35mmol) as the starting materials. TLC (Eluents: 5% MeOH:DCM (5:95); R_{f} for backbone= 0.6; R_{f} for product= 0.2; R_{f} for **27=** 0.0).Yield: 3.5 g, 84 %. ¹H NMR (500.13 MHz, DMSO-d6, rotamer): δ 1.10/1.10 (s, 9H), 3.30-3.59 (m, 14H), 3.744.13 (m, 2H), 4.17 4.42 (m, 4H), 4.60 (dd, J = 33.4, 4.8 Hz, 2H), 5.00 - 5.44 (m, 2H), 5.82- 6.01 (m, 1H), 7.10- 7.55 (m, 6H), 7.69 (d, J = 7.4 Hz, 2H), 7.89 (d, J = 7.5 Hz, 2H), 10.75 (bs, 1H), 11.08 (s, 1H); ¹³C NMR (125.77 MHz, DMSO-d6, rotamer) δ 27.2 (3C), 46.7, 60.6 (2C), 64.7, 65.2, 65.5, 69.6, 69.8, 69.9, 70.4 (2C), 72.2, 107.2, 117.7, 118.1, 120.1 (2C), 125.2, 127.0 (2C), 127.3, 127.6(2C), 132.2/132.3, 139.2, 139.7, 140.7, 143.8/143.8, 151.2/151.3, 155.8, 164.1/164.1, 168.9, 169.4, 170.6; HRMS: calculated m/z for [C₃₇H₄₆N₄O₁₀+H]: 707.3292, observed: 707.3290.

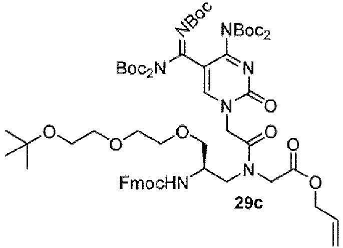

**Allyl Monomer (29c):** This compound was prepared using the above-mentioned procedure for compound **29a** using compound **26** (3.50 g, 4.92mmol) and backbone **28** (3.41 g, 6.15 mmol) as the starting materials. TLC (Eluents: EA:Hex (40:60); R_{f} for backbone= 0.1; R_{f} for product= 0.7; R_{f} for **26** = 0.1).Yield: 4.80 g, 78 %. ¹HNMR (300 MHz, DMSO-d6): δ 1.09 (bs, 9H), 1.34/13.6 (s, 18H), 1.41/1.42 (s, 27H), 3.35 - 3.59 (m, 14H), 4.07 -4.49 (m, 4H), 4.61 (dd, J = 37.6, 5.4 Hz, 3H), 4.97 - 5.42 (m, 3H), 5.77 - 6.04 (m, 1H), 7.32 (t, J = 7.5 Hz, 2H), 7.50 - 7.57 (m, 3H), 7.89 (d, J = 7.5 Hz, 2H), 7.99 (d, J = 8.4 Hz, 2H), 8.94/8.96 (s, 1H); ¹³C NMR (125.77 MHz, DMSO-d6, rotamer): δ 27.2-27.5 (18C), 46.7, 60.2/60.7 (2C), 64.7, 65.5, 69.5, 69.6, 69.8, 69.9, 70.4/70.4 (2C), 72.2, 82.2-83.4 (6C), 108.1, 117.7, 120.1 (2C), 124.4, 125.1, 127.0 (2C), 127.4, 127.6 (2C), 127.8, 132.2 (2C), 140.7 (2C), 143.8 (2C), 145.1, 146.9 (2C), 149.5 (2C), 157.0, 162.5, 166.5, 168.4; HRMS: calculated m/z for [C₆₃H₈₉N₇O₁₉+H]: 1248.6291, observed: 1248.6256.

## Claims

1. A genetic recognition reagent comprising:
(a) a nucleic acid analog backbone, the nucleic acid analog backbone comprising: (i) three or more nucleic acid analog backbone residues, (ii) a first end and a second end, and (iii) a first concatenating group attached to the first end of the backbone and a second concatenating group attached to the second end, either binding non-covalently or self-ligating with the first concatenating group,
wherein the first concatenating group and the second concatenating group are each independently a two- to five-ring fused polycyclic aromatic moiety that is xanthene, riboflavin, mangostin, or mangiferin that stack with an aryl moiety of an adjacent recognition reagent when recognition reagents are hybridized to adjacent sequences of a target nucleic acid,(b) a sequence of bivalent nucleobases attached to the three or more nucleic acid analog backbone residues, where the sequence of bivalent nucleobases binds to a unit target sequence, or one or more sequential iterations of a unit target sequence of an expanded repeat of a repeat expansion disease on two nucleic acid strands, wherein the sequence of bivalent nucleobases comprises a unit recognition reagent sequence that is
3-6-4,
6-4-3,
4-3-6,
4-6-3,
6-3-4,
or 3-
4-6,
wherein:
each 3 is independently 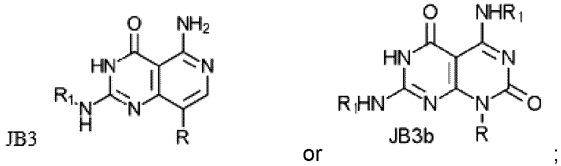
each 4 is independently 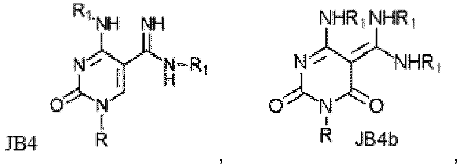 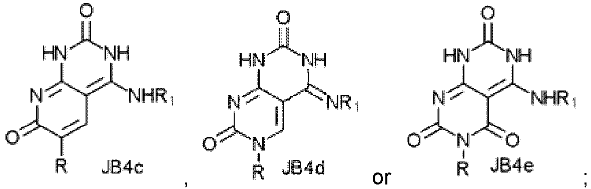
each 6 is independently 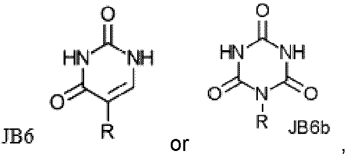
wherein R is a nucleic acid analog backbone residue,
wherein and R₁ is a protecting group or H.

2. The genetic recognition reagent of claim 1, wherein the sequence of bivalent nucleobases is:
a JB3 nucleobase, a JB6 nucleobase, and a JB4 nucleobase;
a JB6 nucleobase, a JB4 nucleobase, and a JB3 nucleobase;
a JB4 nucleobase, a JB3 nucleobase, and a JB6 nucleobase;
JB3 or JB3b; JB6 or JB6b; and JB4, JB4b, JB4c, JB4d, or JB4e;
JB6 or JB6b; JB4, JB4b, JB4c, JB4d, or JB4e; and JB3 or JB3b;
JB4, JB4b, JB4c, JB4d, or JB4e; JB3 or JB3b; and JB6 or JB6b,
JB3, JB6, and JB4;
JB6, JB4, and JB3; or
JB4, JB3, and JB6, or two or more sequential iterations of the preceding.

3. The genetic recognition reagent of claim 1 or claim 2, wherein both of the two nucleic acid strands are RNA.

4. The genetic recognition reagents of any one of claims 1-3, wherein the nucleic acid analog backbone residues are γPNA residues.

5. The genetic recognition reagent of claim 1, wherein the recognition reagent has the structure: 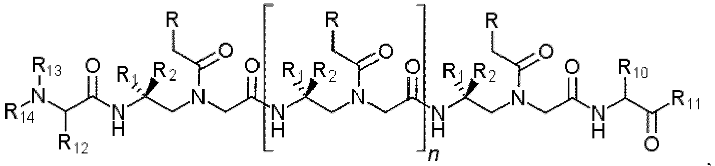 where,
each instance of R is, independently, a nucleobase of the sequence of bivalent nucleobases;
n is an integer ranging from 1 to 6;
each R₁ and R₂ are each, independently, H; a guanidine-containing group; an amino acid side chain, unsubstituted or substituted (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)aryl(C₁-C₆)alkylene, or (C₃-C₈)cycloalkyl(C₁-C₆)alkylene; -CH₂-(OCH₂-CH₂)_{q}OP₁; -CH₂-(OCH₂-CH₂)_{q}-NHP₁; -CH₂-(OCH₂-CH₂-O)_{q}-SP₁; -CH₂-(SCH₂-CH₂)_{q}-SP₁, -CH₂-(OCH₂-CH₂)ᵣ-OH; -CH₂-(OCH₂-CH₂)ᵣ-NH₂; -CH₂-(OCH₂-CH₂)ᵣ-NHC(NH)NH₂; or -CH₂-(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50, inclusive; r and s are each independently integers from 1 to 50, inclusive;
one of R₁₀ or R₁₁, and one of R₁₂, R₁₃, or R₁₄ are -L- R₃, where each instance of R₃ is, independently, the two- to five-ring fused polycyclic aromatic moiety;
L is a linker; and
the remainder of R₁₀, R₁₁, R₁₂, R₁₃, and R₁₄ are each, independently, H, one or more contiguous amino acid residues, a guanidine-containing group, an amino acid side chain, unsubstituted or substituted (C₁-C₈)alkyl, (C₂-C₈)alkenyl, (C₂-C₈)alkynyl, (C₁-C₈)hydroxyalkyl, (C₃-C₈)aryl, (C₃-C₈)cycloalkyl, (C₃-C₈)aryl(C₁-C₆)alkylene, or (C₃-C₈)cycloalkyl(C₁-C₆)alkylene; -CH₂-(OCH₂-CH₂)_{q}OP₁; -CH₂-(OCH₂-CH₂)_{q}-NHP₁; -CH₂-(OCH₂-CH₂-O)_{q}-SP₁; -CH₂-(SCH₂-CH₂)_{q}-SP₁, -CH₂-(OCH₂-CH₂)ᵣ-OH; -CH₂-(OCH₂-CH₂)ᵣ-NH₂; -CH₂-(OCH₂-CH₂)ᵣ-NHC(NH)NH₂; or -CH₂-(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50, inclusive; r and s are each independently integers from 1 to 50,
or a pharmaceutically-acceptable salt thereof.

6. The genetic recognition reagent of claim 5, wherein one or more of R₁, R₂, R₁₀, R₁₁, R₁₂, R₁₃, or R₁₄ is (C₁-C₆)alkyl substituted with -(OCH₂-CH₂)_{q}OP₁; -(OCH₂-CH₂)_{q}-NHP₁; - (SCH₂-CH₂)_{q}-SP₁; -(OCH₂-CH₂)ᵣ-OH; -(OCH₂-CH₂)ᵣ-NH₂; -(OCH₂-CH₂)ᵣ-NHC(NH)NH₂; or -(OCH₂-CH₂)ᵣ-S-S[CH₂CH₂]ₛNHC(NH)NH₂, where P₁ is H, (C₁-C₈)alkyl, (C₂-C₈)alkenyl, (C₂-C₈)alkynyl, (C₃-C₈)aryl, (C₃-C₈)cycloalkyl, (C₃-C₈)aryl(C₁-C₆)alkylene or (C₃-C₈)cycloalkyl(C₁-C₆)alkylene; q is an integer from 0 to 50; r is an integer from 1 to 50, and s is an integer from 1 to 50.

7. The genetic recognition reagents of claim 5, comprising a guanidine-containing group.

## Patentansprüche

1. Genetisches Erkennungsreagenz, umfassend:
(a) ein Nukleinsäureanalogon-Grundgerüst, wobei das Nukleinsäureanalogon-Grundgerüst umfasst: (i) drei oder mehr Nukleinsäureanalogon-Grundgerüstreste, (ii) ein erstes Ende und ein zweites Ende und (iii) eine erste, am ersten Ende des Grundgerüsts befestigte verkettende Gruppe und eine zweite, am zweiten Ende befestigte verkettende Gruppe, die entweder nicht-kovalent oder selbst-ligierend an die erste verkettende Gruppe bindet,
wobei die erste und die zweite verkettende Gruppe jeweils unabhängig voneinander eine fusionierte polyzyklische aromatische Einheit mit zwei bis fünf Ringen darstellen, bei der es sich um Xanthen, Riboflavin, Mangostin oder Mangiferin hindelt, die mit einer Arylgruppe eines benachbarten Erkennungsreagenz eine Stacking-Wechselwirkung eingeht, wenn Erkennungsreagenzien an benachbarte Sequenzen einer Zielnukleinsäure hybridisiert werden; (b) eine Sequenz bivalenter Nukleobasen, die an die drei oder mehr Nukleinsäureanalogon-Grundgerüstreste gebunden ist, wobei die Sequenz bivalenter Nukleobasen an eine Zielsequenzeinheit oder eine oder mehrere aufeinanderfolgende Iterationen einer Zielsequenzeinheit einer expandierten Wiederholung einer Repeat-Expansionskrankheit auf zwei Nukleinsäuresträngen bindet, wobei die Sequenz bivalenter Nukleobasen eine Erkennungsreagenzsequenzeinheit umfasst, bei der es sich handelt um
3-6-4,
6-4-3,
4-3-6,
4-6-3,
6-3-4
oder 3-4-6,
wobei:
jedes 3 unabhängig 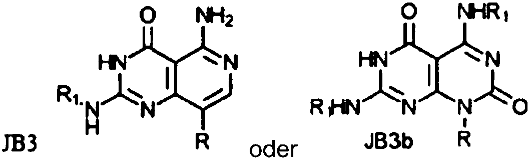 ist;
jedes 4 unabhängig 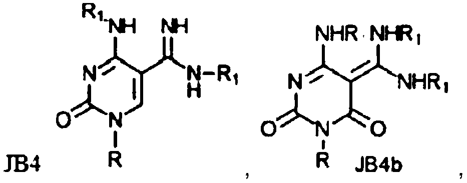 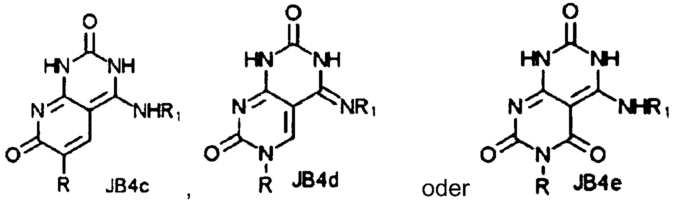 ist;
jedes 6 unabhängig 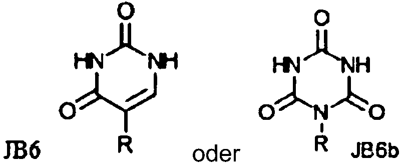 ist,
wobei R ein Nukleinsäureanalogon-Rückgratrest ist,
und wobei R₁ eine Schutzgruppe oder H ist.

2. Genetisches Erkennungsreagenz nach Anspruch 1, wobei es sich bei der Sequenz bivalenter Nukleobasen handelt um:
eine JB3-Nukleobase, eine JB6-Nukleobase und eine JB4-Nukleobase;
eine JB6-Nukleobase, eine JB4-Nukleobase und eine JB3-Nukleobase;
eine JB4-Nukleobase, eine JB3-Nukleobase und eine JB6-Nukleobase;
JB3 oder JB3b; JB6 oder JB6b; und JB4, JB4b, JB4c, JB4d oder JB4e;
JB6 oder JB6b; JB4, JB4b, JB4c, JB4d oder JB4e; und JB3 oder JB3b;
JB4, JB4b, JB4c, JB4d oder JB4e; JB3 oder JB3b; und JB6 oder JB6b, JB3, JB6 und JB4;
JB6, JB4 und JB3; oder
JB4, JB3 und JB6, oder zwei oder mehr aufeinanderfolgende Wiederholungen des Vorstehenden.

3. Genetisches Erkennungsreagenz nach Anspruch 1 oder Anspruch 2, wobei es sich bei beiden Nukleinsäuresträngen um RNA handelt.

4. Genetische Erkennungsreagenzien nach einem der Ansprüche 1 - 3, wobei es sich bei den Nukleinsäureanalogon-Grundgerüstresten um γPNA-Reste handelt.

5. Genetisches Erkennungsreagenz nach Anspruch 1, wobei das Erkennungsreagenz die Struktur aufweist: 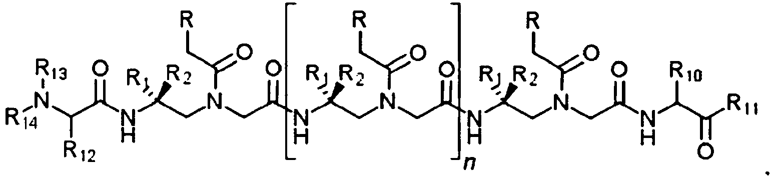 wobei
jedes Vorkommen von R unabhängig voneinander eine Nukleobase der Sequenz bivalenter Nukleobasen ist;
n eine ganze Zahl von 1 bis 6 ist;
jedes R₁ und R₂ jeweils unabhängig voneinander H; eine Guanidin-haltige Gruppe; eine Aminosäureseitenkette, unsubstituiertes oder substituiertes (C₁-C₈)-Alkyl, (C₂-C₈)-Alkenyl, (C₂-C₈)-Alkinyl, (C₁-C₈)-Hydroxyalkyl, (C₃-C₈)-Aryl, (C₃-C₈)-Cycloalkyl, (C₃-C₈)-Aryl-(C₁-C₆)-Alkylen oder (C₃-C₈)-Cycloalkyl-(C₁-C₆)-Alkylen; -CH₂-(OCH₂-CH₂)_{q}OP₁; -CH₂-(OCH₂-CH₂O)_{q}-NHP₁; -CH₂-(OCH₂-CH₂-O)_{q}-SP₁; -CH₂-(SCH₂-CH₂)_{q}-SP₁, -CH₂-(OCH₂-CH₂)ᵣ-OH; -CH₂-(OCH₂-CH₂)r-NH₂; -CH₂-(OCH₂-CH₂)ᵣ-NHC(NH)NH₂; oder -CH₂-(OCH₂-CH₂)ᵣ-S-S[CH₂CH₂]ₛNHC(NH)NH₂, wobei P₁ H, (C₁-C₈)-Alkyl, (C₂-C₈)-Alkenyl, (C₂-C₈)-Alkinyl, (C₃-C₈)-Aryl, (C₃-C₈)-Cycloalkyl, (C₃-C₈)-Aryl-(C₁-C₆)-Alkylen oder (C₃-C₈)-Cycloalkyl-(C₁-C₆)-Alkylen ist; q eine ganze Zahl von 0 bis einschließlich 50 ist; r und s jeweils unabhängig ganze Zahlen von 1 bis einschließlich 50 sind;
eines von R₁₀ oder R₁₁ und eines von R₁₂, R₁₃ oder R₁₄ -L-R₃ sind, wobei es sich bei jedem Vorkommen von R₃ unabhängig um die die zwei- bis fünfringige fusionierte polyzyklische aromatische Einheit mit zwei bis fünf Ringen handelt;
L ein Linker ist; und
die übrigen von R₁₀, R₁₁, R₁₂, R₁₃ und R₁₄ jeweils unabhängig H, ein oder mehrere benachbarte Aminosäurereste, eine Guanidin-haltige Gruppe, eine Aminosäureseitenkette, unsubstituiertes oder substituiertes (C₁-C₈)-Alkyl, (C₂-C₈)-Alkenyl, (C₂-C₈)-Alkinyl, (C₁-C₈)-Hydroxyalkyl, (C₃-C₈)-Aryl, (C₃-C₈)-Cycloalkyl, (C₃-C₈)-Aryl-(C₁-C₆)-Alkylen oder (C₃-C₈)-Cycloalkyl-(C₁-C₆)-Alkylen; -CH₂-(OCH₂-CH₂)_{q}OP₁; -CH₂-(OCH₂-CH₂O)_{q}-NHP₁; -CH₂-(OCH₂-CH₂-O)_{q}-SP₁; -CH₂-(SCH₂-CH₂)_{q}-SP₁, -CH₂-(OCH₂-CH₂)ᵣ-OH; -CH₂-(OCH₂-CH₂)r-NH₂; -CH₂-(OCH₂-CH₂)ᵣ-NHC(NH)NH₂; oder -CH₂-(OCH₂-CH₂)ᵣ-S-S[CH₂CH₂]ₛNHC(NH)NH₂ sind, wobei P₁ H, (C₁-C₈)-Alkyl, (C₂-C₈)-Alkenyl, (C₂-C₈)-Alkinyl, (C₃-C₈)-Aryl, (C₃-C₈)-Cycloalkyl, (C₃-C₈)-Aryl-(C₁-C₆)-Alkylen oder (C₃-C₈)-Cycloalkyl-(C₁-C₆)-Alkylen ist; q eine ganze Zahl von 0 bis einschließlich 50 ist; r und s jeweils unabhängig ganze Zahlen von 1 bis 50 sind,
oder ein pharmazeutisch verträgliches Salz davon.

6. Genetisches Erkennungsreagenz nach Anspruch 5, wobei eines oder mehrere von R₁, R₂, R₁₀, R₁₁, R₁₂, R₁₃ oder R₁₄ (C₁-C₆)-Alkyl ist, substituiert mit -(OCH₂-CH₂)_{q}OP₁; -(OCH₂-CH₂)q-NHP₁; -(SCH₂-CH₂)_{q}-SP₁; -(OCH₂-CH₂)ᵣ-OH; -(OCH₂-CH₂)ᵣ-NH₂; - (OCH₂-CH₂)-NHC(NH)NH₂; oder -(OCH₂-CH₂)-S-S[CH₂CH₂]ₛNHC(NH)NH₂, wobei P₁ H, (C₁-C₈)-Alkyl, (C₂-C₈)-Alkenyl, (C₂-C₈)-Alkinyl, (C₃-C₈)-Aryl, (C₃-C₈)-Cycloalkyl, (C₃-C₈)-Aryl-(C₁-C₆)-Alkylen oder (C₃-C₈)-Cycloalkyl-(C₁-C₆)-Alkylen ist; q eine ganze Zahl von 0 bis 50 ist; r eine ganze Zahl von 1 bis 50 ist und s eine ganze Zahl von 1 bis 50 ist.

7. Genetische Erkennungsreagenzien nach Anspruch 5, umfassend eine Guanidin-haltige Gruppe.

## Revendications

1. Réactif de reconnaissance génétique, comprenant :
(a) un squelette d'analogue d'acide nucléique, le squelette d'analogue d'acide nucléique comprenant : (i) trois résidus de squelette d'analogue d'acide nucléique ou plus, (ii) une première extrémité et une seconde extrémité, et (iii) un premier groupe de concaténation fixé à la première extrémité du squelette et un second groupe de concaténation fixé à la seconde extrémité, se liant de manière non covalente ou s'auto-ligaturant avec le premier groupe de concaténation,
dans lequel le premier groupe de concaténation et le second groupe de concaténation sont chacun indépendamment une fraction aromatique polycyclique fusionnée à deux à cinq cycles qui est le xanthène, la riboflavine, la mangostine ou la mangiférine qui s'empile avec une fraction aryle d'un réactif de reconnaissance adjacent lorsque des réactifs de reconnaissance sont hybridés à des séquences adjacentes d'un acide nucléique cible, (b) une séquence de nucléobases bivalentes attachées aux trois résidus de squelette d'analogue d'acide nucléique ou plus, où la séquence de nucléobases bivalentes se lie à une séquence cible unitaire, ou une ou plusieurs itérations séquentielles d'une séquence cible unitaire d'une répétition étendue d'une maladie d'expansion répétée sur deux brins d'acide nucléique, dans lequel la séquence de nucléobases bivalentes comprend une séquence de réactif de reconnaissance unitaire qui est
3-6-4,
6-4-3,
4-3-6,
4-6-3,
6-3-4,
ou 3-
4-6,
dans lequel :
chaque 3 est indépendamment 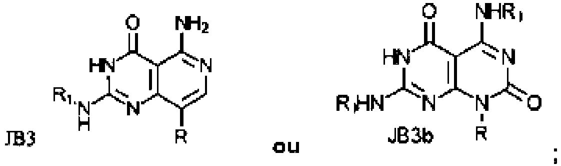
chaque 4 est indépendamment 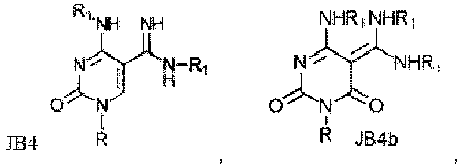 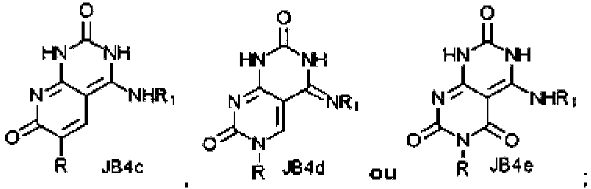
chaque 6 est indépendamment 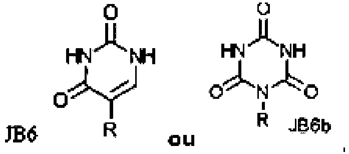
dans lequel R est un résidu de squelette d'analogue d'acide nucléique,
dans lequel R₁ est un groupe protecteur ou H.

2. Réactif de reconnaissance génétique selon la revendication 1, dans lequel la séquence de nucléobases bivalentes est :
une nucléobase JB3, une nucléobase JB6, et une nucléobase JB4 ;
une nucléobase JB6, une nucléobase JB4, et une nucléobase JB3 ;
une nucléobase JB4, une nucléobase JB3, et une nucléobase JB6 ;
JB3 ou JB3b ; JB6 ou JB6b ; et JB4, JB4b, JB4c, JB4d ou JB4e ;
JB6 ou JB6b; JB4, JB4b, JB4c, JB4d ou JB4e ; et JB3 ou JB3b ;
JB4, JB4b, JB4c, JB4d ou JB4e ; JB3 ou JB3b ; et JB6 ou JB6b,
JB3, JB6, JB4,
JB6, JB4, et JB3 ; ou
JB4, JB3 et JB6, ou deux itérations séquentielles ou plus de la précédente.

3. Réactif de reconnaissance génétique selon la revendication 1 ou la revendication 2, dans lequel les deux brins d'acide nucléique sont de l'ARN.

4. Réactifs de reconnaissance génétique selon l'une quelconque des revendications 1 à 3, dans lesquels les résidus de squelette d'analogue d'acide nucléique sont des résidus d'yPNA.

5. Réactif de reconnaissance génétique selon la revendication 1, dans lequel le réactif de reconnaissance présente la structure : 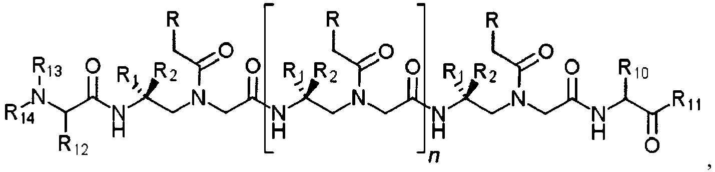 où,
chaque instance de R est, indépendamment, une nucléobase de la séquence de nucléobases bivalentes ;
n est un nombre entier allant de 1 à 6 ;
chaque R₁ et R₂ sont chacun, indépendamment, H ; un groupe contenant de la guanidine ; une chaîne latérale d'acide aminé, un alkyle en (C₁-C₈), un alcényle en (C₂-C₈), un alcynyle en (C₂-C₈), un hydroxyalkyle en (C₁-C₈), un aryle en (C₃-C₈), un cycloalkyle en (C₃-C₈), un (C₃-C₈)aryl(C₁-C₆)alkylène, ou un (C₃-C₈)cycloalkyl(C₁-C₆)alkylène non substitué ou substitué ; -CH₂-(OCH₂-CH₂)_{q}OP₁ ; -CH₂-(OCH₂-CH₂)_{q}-NHP₁ ; -CH₂-(OCH₂-CH₂-O)_{q}-SP₁ ; -CH₂-(SCH₂-CH₂)_{q}-SP₁, -CH₂-(OCH₂-CH₂)ᵣ-OH ; - CH₂-(OCH₂-CH₂)ᵣ-NH₂ ; -CH₂-(OCH₂-CH₂)ᵣ-NHC(NH)NH₂ ; ou -CH₂-(OCH₂-CH₂)ᵣ-S-S[CH₂CH₂]ₛNHC(NH)NH₂, où P₁ est H, un alkyle en (C₁-C₈), un alcényle en (C₂-C₈), un alcynyle en (C₂-C₈), un aryle en (C₃-C₈), un cycloalkyle en (C₃-C₈), un (C₃-C₈)aryl(C₁-C₆)alkylène ou un (C₃-C₈)cycloalkyl(C₁-C₆)alkylène ; q est un entier de 0 à 50 inclus ; r et s sont chacun indépendamment des entiers de 1 à 50 inclus ;
l'un de R₁₀ ou R₁₁, et l'un de R₁₂, R₁₃ ou R₁₄ sont -L-R₃, où chaque instance de R₃ est, indépendamment, la fraction aromatique polycyclique fusionnée à deux à cinq cycles ; L est un lieur ; et
le reste de R₁₀, R₁₁, R₁₂, R₁₃ et R₁₄ sont chacun, indépendamment, H, un ou plusieurs résidus d'acides aminés contigus, un groupe contenant de la guanidine, une chaîne latérale d'acides aminés, un alkyle en (C₁-C₈) non substitué ou substitué, un alcényle en (C₂-C₈), un alcynyle en (C₂-C₈), un hydroxyalkyle en (C₁-C₈), un aryle en (C₃-C₈), un cycloalkyle en (C₃-C₈), un aryl(C₃-C₈)alkylène (C₁-C₆) ou un cycloalkyle(C₃-C ₈) (C₁-C₆)alkylène ; -CH₂-(OCH₂-CH₂)_{q}OP₁ ; -CH₂-(OCH₂-CH₂)_{q}-NHP₁ ; -CH₂-(OCH₂-CH₂-O)_{q}-SP₁ ; -CH₂-(SCH₂-CH₂)_{q}-SP₁, -CH₂-(OCH₂-CH₂)ᵣ-OH ; -CH₂-(OCH₂-CH₂)-NH₂ ; - CH₂-(OCH₂-CH₂)-NHC(NH)NH₂ ; ou -CH₂-(OCH₂-CH₂)-S-S[CH₂CH₂]ₛNHC (NH)NH₂, où P₁ est H, un alkyle en (C₁-C₈), un alcényle en (C₂-C₈), un alcynyle en (C₂-C₈), un aryle en (C₃-C₈), un cycloalkyle en (C₃-C₈), un (C₃-C₈)aryl(C₁-C₆)alkylène ou un (C₃-C₈)cycloalkyl(C₁-C₆)alkylène ; q est un entier de 0 à 50 inclus ; r et s sont chacun indépendamment des entiers de 1 à 50,
ou un sel pharmaceutiquement acceptable de celui-ci.

6. Réactif de reconnaissance génétique selon la revendication 5, dans lequel un ou plusieurs de R₁, R ₂, R₁₀, R ₁₁, R ₁₂, R₁₃ ou R₁₄ est alkyle en (C₁-C₆) substitué par - (OCH₂-CH₂)_{q}OP₁ ; -(OCH₂-CH₂)_{q}-NHP₁ ; -(SCH₂-CH₂)_{q}-SP₁ ; -(OCH₂-CH₂)ᵣ-OH ; - (OCH₂-CH₂)ᵣ-NH₂ ; -(OCH₂-CH₂)ᵣ-NHC(NH)NH₂ ; ou -(OCH₂-CH₂)-S-S[CH₂CH₂]ₛNHC(NH)NH₂, où P₁ est H, un alkyle en (C₁-C₈), un alcényle en (C₂-C₈), un alcynyle en (C₂-C₈), un aryle en (C₃-C₈), un cycloalkyle en (C₃-C₈), un (C₃-C₈)aryl(C₁-C₆)alkylène ou un (C₃-C₈)cycloalkyl(C₁-C₆)alkylène ; q est un nombre entier de 0 à 50 ; r est un nombre entier de 1 à 50, et s est un nombre entier de 1 à 50.

7. Réactifs de reconnaissance génétique selon la revendication 5, comprenant un groupe contenant de la guanidine.
